# EUROPEAN PATENT APPLICATION

(11) **EP 4 442 680 A1**
(43) Date of publication of application: **09.10.2024**
(21) Application number: 22900244.9
(22) Date of filing: 09.11.2022
(51) Int. Cl.: C07D 237/32, C07D 401/14, C07D 401/04, C07D 471/14, C07D 471/04, C07D 487/04, C07D 498/04, A61K 31/502, A61K 31/517, A61K 31/519, A61P 35/00

(54) **PYRAZOLE DERIVATIVE, AND PREPARATION METHOD THEREFOR AND USE THEREOF IN MEDICINE**

(30) Priority: 30.11.2021 CN 202111441550; 14.01.2022 CN 202210044528
(71) Applicant: Abbisko Therapeutics Co., Ltd., Shanghai 201203 (CN)
(72) Inventor: DENG, Haibing, Shanghai 201203 (CN); YANG, Fei, Shanghai 201203 (CN); ZHU, Wei, Shanghai 201203 (CN); LIU, Xiaofeng, Shanghai 201203 (CN); LIU, Jian, Shanghai 201203 (CN); LI, Mingfeng, Shanghai 201203 (CN); LIU, Zhaomin, Shanghai 201203 (CN); YING, Haiyan, Shanghai 201203 (CN); YU, Hongping, Shanghai 201203 (CN); CHEN, Zhui, Shanghai 201203 (CN); XU, Yaochang, Shanghai 201203 (CN)
(74) Representative: dompatent von Kreisler Selting Werner - Partnerschaft von Patent- und Rechtsanwälten mbB
(86) International application number: PCT/CN2022/130953
(87) International publication number: WO 2023/098439

(57) **Abstract**

A pyrazole derivative, and a preparation method therefor and a use thereof in medicine. In particular, the present invention relates to a PRMT5 inhibitor having a structure shown in formula (I), a preparation method therefor, a pharmaceutical composition containing same, a use thereof as a PRMT5 inhibitor, and a use thereof in treatment and/or prevention of a PRMT5-mediated disease.

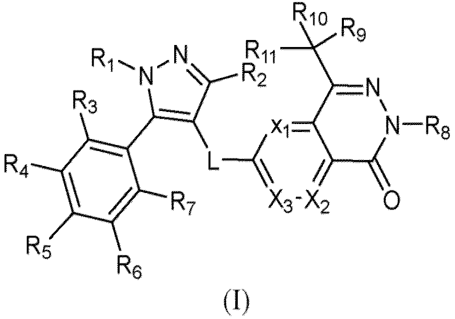

## Description

### TECHNICAL FIELD

The present invention belongs to the field of pharmaceutical synthesis, and particularly relates to a pyrazole derivative, and preparation method therefor and use thereof in medicine.

### BACKGROUND

Epigenetic gene regulation is an important biological regulatory mechanism for protein synthesis and cell differentiation, and plays an important role in many human diseases.

Epigenetic regulation involves regulation of inheritable genetic material without altering its nucleic acid sequence. Generally, epigenetic regulation is a selective and reversible modification (such as methylation) of DNA and proteins (such as histones) to control the transition between transcriptionally active state and inactive state in chromatin conformation. Modifications of these covalent bonds can be controlled by enzymes, such as methyltransferases (e.g., PRMT5), many of which are associated with specific gene changes in many human pathogenic genes. PRMT5 plays an important role in many diseases such as tumors, metabolic diseases and hematological diseases.

Homozygous deletion of tumor suppressor genes is the driver of tumors and often leads to deletions of passenger genes near the suppressor genes. The deletion of these passenger genes creates tumor cell-specific weaknesses that can be targeted by targeted therapies. Homozygous deletion of chromosome 9p21 locus, including the well-known tumor suppressor gene CDKN2A, occurs in 15% of tumors and often contains deletion of the passenger gene MTAP. MTAP is a key enzyme in the methionine and adenine recycling pathways. The deletion of MTAP leads to the accumulation of its substrate, MTA. MTA and S-adenosylmethionine (SAM) are structurally similar, and the latter is a methyl substrate donor of the type II methyltransferase PRMT5. Due to the increase in MTA levels caused by MTAP deletion, it will selectively compete with SAM for binding of PRMT5, leaving methyltransferase in an inactivation state and more likely to be affected by PRMT5 inhibition. shRNA screening of a wide range of tumor cell lines across many different genomic ranges has shown a correlation between MTAP deletion and dependence of the cell line on PRMT5, thus putting the impact of this metabolic susceptibility in the spotlight. However, PRMT5 is a very important gene for cells, and studies on conditional knockout of PRMT5 or siRNA knockout suggest that inhibition of PRMT5 in normal tissues will have significant side effects. (e.g., cytopenia, infertility, decreased skeletal muscle, myocardial hypertrophy, etc.). Therefore, there is a need for new strategies to apply and explore this metabolic susceptibility to selectively target PRMT5 in MTAP-deleted tumors while avoiding its effect on PRMT5 in normal tissues (MTAP wild-type).

Small-molecule inhibitors targeting PRMT5 that works together with MTA can selectively target PRMT5 only in the MTA-bound state, and this PRMT5 is only enriched in MTAP-deleted tumor cells. Therefore, PRMT5 will not be targeted when MTA levels are very low in normal cells with intact MTAP, thus providing a better treatment window.

### SUMMARY

The objective of the present invention is to provide a pyrazole derivative, a preparation method therefor and a pharmaceutical use thereof. The series of compounds of the present invention have a strong inhibitory effect on PRMT5 and can be widely used in the preparation of drugs for the treatment and/or prevention of PRMT5-mediated diseases, thereby promising the development of a new generation of PRMT5 inhibitors.

The first aspect of the present invention provides a compound of formula (I), a stereoisomer or pharmaceutically acceptable salt thereof: 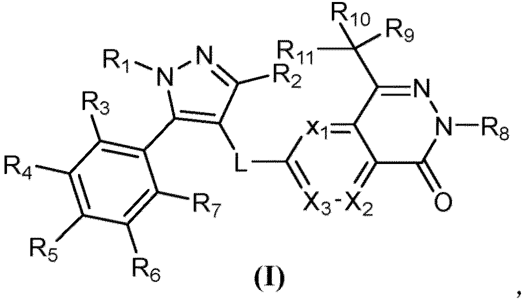
wherein, L is a bond, O, S, C(O), C(O)O, C(O)NH, CH=CH, C=C, S(O), S(O)₂, NR₁₂, S(O)₂NH or CR₁₃R₁₄;
X₁, X₂ and X₃ are each independently N or CR₁₅;
R₁ is selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, and above groups are further optionally and independently substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
R₂ is selected from the group consisting of hydrogen, deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
R₃, R₄, R₅, R₆ and R₇ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, or, two adjacent substituents of R₃, R₄, R₅, R₆ and R₇, together with a moiety to which they are directly attached thereto, form 4-8 membered carbocyclic ring, 4-8 membered heterocycle, 6-8 membered aromatic ring or 5-8 membered heteroaromatic ring, and above groups are further optionally and independently substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkylS(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
Rs is selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, and above groups are further optionally and independently substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
R₉ is selected from the group consisting of hydrogen, deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
R₁₀ and R₁₁ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, or, R₁₀ and R₁₁, together with a carbon atom directly attached thereto, form C(O), C₃₋₁₀ cycloalkyl, 3-10 membered heterocyclyl, C₆₋₁₀ aryl or 5-8 membered heteroaryl, and above groups are further optionally and independently substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkylS(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
R₁₂ is selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, and above groups are further optionally and independently substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
R₁₃ and R₁₄ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, or, R₁₃ and R₁₄, together with a carbon atom directly attached thereto, form C(O), C₃₋₁₀ cycloalkyl, 3-10 membered heterocyclyl, C₆₋₁₀ aryl or 5-8 membered heteroaryl, and above groups are further optionally and independently substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkylS(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
each R₁₅ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, and above groups are further optionally and independently substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, provided that when R₁₅ is hydrogen, at least one of R₃, R₄, R₅, R₆ and R₇ is 3-8 membered nitrogen-containing heterocyclyl, and the nitrogen atom is attached to the benzene ring;
each R₁₆ is independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₉, -C₀₋₈ alkyl-C(O)OR₂₀, -C₀₋₈ alkyl-C(O)R₂₁ or -C₀₋₈ alkyl-C(O)NR₂₂R₂₃, and above groups are further optionally substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
each R₁₇ and each R₁₈ are independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl or 5-10 membered heteroaryl, or, R₁₇ and R₁₈, together with a sulfur atom directly attached thereto, form 3-10 membered heterocyclyl, and above groups are further optionally substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, - C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
each R₁₉ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₂₂R₂₃, and above groups are further optionally and independently substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
each R₂₀ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, and above groups are further optionally and independently substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
each R₂₁ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocyclyloxy, C₆₋₁₀ alkyl, C₆₋₁₀ aryloxy, 5-10 membered heteroaryl, 5-10 membered heteroaryloxy and -NR₂₂R₂₃, and above groups are further optionally and independently substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
each R₂₂ and each R₂₃ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₁₀ alkanoyl, and above groups are further optionally and independently substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkylamino, di-C₁₋₁₀ alkylamino and C₁₋₁₀ alkanoyl, or, R₂₂ and R₂₃, together with a nitrogen atom directly attached thereto, form 4-10 membered heterocyclyl or 5-10 membered heteroaryl, and the 4-10 membered heterocyclyl or 5-10 membered heteroaryl is further optionally substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkylamino, di-C₁₋₁₀ alkylamino and C₁₋₁₀ alkanoyl; and
each r is independently 0, 1, or 2.

As a more preferred embodiment, in the compound of formula (I), the stereoisomer or pharmaceutically acceptable salt thereof, L is a bond, O, S, C(O), S(O), S(O)₂, NR₁₂ or CR₁₃R₁₄;
X₁, X₂ and X₃ are each independently N or CR₁₅;
R₁ is selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, and 5-8 membered heteroaryl, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
R₂ is selected from the group consisting of hydrogen, deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
R₃, R₄, R₅, R₆ and R₇ are each independently selected from the group consisting of hydrogen, deuterium, halogen, cyano, nitro, azido, C₁₋₄ alkyl, C₂₋₄ alkenyl, C₂₋₄ alkynyl, C₃₋₈ cycloalkyl, 3-8 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, or, two adjacent substituents of R₃, R₄, R₅, R₆ and R₇, together with a moiety to which they are directly attached thereto, form 5-8 membered carbocyclic ring, 5-8 membered heterocycle, 6 membered aromatic ring or 5-6 membered heteroaromatic ring, and above groups are further optionally and independently substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -C₀₋₄ alkyl-SF₅, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
Rs is selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, and 5-8 membered heteroaryl, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
R₉ is selected from the group consisting of hydrogen, deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
R₁₀ and R₁₁ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, or, R₁₀ and R₁₁, together with a carbon atom directly attached thereto, form C(O), C₃₋₆ cycloalkyl, 3-6 membered heterocyclyl, C₆₋₈ aryl or 5-6 membered heteroaryl, and above groups are further optionally and independently substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
R₁₂ is selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, and 5-8 membered heteroaryl, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
R₁₃ and R₁₄ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, or, R₁₃ and R₁₄, together with a carbon atom directly attached thereto, form C(O), C₃₋₆ cycloalkyl, 3-6 membered heterocyclyl, C₆₋₈ aryl or 5-6 membered heteroaryl, and above groups are further optionally and independently substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
each R₁₅ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₆)R₁₇, - C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkyl-S(O)ᵣR₁₉, - C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, and above groups are further optionally and independently substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, provided that when R₁₅ is hydrogen, at least one of R₃, R₄, R₅, R₆ and R₇ is 3-8 membered nitrogen-containing heterocyclyl, and the nitrogen atom is attached to the benzene ring;
wherein, R₁₆, R₁₇, R₁₈, R₁₉, R₂₀, R₂₁, R₂₂, R₂₃ and r are defined as in the compound of formula (I).

As a more preferred embodiment, in the compound of formula (I), the stereoisomer or pharmaceutically acceptable salt thereof, each R₁₆ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -S(O)ᵣR₁₉, -C(O)OR₂₀, -C(O)R₂₁ or -C(O)NR₂₂R₂₃, and above groups are further optionally substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₆)R₁₇, - N=S(O)R₁₇R₁₈, -N=SR₁₇R₁₈, -O-S(O)₂R₁₉, -S(O)ᵣR₁₉, -P(O)(OR₂₀)R₁₉, -O-R₂₀, -C(O)OR₂₀, - C(O)SR₂₀, -S-C(O)R₂₁, -C(O)R₂₁, -O-C(O)R₂₁, -NR₂₂R₂₃, -C(=NR₂₂)R₂₁, -N(R₂₂)-C(=NR₂₃)R₂₁, - C(O)NR₂₂R₂₃ and yl-N(R₂₂)-C(O)R₂₁;
each R₁₇ and each R₁₈ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl or 5-8 membered heteroaryl, or, R₁₇ and R₁₈, together with a sulfur atom directly attached thereto, form 4-6 membered heterocyclyl, and above groups are further optionally substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₆)R₁₇, -N=S(O)R₁₇R₁₈, -N=SR₁₇R₁₈, -O-S(O)₂R₁₉, -S(O)ᵣR₁₉, -P(O)(OR₂₀)R₁₉, -O-R₂₀, -C(O)OR₂₀, -C(O)SR₂₀, -S-C(O)R₂₁, -C(O)R₂₁, -O-C(O)R₂₁, -NR₂₂R₂₃, -C(=NR₂₂)R₂₁, - N(R₂₂)-C(=NR₂₃)R₂₁, -C(O)NR₂₂R₂₃ and -N(R₂₂)-C(O)R₂₁;
each R₁₉ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, 5-8 membered heteroaryl and -NR₂₂R₂₃, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
each R₂₀ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl, and 5-8 membered heteroaryl, and above groups are further optionally and independently substituted by one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
each R₂₁ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃, and above groups are further optionally and independently substituted by one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
each R₂₂ and each R₂₃ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₄ alkanoyl, and above groups are further optionally and independently substituted by one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, mono-C₁₋₄ alkylamino, di-C₁₋₄ alkylamino and C₁₋₄ alkanoyl, or, R₂₂ and R₂₃, together with a nitrogen atom directly attached thereto, form 4-6 membered heterocyclyl or 5-6 membered heteroaryl, and the 4-6 membered heterocyclyl or 5-6 membered heteroaryl is further optionally substituted by one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, mono-C₁₋₄ alkylamino, di-C₁₋₄ alkylamino and C₁₋₄ alkanoyl; and
each r is independently 0, 1, or 2.

As a further preferred embodiment, in the compound of formula (I), the stereoisomer or pharmaceutically acceptable salt thereof, the compound of formula (I) is a compound with the structure shown as formula (II): 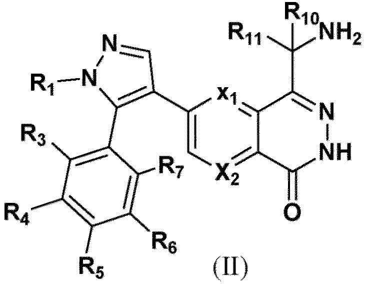
wherein X₁ is N or CH; X₂ is N or CR₁₅;
R₁ is selected from the group consisting of hydrogen, deuterium, C₁₋₄ alkyl, C₃₋₆ cycloalkyl, 3-6 membered heterocyclyl, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
R₃, R₄, R₅, R₆ and R₇ are each independently selected from the group consisting of hydrogen, deuterium, halogen, cyano, C₁₋₄ alkyl, C₂₋₄ alkenyl, C₂₋₄ alkynyl, C₃₋₈ cycloalkyl, 3-8 membered heterocyclyl, C₆₋₈ aryl, 5-8 membered heteroaryl, -C₀₋₄ alkyl-SF₅, -C₀₋₄ alkyl-S(O)(=N-R₁₆)R₁₇, - C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkyl-S(O)ᵣR₁₉, - C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, or, two adjacent substituents of R₃, R₄, R₅, R₆ and R₇, together with a moiety to which they are directly attached thereto, form 5-8 membered carbocyclic ring, 5-8 membered heterocycle, 6 membered aromatic ring or 5-6 membered heteroaromatic ring, and above groups are further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkyl-S(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, - C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
R₁₀ and R₁₁ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SFs, -C₀₋₄ alkyl-O-R₂₀, -C₀₋₄ alkyl-C(O)OR₂₀, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(O)NR₂₂R₂₃ and -C₀₋₄ alkyl-N(R₂₂)-C(O)R₂₁, or, R₁₀ and R₁₁, together with a carbon atom directly attached thereto, form C(O), C₃₋₆ cycloalkyl, 3-6 membered heterocyclyl, C₆₋₈ aryl or 5-6 membered heteroaryl, and above groups are further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SFs, -C₀₋₄ alkyl-O-R₂₀, -C₀-₄ alkyl-C(O)OR₂₀, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(O)NR₂₂R₂₃ and -C₀₋₄ alkyl-N(R₂₂)-C(O)R₂₁;
R₁₅ is selected from the group consisting of hydrogen, deuterium, cyano, C₂₋₄ alkenyl, C₂₋₄ alkynyl, C₃₋₈ cycloalkyl, 3-8 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkyl-S(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, and above groups are further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -C₀₋₄ alkyl-SFs, -C₀₋₄ alkyl-O-R₂₀, -C₀₋₄ alkyl-C(O)OR₂₀, -C₀₋₄ alkyl-C(O)R₂₁, -C₀₋₄ alkyl-O-C(O)R₂₁, - C₀₋₄ alkyl-NR₂₂R₂₃, -C₀₋₄ alkyl-C(O)NR₂₂R₂₃ and -C₀₋₄ alkyl-N(R₂₂)-C(O)R₂₁, provided that when R₁₅ is hydrogen, at least one of R₃, R₄, R₅, R₆ and R₇ is 3-8 membered nitrogen-containing heterocyclyl, and the nitrogen atom is attached to the benzene ring;
wherein, R₁₆, R₁₇, R,₈, R₁₉, R₂₀, R₂₁, R₂₂, R₂₃ and r are defined as in the compound of formula (I).

As a further preferred embodiment, in the compound of formula (I), the stereoisomer or pharmaceutically acceptable salt thereof, the compound of formula (I) is a compound with the structure shown as formula (III): 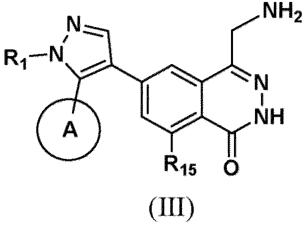 wherein ring A is of the following structure: 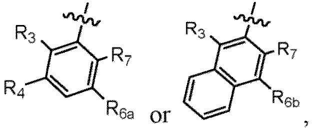
R₁ is selected from the group consisting of hydrogen, deuterium, methyl, ethyl, isopropyl, and cyclopropyl;
each R₃ and each R₇ are independently selected from the group consisting of hydrogen, deuterium, fluorine, chlorine, cyano, C₁₋₄ alkyl, C₃₋₈ cycloalkyl, 3-8 membered heterocyclyl, hydroxy, methoxy, ethoxy, isopropoxy, C₃₋₈ cycloalkoxy, 3-8 membered heterocyclyloxy, methylthio, ethylthio, carboxyl, methoxycarbonyl, ethoxycarbonyl, isopropoxycarbonyl, acetyl, acetoxy, amino, dimethylamino, acetamino, and carbamoyl;
R₁₅ is selected from the group consisting of cyano, ethynyl, cyclopropyl, and hydrogen, and
**when R₁₅ is selected from the group consisting of cyano, ethynyl, and cyclopropyl and** ring A is 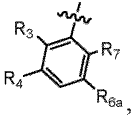 R₄ and R₆ₐ are each independently selected from the group consisting of halogen, C₁₋₄ alkoxy, C₃₋₈ cycloalkoxy, 3-8 membered heterocyclyl, and 3-8 membered heterocyclyloxy, and the C₁₋₄ alkoxy, C₃₋₈ cycloalkoxy, 3-8 membered heterocyclyl, or 3-8 membered heterocyclyloxy is each further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀, -C(O)OR₂₀, -C(O)R₂₁, - O-C(O)R₂₁, -NR₂₂R₂₃, -C(O)NR₂₂R₂₃ and -N(R₂₂)-C(O)R₂₁;
**when R₁₅ is selected from the group consisting of cyano, ethynyl and cyclopropyl,** and ring A is 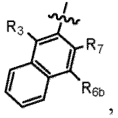
R_{6b} is selected from the group consisting of hydrogen, deuterium, fluorine, chlorine, cyano, C₁₋₄ alkyl, C₃₋₈ cycloalkyl, 3-8 membered heterocyclyl, hydroxy, methoxy, ethoxy, isopropoxy, C₃₋₈ cycloalkoxy, 3-8 membered heterocyclyloxy, carboxyl, amino, and dimethylamino;
**when R₁₅ is hydrogen** and ring A is 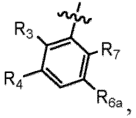 R₄ and R₆ₐ are each independently selected from halogen, C₁₋₄ alkoxy, C₃₋₈ cycloalkoxy, 3-8 membered heterocyclyl or 3-8 membered heterocyclyloxy, and the C₁₋₄ alkoxy, C₃₋₈ cycloalkoxy, 3-8 membered heterocyclyl or 3-8 membered heterocyclyloxy are each further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀, -C(O)OR₂₀, -C(O)R₂₁, - O-C(O)R₂₁, -NR₂₂R₂₃, -C(O)NR₂₂R₂₃ and -N(R₂₂)-C(O)R₂₁, provided that at least one of R₄ and R₆ₐ is 3-8 membered nitrogen-containing heterocyclyl, the nitrogen atom is attached to the benzene ring, and the 3-8 membered nitrogen-containing heterocyclyl is further optionally substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀, -C(O)OR₂₀, -C(O)R₂₁, -O-C(O)R₂₁, -NR₂₂R₂₃, -C(O)NR₂₂R₂₃ and -N(R₂₂)-C(O)R₂₁;
wherein, R₂₀, R₂₁, R₂₂, and R₂₃ are defined as in the compound of formula (I).

As a more further preferred embodiment, in the compound of formula (I), the stereoisomer or pharmaceutically acceptable salt thereof, the compound of formula (I) is a compound with the structure shown as formula (IVa): 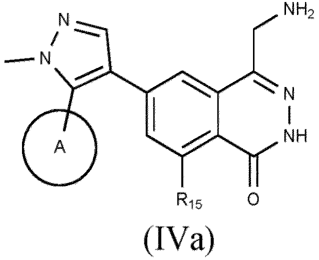
wherein R₁₅ is cyano, ethynyl, or cyclopropyl;
ring A is of the following structure: 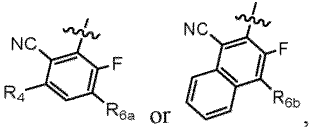
R₄ and R₆ₐ are each independently selected from the group consisting of halogen, C₁₋₄ alkoxy, C₃₋₈ cycloalkoxy, 3-8 membered heterocyclyl, or 3-8 membered heterocyclyloxy, and the C₁₋₄ alkoxy, C₃₋₈ cycloalkoxy, 3-8 membered heterocyclyl, or 3-8 membered heterocyclyloxy are further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀ and -NR₂₂R₂₃;
R_{6b} is selected from the group consisting of hydrogen, deuterium, fluorine, chlorine, cyano, C₁₋₄ alkyl, C₃₋₈ cycloalkyl, 3-8 membered heterocyclyl, hydroxy, methoxy, ethoxy, isopropoxy, C₃₋₈ cycloalkoxy, 3-8 membered heterocyclyloxy, carboxyl, amino, and dimethylamino; wherein, R₂₀, R₂₂ and R₂₃ are defined as in the compound of formula (I).

As a more further preferred embodiment, in the compound of formula (I), the stereoisomer or pharmaceutically acceptable salt thereof, R₄ and R₆ₐ are each independently selected from the group consisting of fluorine, chlorine, cyclopropoxy, cyclobutoxy or 3-8 membered nitrogen-containing heterocyclyl, and the cyclopropoxy, cyclobutoxy or 3-8 membered nitrogen-containing heterocyclyl is further optionally and independently substituted by one or more substituents selected from the group consisting of deuterium, fluorine, chlorine, cyano, methyl, ethyl, n-propyl, isopropyl, trifluoromethyl, difluoromethyl, trideuteriomethyl, dideuteriomethyl, vinyl, ethynyl, cyclopropyl, cyclobutyl, oxa-cyclobutyl, aza-cyclobutyl, phenyl, -SF₅, =O, hydroxy, methoxy, ethoxy, n-propyloxy, isopropyloxy, amino, mono-C₁₋₄ alkylamino and di-C₁₋₄ alkylamino;
R_{6b} is selected from the group consisting of hydrogen, deuterium, fluorine, chloride, cyano, methyl, ethyl, n-propyl, isopropyl, cyclopropyl, cyclobutyl, oxa-cyclobutyl, aza-cyclobutyl, hydroxy, methoxy, ethoxy, isopropoxy, cyclopropoxy, cyclobutoxy, carboxyl, amino, and dimethylamino.

As a more further preferred embodiment, in the compound of formula (I), the stereoisomer or pharmaceutically acceptable salt thereof, the compound of formula (I) is a compound with the structure shown as formula (IVb): 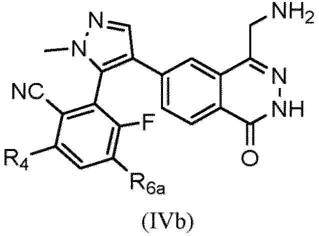
wherein R₄ and R₆ₐ are each independently selected from the group consisting of halogen, C₁₋₄ alkoxy, C₃₋₈ cycloalkoxy, 3-8 membered heterocyclyl or 3-8 membered heterocyclyloxy, and the C₁₋₄ alkoxy, C₃₋₈ cycloalkoxy, 3-8 membered heterocyclyl or 3-8 membered heterocyclyloxy is each further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀ and -NR₂₂R₂₃, provided that at least one of R₄ and R₆ₐ is 3-8 membered nitrogen-containing heterocyclyl, the nitrogen atom is attached to the benzene ring, and the 3-8 membered nitrogen-containing heterocyclyl is each further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, - O-R₂₀ and -NR₂₂R₂₃;
wherein, R₂₀, R₂₂ and R₂₃ are defined as in the compound of formula (I).

As a more further preferred embodiment, in the compound of formula (I), the stereoisomer or pharmaceutically acceptable salt thereof, R₄ and R₆ₐ are each independently selected from the group consisting of fluorine, chlorine, cyclopropoxy, cyclobutoxy and 3-8 membered nitrogen-containing heterocyclyl, and the cyclopropoxy, cyclobutoxy or 3-8 membered nitrogen-containing heterocyclyl is further optionally and independently substituted by one or more substituents selected from the group consisting of deuterium, fluorine, chlorine, cyano, methyl, ethyl, n-propyl, isopropyl, trifluoromethyl, difluoromethyl, trideuteriomethyl, dideuteriomethyl, vinyl, ethynyl, cyclopropyl, cyclobutyl, oxa-cyclobutyl, aza-cyclobutyl, phenyl, -SF₅, =O, hydroxy, methoxy, ethoxy, n-propyloxy, isopropyloxy, amino, mono-C₁₋₄ alkylamino and di-C₁₋₄ alkylamino, provided that
at least one of R₄ and R₆ₐ is 3-8 membered nitrogen-containing heterocyclyl, the nitrogen atom is attached to the benzene ring, and the 3-8 membered nitrogen-containing heterocyclyl is each further optionally and independently substituted by one or more substituents selected from the group consisting of deuterium, fluorine, chlorine, cyano, methyl, ethyl, n-propyl, isopropyl, trifluoromethyl, difluoromethyl, trideuteriomethyl, dideuteriomethyl, vinyl, ethynyl, cyclopropyl, cyclobutyl, oxa-cyclobutyl, aza-cyclobutyl, phenyl, -SF₅, =O, hydroxy, methoxy, ethoxy, n-propyloxy, isopropyloxy, amino, mono-C₁₋₄ alkylamino and di-C₁₋₄ alkylamino.

As a more further preferred embodiment, in the compound of formula (I), the stereoisomer or pharmaceutically acceptable salt thereof, the 3-8 membered nitrogen-containing heterocyclyl is selected from the following structure:

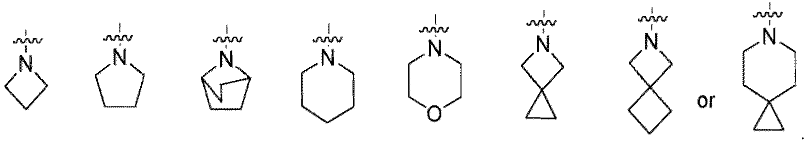

As the most preferred embodiment, the compound of formula (I), the stereoisomer or pharmaceutically acceptable salt thereof includes, but is not limited to, the following compounds:

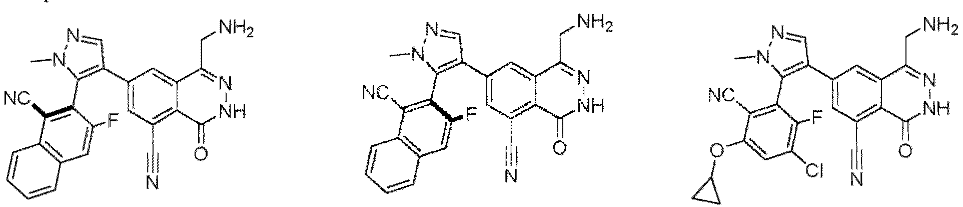

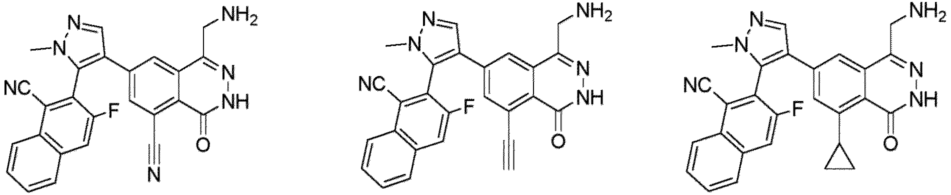

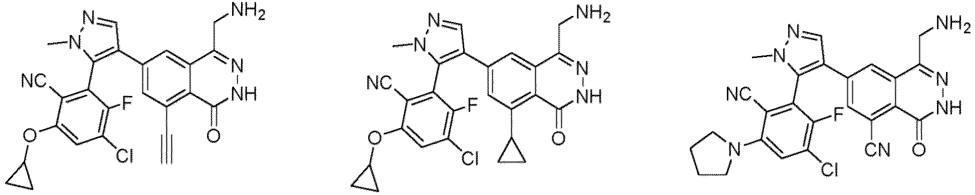

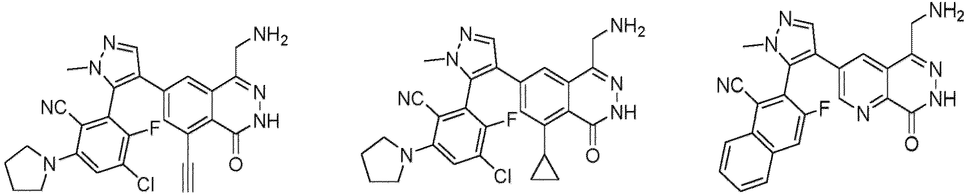

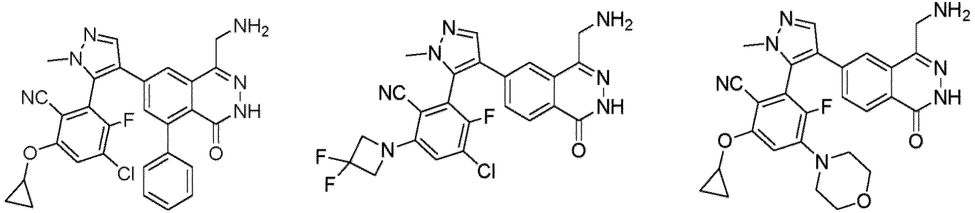

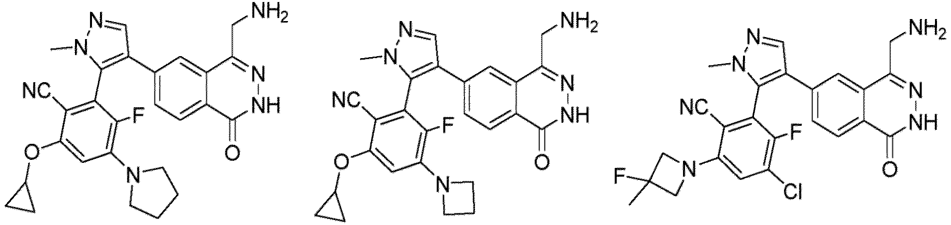

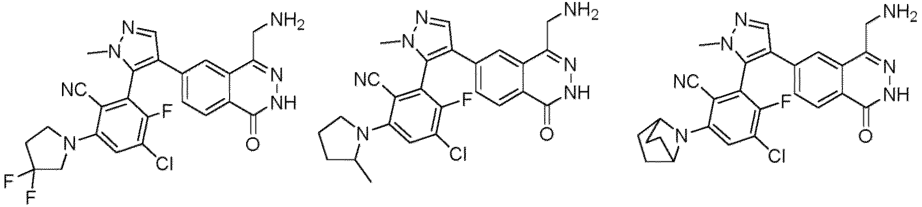

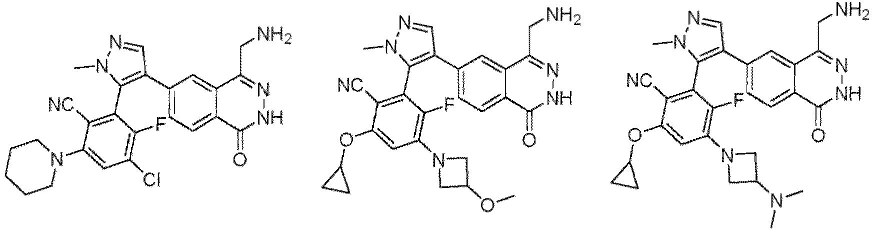

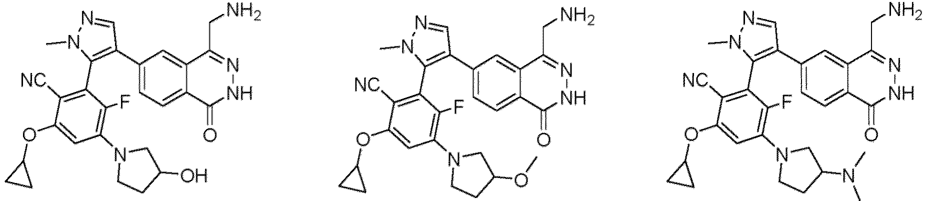

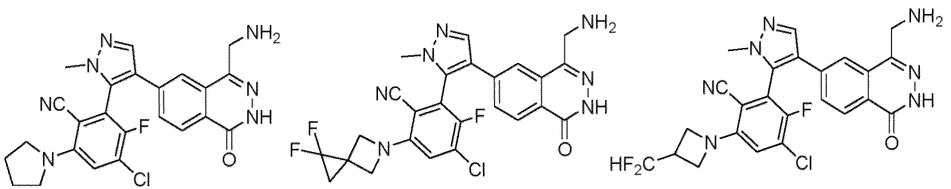

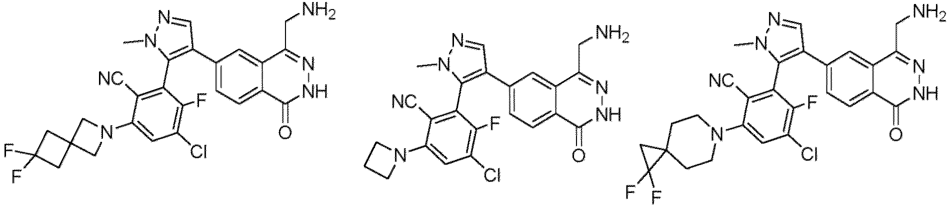

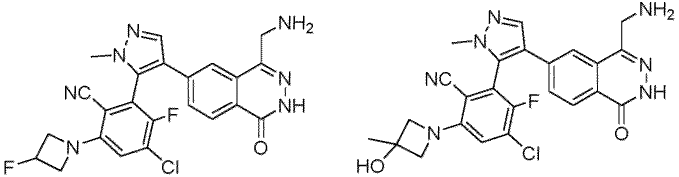

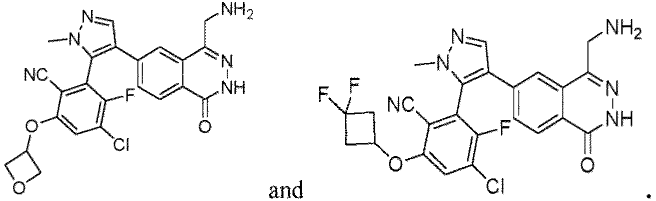

The second aspect of the present invention provides a process for preparing the compound of formula (I), the stereoisomer or pharmaceutically acceptable salt thereof, comprising the following step: The compound of formula (IA) or the salt thereof is synthesized by condensation reaction with the compound of formula (IB) or the salt thereof, to obtain the compound of formula (I) or the salt thereof, comprising the following reaction route:

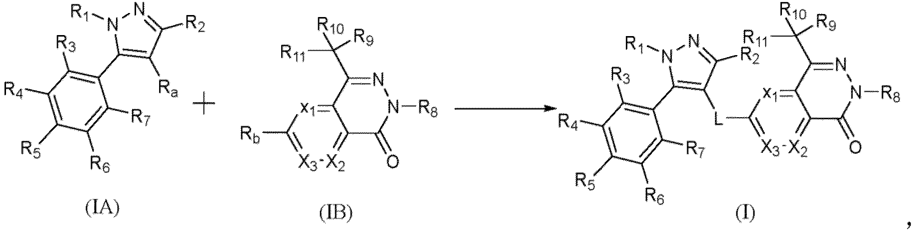

The preparation process may be achieved using a general condensation reaction in the art according to the definition of L, e.g.,
when L is a bond, Rₐ is halogen, R_{b} is -B(OH)₂ or halogen, R_{b} is halogen; 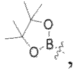 or, Rₐ is -B(OH)₂ or 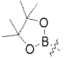
when L is O, Rₐ is hydroxy or alkoxy, R_{b} is halogen, or, Rₐ is halogen, R_{b} is hydroxy or alkoxy;
when L is S, Rₐ is sulfydryl or alkylthio, R_{b} is halogen, or, Rₐ is halogen, R_{b} is sulfydryl or alkylthio;
when L is C(O), C(O)O or C(O)NH, Rₐ is carboxyl or alkoxycarbonyl, R_{b} is halogen, hydroxy or amino, or, Rₐ is halogen, hydroxy or amino, R_{b} is carboxyl or alkoxycarbonyl;
wherein, R₁, R₂, R₃, R₄, R₅, R₆, R₇, R₈, R₉, R₁₀, R₁₁, X₁, X₂ and X₃ are defined as in the compound of formula (I).

The third aspect of the present invention provides a pharmaceutical composition comprising the compound of formula (I), the stereoisomer or pharmaceutically acceptable salt thereof, and a pharmaceutically acceptable carrier.

The fourth aspect of the present invention provides a use of the compound of formula (I), the stereoisomer or pharmaceutically acceptable salt thereof in the preparation of a medicament for treating MATP-associated cancer or tumor.

As a preferred embodiment, the tumor or cancer is selected from the group consisting of endometrial carcinoma, granulosa-theca cell tumor, Sertoli-Leydig cell tumor, germinomas, malignant teratoma, squamous cell carcinoma, intraepithelial cancer, adenocarcinoma, fibrosarcoma, melanoma, clear cell carcinoma, squamous cell carcinoma, botryoid sarcoma, fallopian tube cancer, adenocarcinoma, nephroblastoma, lymphoma, leukemia, bladder cancer, squamous cell carcinoma, transitional cell carcinoma, adenocarcinoma, prostate cancer, seminoma, teratoma, embryonal carcinoma, teratoma, choriocarcinoma, sarcoma, mesenchymal cell carcinoma, fibroma, fibroadenoma, adenomatoid tumor, lipoma, liver cancer, cholangiocarcinoma, hepatoblastoma, hemangiosarcoma, hepatocellular adenoma, hemangioma, gallbladder cancer, ampullary carcinoma, cholangiocarcinoma, malignant melanoma, basal cell carcinoma, squamous cell carcinoma, Kaposi's sarcoma, moles, dysplastic nevus, lipomyoma, hemangioma, acute and chronic myeloid leukemia, acute lymphoblastic leukemia, chronic lymphoblastic leukemia, myeloproliferative disorder, multiple myeloma, myelodysplastic syndrome, Hodgkin's disease, non-Hodgkin's lymphoma, osteosarcoma, fibrosarcoma, malignant fibrous histiocytoma, chondrosarcoma, Ewing's sarcoma, malignant lymphoma, multiple myeloma, malignant giant cell tumor chordoma, osteochondroma, benign chondroma, chondroblastoma, chondromyxoid fibroma, osteoid osteoma, giant cell tumor, angiosarcoma, fibrosarcoma, rhabdomyosarcoma, liposarcoma, myxoma, rhabdomyoma, fibroma, lipomyoma and teratoma, bronchial carcinoma, alveolar carcinoma, bronchial adenoma, sarcoma, lymphoma, chondromatoid hamartoma, mesothelioma squamous cell carcinoma, adenocarcinoma, leiomyosarcoma, lymphoma, gastric cancer, lymphoma, leiomyosarcoma, ductal adenocarcinoma, insulinoma, glucagonoma, gastrinoma, carcinoid tumor, serpentine tumor, adenocarcinoma, lymphoma, carcinoid tumor, Kaposis sarcoma, leiomyoma, hemangioma, lipomyoma, neurofibroma, fibroma, adenocarcinoma of large intestine, tubular adenoma, villous adenoma, hamartoma, leiomyoma, osteoma of skull, hemangioma, granuloma, xanthoma, osteitis deformans, meningioma, meningeal sarcoma, gliomatosis, astrocytoma, medulloblastoma, glioma, ependymoma, germ cell tumor, glioblastoma multiforme, oligodendroglioma, neurilemmoma, retinoblastoma, congenital tumor, spinal cord neurofibroma, meningioma, glioma and sarcoma.

As a more preferred embodiment, the cancer or tumor is selected from breast cancer, pancreatic cancer, skin cancer, bladder cancer, liver cancer, or head and neck cancer.

The present invention also relates to the compound of formula (I), the stereoisomer or pharmaceutically acceptable salt thereof, for use as a PRMT5 inhibitor drug.

The present invention also relates to a use of the compound of formula (I), the stereoisomer or pharmaceutically acceptable salt thereof in the preparation of a medicament for treating and/or preventing PRMT5-mediated diseases.

The present invention also relates to a method for treating and/or preventing PRMT5-mediated diseases, comprising administering to a patient in need a therapeutically effective amount of the compound of formula (I), the stereoisomer or pharmaceutically acceptable salt thereof.

### DETAILED DESCRIPTION OF EMBODIMENTS

After an extensive and intensive research , the inventors of the present invention develop a PRMT5 inhibitor with the structure of formula (I) for the first time. The series of compounds of the present invention can be widely applied in the preparation of drugs for treating and/or preventing PRMT5-mediated diseases, and are expected to be developed into a new generation of PRMT5 inhibitors. The present invention is achieved on this basis.

Detailed description: unless otherwise stated or specified, the following terms used in the specification and claims have the following meanings.

"Alkyl" refers to linear or branched saturated aliphatic alkyl groups, preferably including a linear or branched alkyl having 1 to 10 or 1 to 6 carbon atoms or 1 to 4 carbon atoms, including but not limited to methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, etc. "C₁₋₁₀ alkyl" means a linear or branched alkyl containing 1 to 10 carbon atoms, "C₁₋₄ alkyl" means a linear or branched alkyl containing 1 to 4 carbon atoms, "C₀₋₈ alkyl" means a linear or branched alkyl containing 0 to 8 carbon atoms, and "C₀₋₄ alkyl" means a linear or branched alkyl containing 0 to 4 carbon atoms.

Alkyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkylS-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"Cycloalkyl" or "carbocyclic ring" refers to monocyclic or polycyclic hydrocarbon substituents that are saturated or partially unsaturated, wherein the partially unsaturated cyclic hydrocarbon refers to a cyclic hydrocarbon that may contain one or more (preferably 1, 2 or 3) double bonds, but none of the rings have a fully conjugated π-electron system. Cycloalkyl may be monocyclic cycloalkyl or polycyclic cycloalkyl, preferably including a cycloalkyl containing 3 to 12 or 3 to 8 or 3 to 6 carbon atoms. For example, "C₃₋₁₂ cycloalkyl" means a cycloalkyl having 3 to 12 carton atoms, "C₄₋₈ cycloalkyl" means a cycloalkyl having 4 to 8 carton atoms, "C₃₋₈ cycloalkyl" means a cycloalkyl having 3 to 8 carton atoms, and "C₃₋₆ cycloalkyl" means a cycloalkyl having 3 to 6 carton atoms, wherein:
monocyclic cycloalkyl includes but is not limited to cyclopropyl, cyclobutyl, cyclopentyl, cyclopentenyl, cyclohexyl, cyclohexenyl, cyclohexadienyl, cycloheptyl, cycloheptarienyl, cyclooctyl, etc.

Polycyclic cycloalkyl includes spirocycloalkyl, fused cycloalkyl and bridged cycloalkyl. "Spirocycloalkyl" refers to a polycyclic group in which a carton atom (called spiro-atom) is shared among monocyclic rings, wherein these rings may contain one or more (preferably 1, 2 or 3) double bonds, but none of them have a fully conjugated π-electron system. According to the number of the spiro-atoms shared among the rings, the spirocycloalkyl may be monospirocycloalkyl, bispirocycloalkyl or polyspirocycloalkyl, including but not limited to:

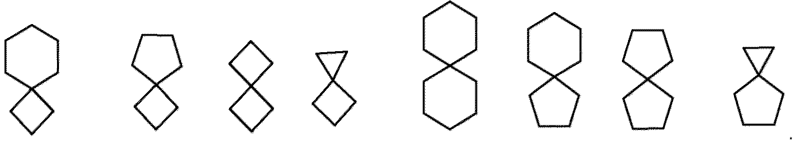

"Fused cycloalkyl" refers to an all-carbon polycyclic group in which each ring shares a pair of adjacent carbon atoms with other rings in the system, wherein one or more of the rings may contain one or more (preferably 1, 2 or 3) double bonds, but none of them have a fully conjugated π-electron system. According to the number of formed rings, the fused cycloalkyl may be bicyclic, tricyclic, tetracyclic or polycyclic, including but not limited to:

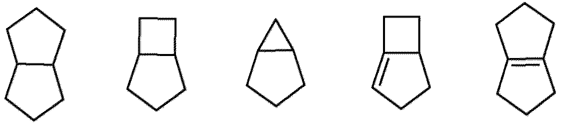

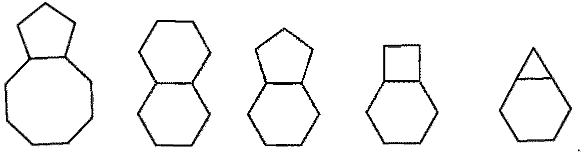

"Bridged cycloalkyl" refers to an all-carbon polycyclic group in which any two rings share two carbon atoms that are not directly connected to each other, wherein these rings may contain one or more (preferably 1, 2 or 3) double bonds, but none of them have a fully conjugated π-electron system. According to the number of formed rings, the bridged cycloalkyl may be bicyclic, tricyclic, tetracyclic or polycyclic, including but not limited to:

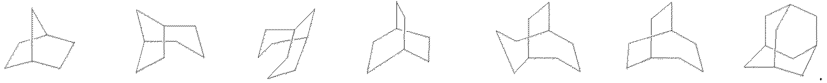

The cycloalkyl ring may be fused to an aryl, heteroaryl or heterocycloalkyl ring, wherein the ring attached to the parent structure is cycloalkyl, which includes but is not limited to indenyl, tetrahydronaphthyl, benzocycloheptyl, etc.

"Cycloalkyl" or "carbocycle" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"Heterocyclyl" or "heterocycle" refers to a monocyclic or polycyclic hydrocarbon substituent that is saturated or partially unsaturated, wherein the partially unsaturated cyclic hydrocarbon refers to a cyclic hydrocarbon that may contain one or more (preferably 1, 2 or 3) double bonds, but none of the rings have a fully conjugated π-electron system, wherein one or more (preferably 1, 2, 3 or 4) of the rings atoms in heterocyclyl are heteroatoms selected from N, O, N•O or S(O)ᵣ (wherein r is an integer of 0, 1 or 2), excluding ring portions of -O-O-, -O-S- or -S-S-, and the remaining ring atoms are carton atoms. It preferrably includes heterocyclyl containing 3 to 12 or 3 to 8 or 3 to 6 ring atoms. For example, "3-6 membered heterocyclyl" means a heterocyclyl containing 3 to 6 ring atoms, "3-8 membered heterocyclyl" means a heterocyclyl containing 3 to 8 ring atoms, "4-8 membered heterocyclyl" means a heterocyclyl containing 4 to 8 ring atoms, "4-10 membered heterocyclyl" means a heterocyclyl containing 4 to 10 ring atoms, "5-8 membered heterocyclyl" means a heterocyclyl containing 5 to 8 ring atoms, and "3-12 membered heterocyclyl" means a heterocyclyl containing 3 to 12 ring atoms.

Monocyclic heterocyclyl includes but is not limited to pyrrolidinyl, piperidinyl, piperazinyl, morpholinyl, thiomorpholinyl, homopiperazinyl, oxa-cyclobutyl, tetrahydrofuranyl, etc.

Polycyclic heterocyclyl includes spiroheterocyclyl, fused heterocyclyl, and bridged heterocyclyl. "Spiroheterocyclyl" refers to a polycyclic heterocyclyl group in which an atom (called spiro-atom) is shared among monocyclic rings, wherein one or more (preferably 1, 2, 3 or 4) of the ring atoms are heteroatoms selected from N, O, N•O, or S(O)ᵣ (wherein r is an integer of 0, 1 or 2), and the remaining ring atoms are carbon atoms. These rings may contain one or more (preferably 1, 2 or 3) double bonds, but none of them have a fully conjugated π-electronic system. According to the number of sprio-atoms shared among the rings, spiroheterocyclyl may be monospiroheterocyclyl, bispiroheterocyclyl or polyspiroheterocyclyl. Spiroheterocyclyl includes but is not limited to:

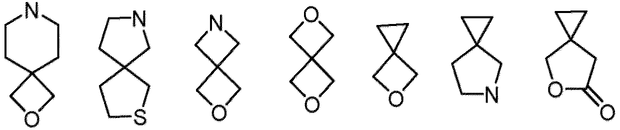

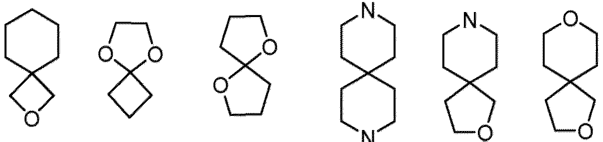

"Fused heterocyclyl" refers to a polycyclic heterocyclyl in which each ring shares a pair of adjacent atoms with the other rings in the system, wherein one or more (preferably 1, 2, 3 or 4) of the rings may contain one or more (preferably 1, 2, or 3) double bonds, but none of them have a fully conjugated π-electron system, wherein one or more (preferably 1, 2, 3, or 4) of the ring atoms are heteroatoms selected from N, O, N•O, or S(O)ᵣ (wherein r is an integer of 0, 1 or 2), and the remaining ring atoms are carbon atoms. According to the number of formed rings, the fused heterocyclyl may be bicyclic, tricyclic, tetracyclic or polycyclic, including but not limited to:

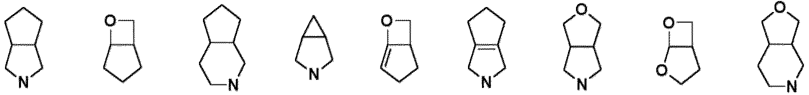

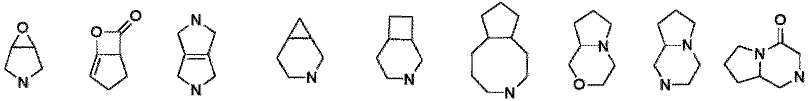

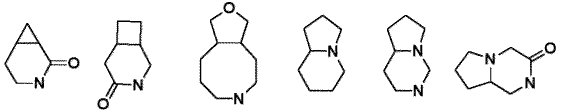

"Bridged heterocyclyl" refers to a polycyclic heterocyclyl in which any two rings share two carton atoms that are not directly attached to each other, wherein these rings may contain one or more (preferably 1, 2, or 3) double bonds, but none of them have a fully conjugated π-electron system, wherein one or more (preferably 1, 2, 3, or 4) of the ring atoms are heteroatoms selected from N, O, N•O, or S(O)ᵣ (wherein r is an integer of 0, 1 or 2), and the remaining ring atoms are carbon atoms. According to the number of formed rings, the bridged heterocyclyl may be bicyclic, tricyclic, tetracyclic or polycyclic, including but not limited to:

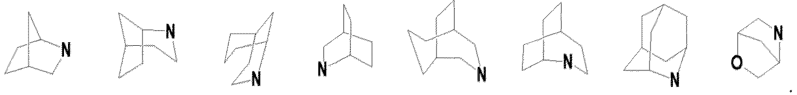

The heterocyclyl ring may be fused to an aryl, heteroaryl or cycloalkyl ring, wherein the ring attached to the parent structure is heterocyclyl, which includes but is not limited to:

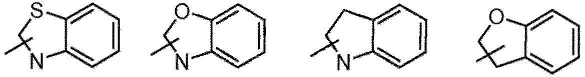

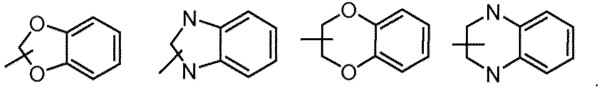

"Heterocyclyl" or "heterocycle" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"Aryl" or "aromatic ring" means an all-carbon monocyclic or fused-polycyclic group (i.e., rings that share a pair of adjacent carbon atoms) and a polycyclic group having a conjugated π-electron system (i.e., rings with adjacent pairs of carbon atoms), preferably including all-carbon aryl containing 6-10 or 6-8 carbon atoms. For example, "C₆₋₁₀ aryl" means an all-carbon aryl containing 6-10 carbon atoms, including but not limited to phenyl and naphthyl, and "C₆₋₈ aryl" means an all-carbon aryl containing 6-8 carbon atoms. The aryl ring may be fused to an heteroaryl, heterocyclyl or cycloalkyl ring, wherein the ring attached to the parent structure is aryl ring, including but not limited to:

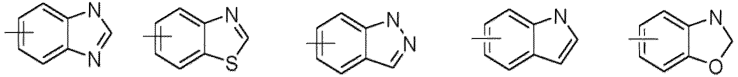

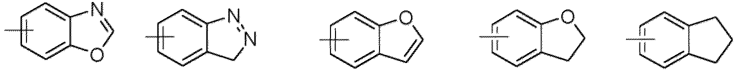

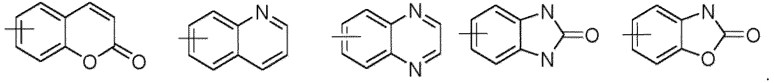

"Aryl" or "aromatic ring" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkylS-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"Heteroaryl" or "heteroaromatic ring" refers to a heteroaromatic system containing one or more (preferably 1, 2, 3 or 4) of the heteroatoms, wherein the heteroatoms include heteroatoms selected from N, O, N•O, and S(O)r (wherein r is an integer of 0, 1 or 2), preferably including a heteroaromatic system containing 5-10 or 5-8 or 5-6 ring atoms. For example, "5-8 membered heteroaryl" means a heteroaromatic system containing 5-8 ring atoms, and "5-10 membered heteroaryl" means a heteroaromatic system containing 5-10 ring atoms, including but not limited to furyl, thiophenyl, pyridyl, pyrrolyl, N-alkylpyrrolyl, pyrimidinyl, pyrazinyl, imidazolyl, tetrazolyl, etc. The heteroaryl ring may be fused to an aryl, heterocyclyl or cycloalkyl ring, wherein the ring attached to the parent structure is heteroaryl ring, including but not limited to:

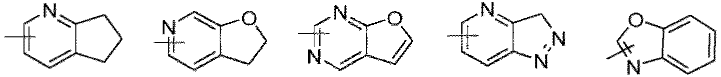

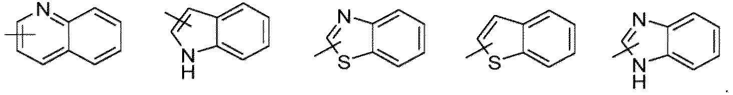

"Heteroaryl" or "heteroaromatic ring" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"Alkenyl" refers to an alkyl defined as above consisting of at least two carbon atoms and at least one carbon-carbon double bond, preferably including a linear or branched alkenyl containing 2-10 or 2-4 carbon atoms. For example, "C₂₋₁₀ alkenyl" means a linear or branched alkenyl containing 2-10 carbon atoms, and "C₂₋₄ alkenyl" means a linear or branched alkenyl containing 2-4 carbon atoms. The alkenyl includes but is not limited to vinyl, 1-propenyl, 2-propenyl, 1-,2-, or 3-butenyl, etc.

"Alkenyl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈alkyl-SF₅, -C₀₋₈ alkyl-S(O)(=N-R₁₆)R₁₇, -C₀₋₈alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"Alkynyl" refers to an alkyl defined as above consisting of at least two carbon atoms and at least one carbon-carbon triple bond, preferably including a linear or branched alkynyl containing 2-10 or 2-4 carbon atoms. For example, "C₂₋₁₀ alkynyl" means a linear or branched alkynyl containing 2-10 carbon atoms, and "C₂₋₄ alkynyl" means a linear or branched alkynyl containing 2-4 carbon atoms. The alkynyl includes but is not limited to ethynyl, 1-propynyl, 2-propynyl, 1-,2-, or 3-butynyl, etc.

"Alkynyl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈alkyl-SF₅, -C₀₋₈ alkyl-S(O)(=N-R₁₆)R₁₇, -C₀₋₈alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"Alkoxy" refers to an -O-alkyl group, wherein the alkyl is defined as above. For example, "C₁₋₁₀ alkoxy" means an alkyloxy containing 1-10 carbon atoms, "C₁₋₄ alkoxy" means an alkyloxy containing 1-4 carbon atoms, and "C₁₋₂ alkoxy" means an alkyloxy containing 1-2 carbon atoms, including but not limited to, methoxy, ethoxy, propoxy, butoxy, etc.

"Alkoxy" may be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkylS-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"Cycloalkoxy" or "cycloalkyloxy" refers to an -O-cycloalkyl group, wherein cycloalkyl is defined as above. For example, "C₃₋₁₂ cycloalkoxy" means a cycloalkyloxy containing 3-12 carbon atoms, and "C₃₋₆ cycloalkoxy" means a cycloalkyloxy containing 3-6 carbon atoms, including but not limited to cyclopropoxy, cyclobutoxy, cyclopentoxy, cyclohexoxy, etc.

"Cycloalkoxy" or "cycloalkyloxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"Heterocycloxy" or "heterocyclyloxy" refers to an -O-heterocyclyl group, wherein the heterocyclyl is defined as above, including but not limited to, azetidyloxy, oxetanyloxy, azetidyloxy, nitrogen, oxetanyloxy, etc.

"Heterocycloxy" or "heterocyclyloxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(o)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁.

"C₁₋₁₀ alkanoyl" refers to a monovalent atomic group which is obtained after a hydroxy is removed from the C₁₋₁₀ alkyl acid, and is also generally referred to as "C₀₋₉ alkyl-C(O)-". For example, "C₁ alkyl-C(O)-" refers to acetyl; "C₂ alkyl-C(O)-" refers to propanoyl; "C₃ alkyl-C(O)-" refers to butanoyl or isobutanoyl.

"-C₀₋₈ alkyl-S(O)(=N-R₁₆)R₁₇" refers to the sulfur atom in -S(O)(=N-R₁₆)R₁₇ attached to C₀₋₈ alkyl, wherein the C₀₋₈ alkyl is defined as above.

"-C₀₋₈ alkyl-N=S(O)R₁₇R₁₈" refers to the nitrogen atom in -N=S(O)R₁₇R₁₈ attached to C₀₋₈ alkyl, wherein the C₀₋₈ alkyl is defined as above.

"-C₀₋₈ alkyl-N=SR₁₇R₁₈" refers to the nitrogen atom in -N=SR₁₇R₁₈ attached to C₀₋₈ alkyl, wherein the C₀₋₈ alkyl is defined as above.

"-C₀₋₈ alkyl-O-S(O)₂R₁₉" refers to the oxygen atom in -O-S(O)₂R₁₉ attached to C₀₋₈ alkyl, wherein the C₀₋₈ alkyl is defined as above.

"-C₀₋₈ alkyl-S(O)ᵣR₁₉" refers to the sulfur atom in -S(O)ᵣR₁₉ attached to C₀₋₈ alkyl, wherein the C₀₋₈ alkyl is defined as above.

"-C₀₋₈ alkyl-P(O)(OR₂₀)R₁₉" refers to the phosphorus atom in -P(O)(OR₂₀)R₁₉ attached to C₀₋₈ alkyl, wherein the C₀₋₈ alkyl is defined as above.

"-C₀₋₈ alkyl-O-R₂₀" refers to the oxygen atom in -O-R₂₀ attached to C₀₋₈ alkyl, wherein the C₀₋₈ alkyl is defined as above.

"-C₀₋₈ alkyl-C(O)OR₂₀" refers to the carbonyl in -C(O)OR₂₀ attached to C₀₋₈ alkyl, wherein the C₀₋₈ alkyl is defined as above.

"-C₀₋₈ alkyl-C(O)SR₂₀" refers to the carbonyl in -C(O)SR₂₀ attached to C₀₋₈ alkyl, wherein the C₀₋₈ alkyl is defined as above.

"-C₀₋₈ alkyl-S-C(O)R₂₁" refers to the sulfur atom in -S-C(O)R₂₁ attached to C₀₋₈ alkyl, wherein the C₀₋₈ alkyl is defined as above.

"-C₀₋₈ alkyl-C(O)R₂₁" refers to the carbonyl in -C(O)R₂₁ attached to C₀₋₈ alkyl, wherein the C₀₋₈ alkyl is defined as above.

"-C₀₋₈ alkyl-O-C(O)R₂₁" refers to the oxygen atom in -O-C(O)R₂₁ attached to C₀₋₈ alkyl, wherein the C₀₋₈ alkyl is defined as above.

"-C₀₋₈ alkyl-NR₂₂R₂₂" refers to the nitrogen atom in -NR₂₁R₂₂ attached to C₀₋₈ alkyl, wherein the C₀₋₈ alkyl is defined as above.

"-C₀₋₈ alkyl-C(=NR₂₂)R₂₁" refers to the carbon atom in -C(=NR₂₂)R₂₁ attached to C₀₋₈ alkyl, wherein the C₀₋₈ alkyl is defined as above.

"-C₀₋₈ alkyl-N(R₂₂)-C(=NR₂₃)R₂₁" refers to the nitrogen atom in -N(R₂₂)-C(=NR₂₃)R₂₁ attached to C₀₋₈ alkyl, wherein the C₀₋₈ alkyl is defined as above.

"-C₀₋₈ alkyl-C(O)NR₂₂R₂₃" refers to the carbonyl in -C(O)NR₂₂R₂₃ attached to C₀₋₈ alkyl, wherein the C₀₋₈ alkyl is defined as above.

"-C₀₋₈ alkyl-N(R₂₂)-C(O)R₂₁" refers to the nitrogen atom in -N(R₂₂)-C(O)R₂₁ attached to C₀₋₈ alkyl, wherein the C₀₋₈ alkyl is defined as above.

"C₁₋₁₀ haloalkyl" refers to an alkyl having 1-10 carbon atoms in which hydrogens on the alkyl are optionally substituted by a fluorine, chlorine, bromine and iodine atom, including but not limited to difluoromethyl, dichloromethyl, dibromomethyl, trifluoromethyl, trichloromethyl, tribromomethyl, etc.

"C₁₋₁₀ haloalkoxy" refers to an alkoxy having 1-10 carbon atoms in which hydrogens on the alkyl are optionally substituted by a fluorine, chlorine, bromine, and iodine atom, including but not limited to difluoromethoxy, dichloromethoxy, dibromomethoxy, trifluoromethoxy, trichloromethoxy, tribromomethoxy, etc.

"C₁₋₁₀ deuterioalkyl" refers to an alkyl having 1-10 carbon atoms in which hydrogens on the alkyl are optionally substituted by a deuterium atom, including but not limited to monodeuterium, dideuterium, trideuterium, etc.

"Halogen" refers to fluorine, chlorine, bromine, or iodine; "n-BuLi" refers to n-butyllithium; "THF" refers to tetrahydrofuran; "m-CPBA" refers to m-chloroperoxybenzoic acid; "CuCN" refers to copper cyanide; "DMF" refers to dimethylformamide; "K₂CO₃" refers to potassium carbonate; "dioxane" refers to dioxane; "NBS" refers to N-Bromosuccinimide; "MeCN" refers to acetonitrile, "DMSO" refers to dimethyl sulfoxide.

The term "optional" or "optionally" means that the event or circumstance subsequently described may, but not necessarily, occur, and that the description includes instances where the event or circumstance occurs or dose not occur, i.e., both substituted or unsubstituted instances. For example, "heterocyclyl group optionally substituted by alkyl" means that alkyl may be, but not necessarily, present, and that the description includes instances where the heterocyclyl group is and is not substituted by alkyl.

The term "substituted" means that one or more "hydrogen atoms" in a group are each independently substituted by a corresponding number of substituents. It goes without saying that a substituent is only in its possible chemical position and consistent with the valence bond theory in chemistry, and those skilled in the art will be able to determine (by experiments or theories) possible or impossible substitution without undue efforts. For example, it may be unstable when an amino or hydroxy having a free hydrogen is bound to a carbon atom having an unsaturated bond (such as olefin).

"Stereoisomer" refers to isomers produced by different spatial arrangements of atoms in the molecules. They can be divided into cis-trans isomers and enantiomers and can also be divided into two categories: enantiomers and diastereomers. A stereoisomer produced by the rotation of a single bond is referred to as a conformational stereo-isomer, sometimes also referred to as a rotamer. A stereoisomer produced by bond length, bond angle, double bonds in the molecule, ring and other reasons is referred to as a configuration stereo-isomer, which can be divided into two categories. An isomer produced by the inability of a double bond or a single bond of ring carbon atoms to rotate freely becomes a geometric isomer, also known as a cis-trans isomer, which can be divided into Z and E configurations. For example: cis-2-butene and trans-2-butene are a pair of geometric isomers. Stereoisomers with different optical properties produced by the absence of anti-axial symmetry in the molecules are called optical isomers, which can be divided into R and S configurations. "Stereoisomers" as described in the present invention, if not specifically indicated, are to be understood to include one or more of the enantiomers, configurational isomers, and conformational isomers described above.

"Pharmacologically acceptable salt" in the present invention refers to pharmaceutically acceptable acid addition salts, including inorganic and organic acid salts, which may be prepared by methods known in the art.

"Pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities.

**The present invention is further explained in detail below with reference to examples, which are not intended to limit the present invention, and the present invention is not merely limited to the contents of the examples.**

The compound structure of the present invention is determined by nuclear magnetic resonance (NMR) or/and liquid chromatography-mass spectrometry (LC-MS). The NMR chemical shift (δ) is given in parts per million (ppm). The NMR determination is conducted by using a Bruker AVANCE-400/500 nuclear magnetic resonance apparatus, with hexadeuterodimethyl sulfoxide (DMSO-*d*₆), tetradeuteromethanol (CD₃OD) and deuterated chloroform (CDCl₃) as determination solvents, and tetramethylsilane (TMS) as internal standard.

The LC-MS determination is conducted by using an Agilent 6120 mass spectrometer. The HPLC determination is conducted by using an Agilent 1200 DAD high pressure liquid chromatography (Sunfire C18 150 × 4.6 mm chromatographic column) and a Waters 2695-2996 high pressure liquid chromatography (Gimini C18 150 × 4.6 mm chromatographic column).

Yantai Yellow Sea HSGF254 or Qingdao GF254 silica gel plate is adopted as a thin layer chromatography (TCL) silica gel plate. The specification adopted by the TLC is 0.15-0.20 mm, and the specification adopted by the thin layer chromatography for the separation and purification of products is 0.4-0.5 mm. The Yantai Yellow Sea silica gel of 200-300 mesh is generally utilized as a carrier in column chromatography.

Starting materials in the examples of the present invention are known and commercially available, or may be synthesized by using or according to methods known in the art.

Unless otherwise stated, all reactions of the present invention are carried out under a dry nitrogen or argon atmosphere with continuous magnetic stirring, wherein the solvent is a dry solvent and the reaction temperature is in degree centigrade (°C).

### I. Preparation of Intermediates

### Intermediate 1: Preparation of 2-(4-bromo-1-methyl-1H-pyrazol-5-yl)-3-fluoro-1-naphthonitrile

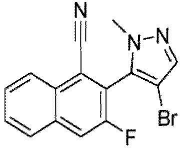

### Step 1: Synthesis of 5-bromo-7-fluoro-9-methyl-1,4-dihydro-1,4-cycloiminonaphthalene

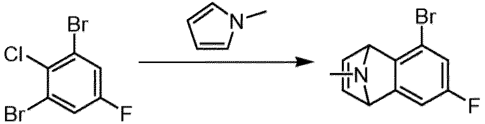

1,3-dibromo-2-chloro-5-fluorobenzene (45.0 g, 156 mmol) and N-methyl-2-pyrrole (27.8 mL, 312 mmol) were dissolved in tetrahydrofuran (1,000 mL), and n-butyllithium (65 mL, 162 mmol) was added dropwise at -30°C under nitrogen protection. After dropwise addition, it was stirred at -30°C for 0.5 hours and then was stirred at 25°C for 12 hours. The reaction solution was slowly poured into 1,000 mL water, extracted with ethyl acetate (1,000 mL * 3), and washed with saturated aqueous sodium chloride solution (1,000 mL). The organic phase was dried over anhydrous sodium sulfate and concentrated under reduced pressure to remove the solvent. The remaining was separated by using a fast silica gel column to obtain 5-bromo-7-fluoro-9-methyl-1,4-dihydrogen-1,4-cycloiminonaphthalene (19.0 g, yield: 48%).

¹H NMR (400MHz, CDCl₃) δ 7.16-6.62 (m, 4H), 4.85-4.44 (m, 2H), 2.32 (br s, 1H), 2.16 (br s, 1H), 2.06 (s, 1H), 1.81 (br s, 1H).

### Step 2: Synthesis of 1-bromo-3-fluoronaphthalene

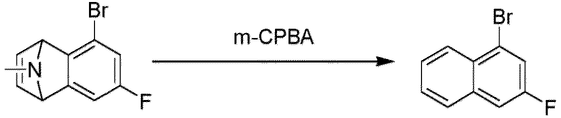

5-bromo-7-fluoro-9-methyl-1,4-dihydro-1,4-cycloiminonaphthalene (44.0 g, 173 mmol) was dissolved in chloroform (450 mL), m-CPBA (70.2 g, 85% purity, 346 mmol) was slowly added in batches at 0°C, and the reaction solution was reacted at 25°C for 14 hours. The reaction solution was slowly poured into 500 mL of water, extracted with dichloromethane (500 mL * 3), washed with saturated aqueous sodium sulfite solution (400 mL * 2), and then washed with saturated brine (400 mL). The organic phase was dried over anhydrous sodium sulfate and concentrated under reduced pressure to remove the solvent. The remaining was separated by using a fast silica gel column to obtain 1-bromo-3-fluoronaphthalene (34.0 g, yield: 87%).

¹H NMR (400MHz, CDCl₃) δ 8.29-8.19 (m, 1H), 7.78 (br dd, J=2.9, 4.5 Hz, 1H), 7.67-7.52 (m, 3H), 7.50-7.41 (m, 1H).

### Step 3: Synthesis of 3-fluoro-1-naphthonitrile

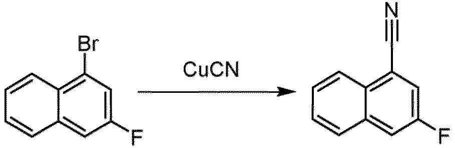

1-bromo-3-fluoronaphthalene (34.0 g, 151 mmol) was dissolved in DMF (450 mL), and copper cyanide (23.2 mL, 755 mmol) was slowly added under nitrogen protection at 0°C. The mixture was reacted under nitrogen protection at 145°C for 12 hours. The reaction solution was slowly poured into 250 mL saturated brine, extracted with ethyl acetate, washed with saturated sodium chloride aqueous solution. The organic phase was dried over anhydrous sodium sulfate and concentrated under reduced pressure to remove the solvent. The remaining was separated by using a fast silica gel column to obtain 3-fluoro-1-naphthonitrile (19.0 g, yield: 66%).

¹H NMR (400 MHz, CDCl₃) δ 8.17 (m, 1H) 7.81 (m, 1H) 7.62 (m, 4H).

### Step 4: Synthesis of 3-fluoro-2-iodo-1-naphthonitrile

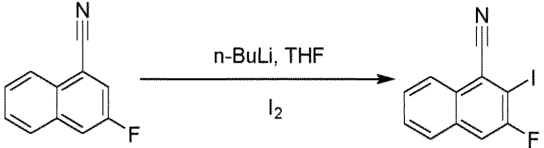

3-fluoro-1-naphthonitrile (1.89 g, 3.57 mmol) was dissolved in tetrahydrofuran (200 mL), and LDA (48.2 mL, 96.4 mmol) was added slowly dropwise under nitrogen protection. After the dropwise addition was completed, it was reacted under nitrogen protection at -70°C for 30 minutes. After iodine (28.3 mL, 114 mmol) was dissolved in tetrahydrofuran (200 mL), it was added to the reaction solution under nitrogen protection at -70°C. The mixture was stirred at -70°C for 30 minutes and reacted at 25°C for 17 hours. After the reaction was completed, it was diluted with water (300 mL), extracted with ethyl acetate (3 * 300 mL), and washed with saturated aqueous sodium chloride solution (400 mL). The organic phase was dried over anhydrous sodium sulfate and concentrated under reduced pressure to remove the solvent. The remaining was separated by using a fast silica gel column to obtain 3-fluoro-2-iodo-1-naphthonitrile (16.0 g, yield: 52 %).

¹H NMR (400MHz, CDCl₃) δ 8.29-8.22 (m, 1H), 7.91-7.84 (m, 1H), 7.76-7.64 (m, 3H).

### Step 5: Synthesis of 3-fluoro-2-(1-methyl-1H-pyrazol-5-yl)-1-naphthonitrile

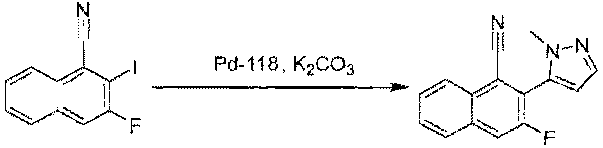

3-fluoro-2-iodo-1-naphthonitrile (8.00 g, 26.9 mmol) was dissolved in 1,4-dioxane (80 mL) and water (16 mL), and sodium bicarbonate (42.4 g, 505 mmol), potassium carbonate (608 mg, 4.40 mmol), 1-methyl-1*H* pyrazol-5-boronatate (14.0 g, 67.3 mmol) and Pd-118 (0.88 g, 1.34 mmol) were added to the reaction solution under nitrogen protection. The reaction solution was reacted under nitrogen protection at 80°C for 2 hours. The reaction solution was extracted with ethyl acetate (80 mL * 3) and washed with saturated aqueous sodium chloride solution (200 mL). The organic phase was dried over anhydrous sodium sulfate and concentrated under reduced pressure to remove the solvent. The remaining was separated by using a fast silica gel column to obtain 3-fluoro-2-(1-methyl-1*H*-pyrazol-5-yl)-1-naphthylnitrile (6.0 g, yield: 76%). MS m/z (ESI): 252 [M+H]⁺.

¹H NMR (400MHz, CDCl₃) δ 8.36-8.30 (m, 1H), 7.98-7.92 (m, 1H), 7.88 (d, *J* = 9.8 Hz, 1H), 7.77-7.73 (m, 2H), 7.69 (d, *J* = 2.0 Hz, 1H), 7.28 (s, 1H), 6.63 (d, *J* = 2.0 Hz, 1H), 3.86 (d, *J* = 1.5 Hz, 3H).

### Step 6: Synthesis of 2-(4-bromo-1-methyl-1H-pyrazol-5-yl)-3-fluoro-1-naphthonitrile

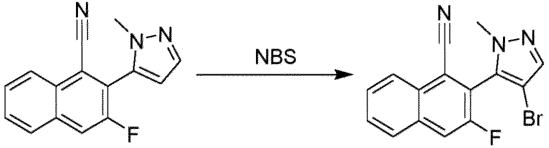

3-fluoro-2-(1-methyl-1H-pyrazol-5-yl)-1-naphthonitrile (6.00 g, 23.8 mmol) was dissolved in acetonitrile (500 mL), NBS (7.65 g, 42.9 mmol) was added, and the reaction solution was reacted at 25°C for 24 hours. After the reaction was completed, the reaction solution was extracted with water (100 mL) and ethyl acetate (100 mL * 3), and washed with saturated aqueous sodium chloride solution (200 mL). The organic phase was dried over anhydrous sodium sulfate and concentrated under reduced pressure to remove the solvent. The remaining was separated by using a fast silica gel column to obtain 2-(4-bromo-1-methyl-1*H*-pyrazol-5-yl)-3-fluoro-1-naphthitrile (8.00 g, yield: 91%).

¹H NMR (400MHz, DMSO-*d*₆) δ 8.49 (d, *J* = 10.4 Hz, 1H), 8.27-8.19 (m, 2H), 7.93-7.85 (m, 3H), 3.81 (s, 3H).

### Intermediate 2: Preparation of tert-butyl ((3-bromo-8-oxo-7,8-dihydropyrido[2,3-d] pyridazin-5-yl)methyl)carbamate

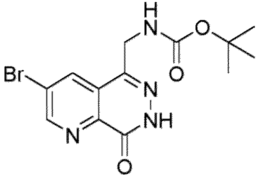

### Step 1: Synthesis of methyl 5-bromo-3-(bromomethyl)picolinate

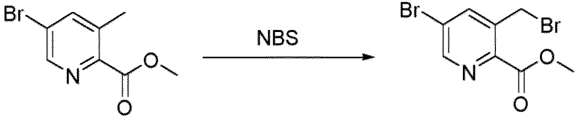

Methyl 5-bromo-3-methylpicolinate (3.0 g, 13.04 mmol) and NBS (3.48 g, 19.56 mmol) were put into carbon tetrachloride (50 mL), and the mixture was stirred under nitrogen protection at 80°C for 18 hours. After concentration of the reaction solution, it was separated by using column chromatography to obtain methyl 5-bromo-3-(bromomethyl)picolinate (4.0, yield: 90%).

¹H NMR (400MHz, DMSO-*d*₆) δ 8.69 (d, *J* = 2.0 Hz, 1H), 8.06 (d, *J* = 2.0 Hz, 1H), 4.89 (s, 2H), 4.02 (s, 3H).

### Step 2: Synthesis of 3-bromofuro[3,4-b]pyridin-7(5H)-one

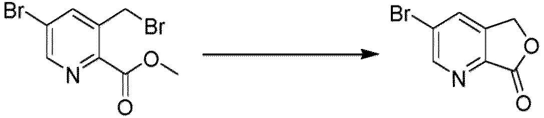

Methyl 5-bromo-3-(bromomethyl)picolinate (2,000 mg, 6.47 mmol) and water (10 mL) were put into 1,4-dioxane (50 mL), and the mixture was stirred under nitrogen protection at 100°C for 18 hours. After concentration of the reaction solution, it was separated by using column chromatography to obtain 3-bromofuro[3,4-b]pyridin-7(5H)-one (1.0 g, yield: 61%). MS m/z (ESI): 213/215 [M+H]⁺.

### Step 3: Synthesis of (Z)-3-bromo-5-((dimethylamino)methylene)furo[3,4-b]pyridin-7(5H)-one

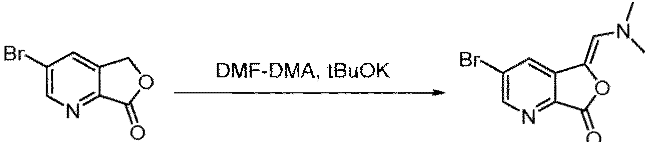

3-bromofuro[3,4-b]pyridin-7(5H)-one (1.0 g, 4.67 mmol) and potassium tert-butoxide (52 mg, 0.47 mmol) were put into DMFDMA, and the mixture was stirred under nitrogen protection at 100°C for 18 hours. After concentration of the reaction solution, it was slurried with PE to obtain(Z)-3-bromo-5-((dimethylamino)methylene)furo[3,4-b]pyridin-7(5H)-one (1.0 g, yield: 70%). MS m/z (ESI): 269/271 [M+H]⁺.

### Step 4: Synthesis of 3-bromo-5-((dimethylamino)methyl)pyrido[2,3-d]pyridazin-8(7H)-one

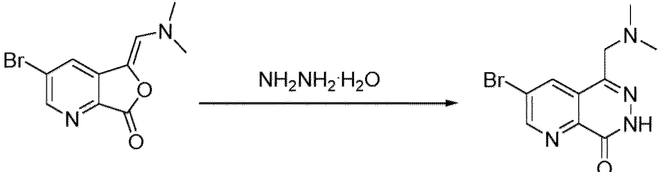

(Z)-3-bromo-5-((dimethylamino)methylene)furo[3,4-b]pyridin-7(5H)-one (7.0 g, 26.0 mmol) and hydrazine hydrate (7.44 mL, 130.1 mmol) were put into ethanol (50 mL), and the mixture was stirred under nitrogen protection at 80°C for 18 hours. After concentration of the reaction solution, it was separated by using column chromatography to obtain 3-bromo-5-((dimethylamino)methyl)pyrido[2,3-d]pyridazin-8(7H)-one (3.0 g, yield: 25%). MS m/z (ESI): 283/285 [M+H]⁺.

### Step 5: Synthesis of 3-bromo-5-(chloromethyl)pyrido[2,3-d]pyridazin-8(7H)-one

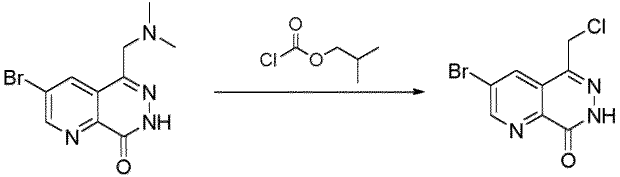

3-bromo-5-((dimethylamino)methyl)pyrido[2,3-d]pyridazin-8(7H)-one (300 mg, 1.06 mmol) and 2-methylpropyl chloroformate (0.28 mL, 2.12 mmol) were put into THF (10 mL), and the mixture was stirred under nitrogen protection at room temperature for 4 hours. After concentration of the reaction solution, it was slurried with PE to obtain 3-bromo-5-(chloromethyl)pyrido[2,3-d]pyridazin-8(7H)-one (250 mg, yield: 38%). MS m/z (ESI): 275/277 [M+H]⁺.

### Step 6: Synthesis of 2-((3-bromo-8-oxo-7,8-dihydropyrido[2,3-d]pyridazin-5-yl)methyl)isoindoline-1,3-dione

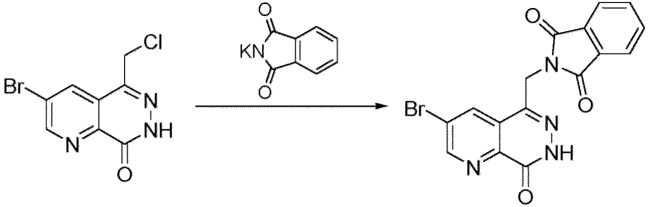

3-bromo-5-(chloromethyl)pyrido[2,3-d]pyridazin-8(7H)-one (1.5 g, 5.46 mmol) and phthalimide potassium salt (1.52 g, 8.20 mmol) were put into DMF (50 mL), and the mixture was stirred under nitrogen protection at 90°C for 2 hours. The reaction solution was diluted with water and extracted with EtOAc. The organic phase was washed with saturated brine and dried over anhydrous sodium sulfate. After concentration, it was separated by using column chromatography to obtain 2-((3-bromo-8-oxo-7,8-dihydropyrido[2,3-d]pyridazin-5-yl)methyl)isoindoline-1,3-dione (2.0 g, yield: 95%). MS m/z (ESI): 385/387 [M+H]⁺.

### Step 7: Synthesis of 5-(aminomethyl)-3-bromopyrido[2,3-d]pyridazin-8(7H)-one

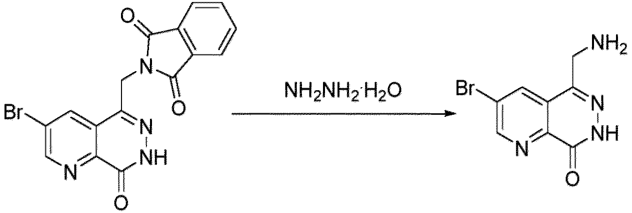

2-((3-bromo-8-oxo-7,8-dihydropyrido[2,3-d]pyridazin-5-yl)methyl)isoindoline-1,3-dione (1.0 g, 4.67 mmol) and hydrazine hydrate (498 mg, 15.58 mmol) were put into ethanol (200 mL), and the mixture was stirred under nitrogen protection at 70°C for 18 hours. The reaction solution was concentrated to obtain 5-(aminomethyl)-3-bromopyrido[2,3-d]pyridazin-8(7H)-one (900 mg, yield: 67%). MS m/z (ESI): 256/258 [M+H]⁺.

### Step 8: Synthesis of tert-butyl ((3-bromo-8-oxo-7,8-dihydropyrido[2,3-d]pyridazin-5-yl)methyl)carbamate

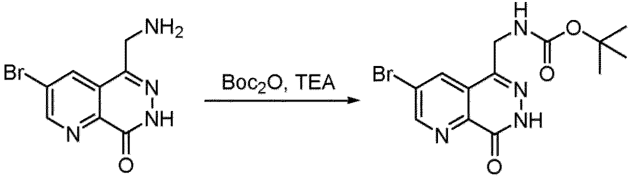

5-(aminomethyl)-3-bromopyrido[2,3-d]pyridazin-8(7H)-one (900 mg, 3.53 mmol) and triethylamine (2.45 mL, 17.64 mmol) were put into dichloromethane (100 mL). Under nitrogen protection, di-tert-butyl dicarbonate (3.02 mL, 14.11 mmol) was added, and the mixture was stirred at room temperature for 18 hours. The reaction solution was diluted with water and extracted with EtOAc. The organic phase was washed with saturated brine and dried over anhydrous sodium sulfate. After concentration, it was separated by using column chromatography to obtain tert-butyl ((3-bromo-8-oxo-7,8-dihydropyrido[2,3-d]pyridazin-5-yl)methyl)carbamte (300 mg, yield: 23%). MS m/z (ESI): 355/357 [M+H]⁺.

### Intermediate 3: Preparation of (4-((bis(tert-butoxycarbonyl)amino)methyl)-8-cyano-1-oxo-1,2-dihydrophthalazin-6-yl)boronic acid

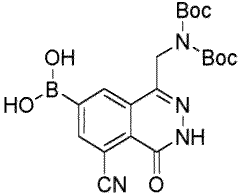

### Step 1: Synthesis of 4-bromo-2-iodo-6-methylbenzoic acid

A mixture of 4-bromo-2-methylbenzoic acid (100 g, 465.1 mmol), NIS (156.93 g, 697.5 mmol) and Pd(OAc)₂ (10.44 g, 46.5 mmol) in DMF (600 mL) was degassed with nitrogen and then stirred at 100°C for 3 hours. The reaction solution was extracted with ethyl acetate (1 L) and water (1 L), and washed with saturated brine (1 L). The organic phase was dried over anhydrous sodium sulfate and concentrated under reduced pressure to remove the solvent. The remaining was separated by using column chromatography to obtain 4-bromo-2-iodo-6-methylbenzoic acid (120 g, yield: 57%).

### Step 2: Synthesis of 5-bromo-7-iodo-1,3-dihydro-2-benzofuran-1-one

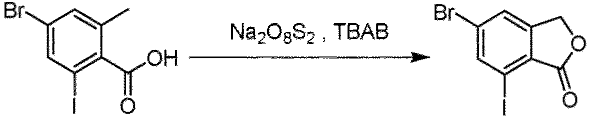

4-bromo-2-iodo-6-methylbenzoic acid (60 g, 176.0 mmol), TBAB (109.2 mL, 352.0 mmol) and Na₂O₈S₂ (99.5 mL, 1003.1 mmol) were respectively added to CH₃CN (600 mL) solution. The mixture was stirred at 80°C for 24 hours. TLC showed that the reaction was completed. The reaction solution was extracted with ethyl acetate (600 mL) and water (600 mL) and washed with saturated brine (600 mL). The organic phase was dried over anhydrous sodium sulfate and concentrated under reduced pressure to remove the solvent. The remaining was separated by using column chromatography to obtain 5-bromo-7-iodo-1,3-dihydro-2-benzofuran-1-one (20 g, yield: 33%).

¹H NMR (400 MHz, DMSO-d₆) δ 8.31 (d, *J=* 0.72 Hz, 1H), 8.04 (d, *J=* 1.07 Hz, 1H), 5.35 (s, 2H).

### Step 3: Synthesis of (3Z)-5-bromo-3-[(dimethylamino)methylethylene]-7-iodo-1,3-dihydro-2-benzofuran-1-one

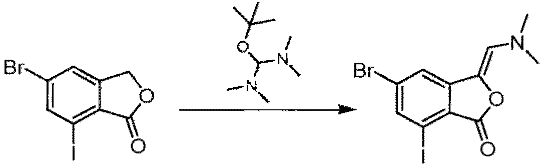

Toluene (330 mL) was added to a flask containing 5-bromo-7-iodo-1,3-dihydro-2-benzofuran-1-one (33.5 g, 98.8 mmol), and then [(tert-butoxy)(dimethylamino)methyl]dimethylamine (26.5 mL, 128.5 mmol) was added. The mixture was stirred at 90°C for 5 hours. TLC showed that the reaction was completed. The mixture was filtered and the filter cake was dried to obtain compound (3Z)-5-bromo-3-[(dimethylamino)methylethylene]-7-iodo-1,3-dihydro-2-benzofuran-1-one as a yellow solid (20.1 g, yield: 52%).

¹H NMR (400 MHz, DMSO-d₆) δ 7.96 (d, *J* = 1.19 Hz, 1H), 7.68 (d, *J* = 1.07 Hz, 1H), 7.07 (s, 1H), 3.12 (s, 6H).

### Step 4: Synthesis of 6-bromo-4-[(dimethylamino)methyl]-8-iodo-1,2-dihydrophthalazin-1-one

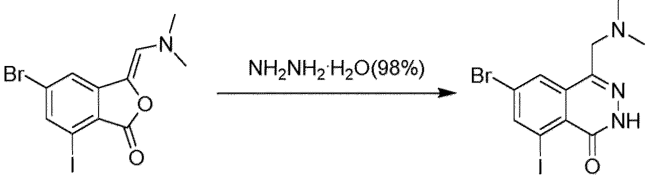

In a flask of 500 mL, a solution of (3Z)-5-bromo-3-[(dimethylamino)methylethylene]-7-iodo-1,3-dihydro-2-benzofuran-1-one (18 g, 45.7 mmol) in ethanol (180 mL) was added, hydrazine hydrate (5.2 mL, 105.1 mmol) was added at 0°C, and it was stirred at 25°C for 0.5 hours and then stirred at 50 °C for 18 hours. The precipitate was directly filtered and dried under reduced pressure to obtain 6-bromo-4-[(dimethylamino)methyl]-8-iodo-1,2-dihydrophthalazin-1-one as a white solid (11.73 g, yield: 63%).

¹H NMR (400 MHz, DMSO-d₆) δ 8.53 (d, *J* = 1.67 Hz, 1H), 8.35 (d, *J* = 1.67 Hz, 1H), 3.56 (s, 2H), 2.18 (s, 6H).

### Step 5: Synthesis of 6-bromo-4-(chloromethyl)-8-iodo-1,2-dihydrophthalazin-1-one

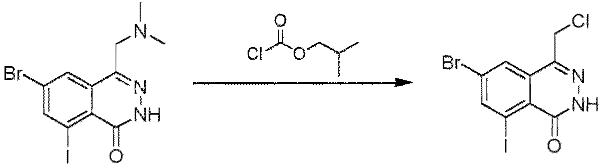

A mixture of 6-bromo-4-[(dimethylamino)methyl]-8-iodo-1,2-dihydrophthalazin-1-one (12 g, 29.48 mmol) in THF (140 mL) was degassed multiple times with N₂, cooled to 0°C, and chloro(2-methylpropoxy)methane (13.8 mL, 106.1 mmol) was added dropwise, and then it was stirred under nitrogen at 25°C for 16 hours. The reaction solution was extracted with ethyl acetate (100 mL) and water (100 mL) and washed with saturated brine (100 mL). The organic phase was dried over anhydrous sodium sulfate and concentrated under reduced pressure to obtain a white crude product. The crude product was stirred together with MTBE (20 mL) at 25°C for 30 minutes. The mixture was filtered, and the filter cake was dried to obtain compound 6-bromo-4-(chloromethyl)-8-iodo-1,2-dihydrophthalazin-1-one as a white solid (9.2 g, yield: 78%).

¹H NMR (400 MHz, DMSO-d₆) δ ppm 12.88 (s, 1H), 8.59 (d, *J=* 1.76 Hz, 1H), 8.27 (d, *J=* 1.76 Hz, 1H), 5.02 (s, 2H).

### Step 6: Synthesis of tert-butyl N-[(7-bromo-5-iodo-4-oxy-3,4-dihydrophthalazin-1-yl)methyl]-N-[(tert-butyl)carbonyl]carbamate

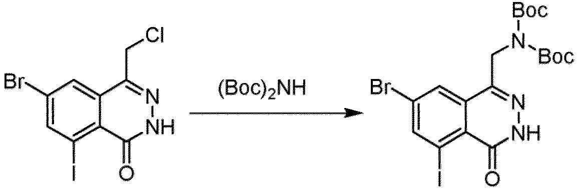

Bis(tert-butoxycarbonyl)amine (5.16 g, 23.73 mmol) was added to THF (100 mL), lithium bis(trimethylsilyl)amide (25.7 mL, 25.71 mmol) was added under N₂ atmosphere at 0°C, and it was stirred under N₂ atmosphere at -70°C for 1 hour. Then, a mixture of 6-bromo-4-(chloromethyl)-8-iodo-1,2-dihydrophthalazin-1-one (7.9 g, 19.80 mmol) in tetrahydrofuran (100 mL) was added to the mixture. The resulting mixture was stirred at -70°C for 2 hours. The reaction solution was quenched with saturated NH₄Cl (100 ml), extracted with ethyl acetate (80 ml) and water (100 ml), and washed with saturated brine (100 ml). The organic phase was dried over anhydrous sodium sulfate and concentrated under reduced pressure to remove the solvent. The remaining was separated by using column chromatography to obtain tert-butyl N-[(7-bromo-5-iodo-4-oxo-3,4-dihydrophthalazin-1-yl)methyl]-N-[(tert-butyl)carbonyl]carbamate (4.09 g, yield: 33%).

¹H NMR (400 MHz, DMSO-d₆) δ ppm 12.69 (s, 1H), 8.60 (d, *J* = 1.76 Hz, 1H), 8.26 (d, *J=* 1.76 Hz, 1H), 4.98 (s, 2H), 1.39 (s, 18H).

### Step 7: Synthesis of tert-butyl ((7-bromo-5-cyano-4-oxo-3,4-dihydrophthalazin-1-yl)methyl)(tert-butoxycarbonyl)carbamate

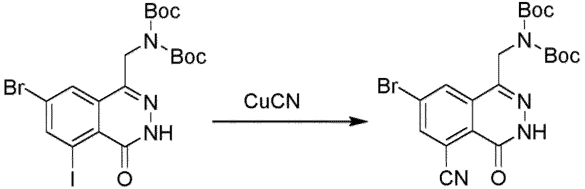

Tert-butyl N-[(7-bromo-5-iodo-4-oxy-3,4-dihydrophthalazin-1-yl)methyl]-N-[(tert-butyl)carbonyl]carbamate (1.4 g, 2.17 mmol), N,N-dimethylformamide (20 mL) and copper cyanide (194 mg, 2.17 mmol) were added to a single-neck flask of 250 mL, and stirred at 80°C for 3 hours. The reaction solution was concentrated under reduced pressure. The residue was diluted with dichloromethane, and washed with 5% aqueous ammonia solution and saturated brine respectively. The organic phase was dried over anhydrous sodium sulfate, evacuated and filtered, and the filtrate was concentrated under reduced pressure to obtain tert-butyl ((7-bromo-5-cyano-4-oxo-3,4-dihydrophthalazin-1-yl)methyl)(tert-butoxycarbonyl)carbamate (1.1 g, 88% purity, yield: 93%). MS m/z (ESI): 479/481 [M+H]⁺.

### Step 8: Synthesis of (4-((bis(tert-butoxycarbonyl)amino)methyl)-8-cyano-1-oxo-1,2-dihydrophthalazin-6-yl)boronic acid

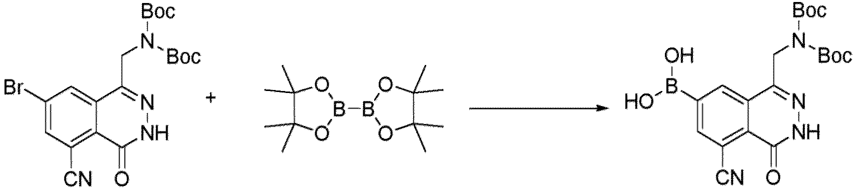

Tert-butyl ((7-bromo-5-cyano-4-oxo-3,4-dihydrophthalazin-1-yl)methyl)(tert-butoxycarbonyl)carbamate (1.5 g, 93% purity, 2.91 mmol), 1,4-dioxane (20 mL), potassium acetate (857 mg, 8.73 mmol), 1,1-bis(diphenylphosphino)ferrocene dichloropalladium (213 mg, 0.29 mmol) and pinacol diborate (2.22 g, 8.73 mmol) were added to a single-neck flask of 100 mL and stirred at 80°C for 2 hours. The reaction solution was concentrated under reduced pressure. The residue was separated by reversed-phase column chromatography to obtain (4-((bis(tert-butoxycarbonyl)amino)methyl)-8-cyano-1-oxo-1,2-dihydrophthalazin-6-yl)boronic acid (1,090 mg, 65% purity, yield: 55%). MS m/z (ESI): 445 [M+H]⁺.

### Intermediates 4-7 can be prepared by selecting the corresponding raw materials with reference to all or part of the synthesis methods of intermediates 1, 2 or 3:

| **Intermediate No.** | **Structural Formula** | **Chemical Name** | **[M+H]⁺** |
|---|---|---|---|
| **4** | 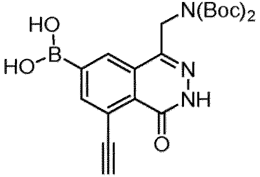 | (4-((bis(tert-butoxycarbonyl)amino)methyl)-8-ethynyl-1 -oxo-1,2-dihydrophthalazin-6-yl)boronic acid | 444 |
| **5** | 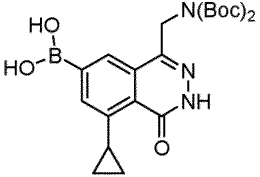 | (4-((bis(tert-butoxycarbonyl)amino)methyl)-8-cyclopropyl-1 -oxo-1,2-dihydrophthalazin-6-yl)boronic acid | 460 |
| **6** | 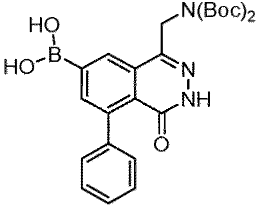 | (4-((bis(tert-butoxycarbonyl)amino)methyl)-1-oxo-8-phenyl-1,2-dihydrophthalazin-6-yl)boronic acid | 496 |
| **7** | 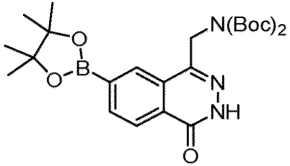 | tert-butyl (tert-butoxycarbonyl)((4-oxo-7-(4,4,5,5-tetramethyl-1,3,2-dioxaborolan-2-yl)-3,4-dihydrophthalazin-1-yl)methyl)carbamate | 502 |

### Preparation of Intermediate 8: 2-(4-bromo-1-methyl-1H-pyrazol-5-yl)-4-chloro-6-(3,3-difluoroazetidin-1-yl)-3-fluorobenzonitrile

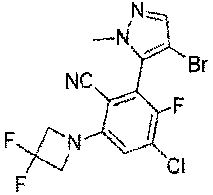

### Step 1: Synthesis of 2-bromo-4-chloro-6-(3,3-difluoroazetidin-1-yl)-3-fluorobenzonitrile

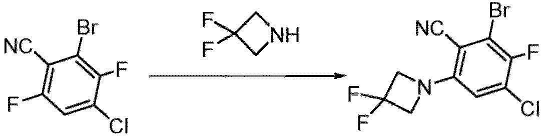

2-bromo-4-chloro-3,6-difluorobenzonitrile (100 mg, 0.40 mmol), potassium carbonate (219 mg, 1.58 mmol) and 3,3-difluorobutyridine hydrochloride (103 mg, 0.792 mmol) were dissolved in N-methylpyrrolidone (2 mL) and stirred overnight at 75°C. After the reaction was completed, it was cooled to room temperature, and water (20 ml) was added. The reaction solution was extracted with ethyl acetate (30 mL * 3). The combined organic phase was washed with saturated brine, dried over anhydrous sodium sulfate and concentrated under reduced pressure to remove the solvent. The crude product was separated by using column chromatography to obtain 2-bromo-4-chloro-6-(3,3-difluoroazetidin-1-yl)-3-fluorobenzonitrile (50 mg, yield: 39%). ¹H NMR (400 MHz, CDCl₃) δ 6.49 (d, *J=* 5.6 Hz, 1H), 4.53 (t, *J=* 11.7 Hz, 4H).

### Step 2: Synthesis of 4-chloro-6-(3,3-difluoroazetidin-1-yl)-3-fluoro-2-(1-methyl-1H-pyrazol-5-yl)benzonitrile

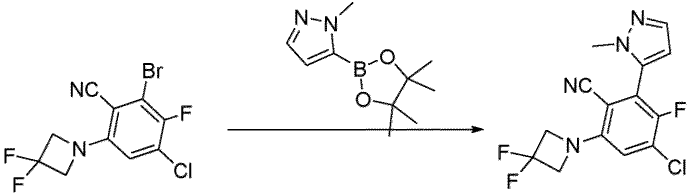

2-bromo-4-chloro-6-(3,3-difluoroazetidin-1-yl)-3-fluorobenzonitrile (300 mg, 0.92 mmol), 1-methyl-5-(tetramethyl-1,3,2-dioxaborolan-2-yl)-1*H*-pyrazol (249 mg, 1.20 mmol) and sodium bicarbonate (310 mg, 3.69 mmol) were dissolved in 1,4-dioxane (16 mL) and water (4 mL). cataCXium A Pd G3 (67 mg, 0.09 mmol) was added under nitrogen protection. The reaction solution was stirred under nitrogen protection at 60°C for 40 hours. Saturated sodium bicarbonate solution (30 mL) was added to the reaction solution, which then was extracted with dichloromethane (30 mL * 3). After the organic phase was combined and then concentrated, the crude product was separated by using column chromatography to obtain 4-chloro-6-(3,3-difluoroazetidin-1-yl)-3-fluoro-2-(1-methyl-1*H*-pyrazol-5-yl)benzonitrile (120 mg, yield: 40%). MS m/z (ESI): 327 [M+H]⁺.

### Step 3: Synthesis of 2-(4-bromo-1-methyl-1H-pyrazol-5-yl)-4-chloro-6-(3,3-difluoroazetidin-1-yl)-3-fluorobenzonitrile

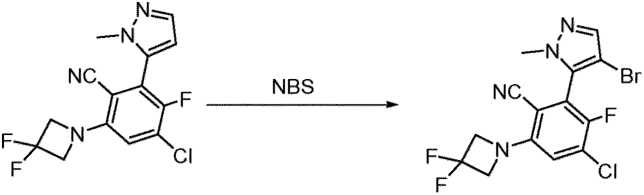

4-chloro-6-(3,3-difluoroazetidin-1-yl)-3-fluoro-2-(1-methyl-1*H*-pyrazol-5-yl)benzonitrile (120 mg, 0.37 mmol) was dissolved in anhydrous acetonitrile (5 mL), and NBS (98.0 mg, 0.55 mmol) was added. The reaction solution was stirred overnight at 50°C. After the reaction was completed, the reaction system was concentrated, and the crude product was separated by using a C18 column reversed-phase column to obtain 2-(4-bromo-1-methyl-1*H*-pyrazol-5-yl)-4-chloro-6-(3,3-difluoroazetidin-1-yl)-3-fluorobenzonitrile (120 mg, yield: 80.6%). MS m/z (ESI): 405/407 [M+1]⁺.

### Intermediates 9-21 can be prepared by selecting the corresponding raw materials with reference to all or part of the synthesis methods of intermediate 8:

| **Intermediate No.** | **Structural Formula** | **Name** | **[M+H]⁺** |
|---|---|---|---|
| **9** | 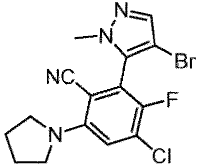 | 2-(4-bromo-1 -methyl- 1H-pyrazol-5-yl)-4-chloro-3-fluoro-6-(pyrrolidin-1 - yl)benzonitrile | 383/385 |
| **10** | 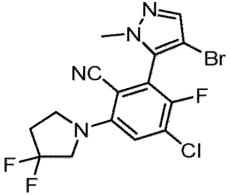 | 2-(4-bromo-1-methyl- 1H-pyrazol-5-yl)-4-chloro-6-(3,3-difluoropyrrolidin-1-yl)-3-fluorobenzonitrile | 419/421 |
| **11** | 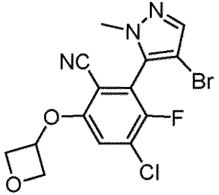 | 2-(4-bromo-1 -methyl- 1H-pyrazol-5-yl)-4-chloro-3-fluoro-6-(oxetane-3 - oxy)benzonitrile | 386/388 |
| **12** | 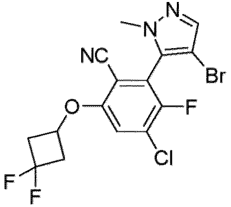 | 2-(4-bromo-1-methyl- 1H-pyrazol-5-yl)-4-chloro-6-(3,3-difluorocyclobutoxy)-3 - fluorobenzonitrile | 420/422 |
| **11** | 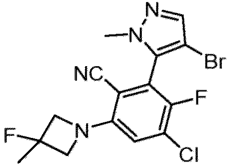 | 2-(4-bromo-1-methyl- 1H-pyrazol-5-yl)-4-chloro-3-fluoro-6-(3 -fluoro-3 - methylazetidin-1-yl)benzonitrile | 401/403 |
| **13** | 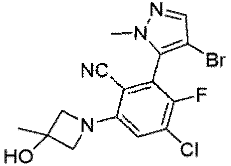 | 2-(4-bromo-1-methyl- 1H-pyrazol-5-yl)-4-chloro-3-fluoro-6-(3 -hy droxy-3 - methylazetidin-1-yl)benzonitrile | 399/401 |
| **14** | 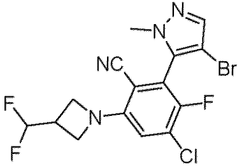 | 2-(4-bromo-1-methyl- 1H-pyrazol-5-yl)-4-chloro-6-(3-(difluoromethyl)azetidin-1-yl)-3-fluorobenzonitrile | 419/421 |
| **15** | 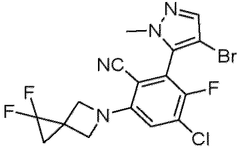 | 2-(4-bromo-1-methyl- 1H-pyrazol-5-yl)-4-chloro-6-(1,1-difluoro-5-azaspiro[2.3]hexan-5-yl)-3-fluorobenzonitrile | 431/433 |
| **16** | 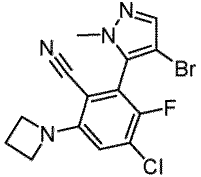 | 6-(azetidin-1-yl)-2-(4-bromo-1-methyl-1*H*-pyrazol-5-yl)-4-chloro-3-fluorobenzonitrile | 369/371 |
| **17** | 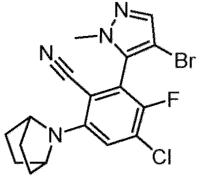 | 6-(7-azabicyclo[2.2.1]heptan-7-yl)-2-(4-bromo-1-methyl-1*H*-pyrazol-5-yl)-4-chloro-3-fluorobenzonitrile | 409/411 |
| **18** | 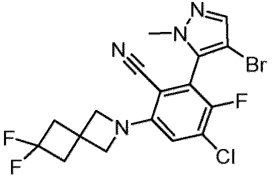 | 2-(4-bromo-1-methyl- 1*H-*pyrazol-5-yl)-4-chloro-6-(6,6-difluoro-2-azaspiro[3.3]heptan-2-yl )-3-fluorobenzonitrile | 445/447 |
| 19 | 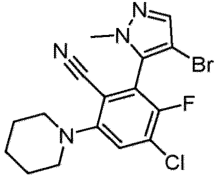 | 2-(4-bromo-1 -methyl-1*H-*pyrazol-5-yl)-4-chloro-3-fluoro-6-(piperidin-1 - yl)benzonitrile | 397/399 |
| 20 | 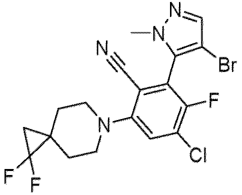 | 2-(4-bromo-1-methyl-1*H-*pyrazol-5-yl)-4-chloro-6-(1,1-difluoro-6-azaspiro[2.5]octane-6-yl )-3-fluorobenzonitrile | 459/461 |
| 21 | 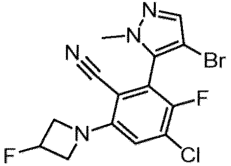 | 2-(4-bromo-1-methyl-1*H-*pyrazol-5-yl)-4-chloro-3-fluoro-6-(3 -fluoroazetidin-1 - yl)benzonitrile | 387/389 |
| 22 | 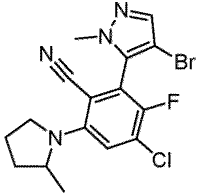 | 2-(4-bromo-1-methyl- 1H-pyrazol-5-yl)-4-chloro-3-fluoro-6-(2-methylpyrrolidin-1-yl)benzonitrile | 397/399 |
| 23 | 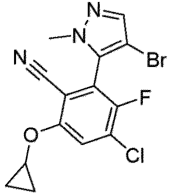 | 2-(4-bromo-1-methyl-1*H-*pyrazol-5-yl)-4-chloro-6-cyclopropoxy-3-fluorobenzonitrile | 370/372 |

### Preparation of intermediate 24: 2-(4-bromo-1-methyl-1H-pyrazol-5-yl)-6-cyclopropoxy-3-fluoro-4-(3-methoxypyrrolidin-1-yl)benzonitrile

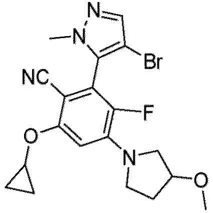

### Step 1: Synthesis of 2-bromo-4-chloro-3,6-difluorobenzonitrile

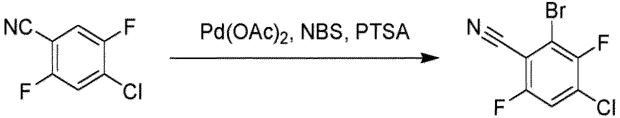

A mixture of 4-chloro-2,5-difluorobenzonitrile (25 g, 144.1 mmol), NBS (51.28 g, 288.1 mmol), palladium acetate (3.23 g, 14.4 mmol) and p-toluenesulfonic acid (11.59 mL, 72.0 mmol) in dichloroethane (250 mL) was degassed with nitrogen and then stirred at 75°C for 12 hours. The reaction solution was extracted with dichloroethane (300 mL) and water (300 mL) and washed with saturated brine (300 mL). The organic phase was dried over anhydrous sodium sulfate and concentrated under reduced pressure to remove the solvent. The remaining was separated by using column chromatography to obtain 2-bromo-4-chloro-3,6-difluorobenzonitrile (13.33 g, yield: 38%).

### Step 2: Synthesis of 2-bromo-4-chloro-6-cyclopropoxy-3-fluorobenzonitrile

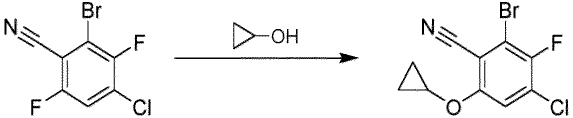

A mixture of 2-bromo-4-chloro-3,6-difluorobenzonitrile (15 g, 59.4 mmol), cyclopropanol (5.18 g, 89.1 mmol) and potassium carbonate (20.53 g, 148.6 mmol) in DMF (200 mL) was degassed with nitrogen and then stirred at 75°C for 2 hours. The reaction solution was extracted with ethyl acetate (200 mL) and water (200 mL) and washed with saturated brine (200 mL). The organic phase was dried over anhydrous sodium sulfate and concentrated under reduced pressure to remove the solvent. The remaining was separated by using column chromatography to obtain 2-bromo-4-chloro-6-cyclopropoxy-3-fluorobenzonitrile (8.1 g, yield: 47%).

¹H NMR (400 MHz, CDCl₃) δ 7.28 (d, *J* = 5.63 Hz, 1H) 3.77 (dt, *J* = 8.63, 4.44 Hz, 1H) 0.83 (br d, *J* = 4.63 Hz, 4H).

### Step 3: Synthesis of 4-chloro-6-cyclopropoxy-3-fluoro-2-(1-methyl-1H-pyrazol-5-yl)benzonitrile

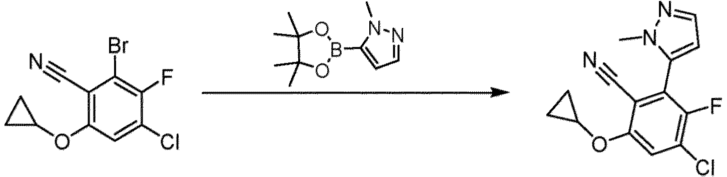

2-bromo-4-chloro-6-cyclopropoxy-3-fluorobenzonitrile (5.0 g, 17.21 mmol), 1-methyl-5-tetramethyl-1,3,2-dioxaborinane-2-pyrazol and cesium carbonate (16.82 g, 51.63 mmol) were dissolved in water (20 mL) and dioxane (100 mL). It was degassed with nitrogen, Pd-118 (1.12 g, 1.72 mmol) was added, and it was stirred at 80°C for 12 hours. The reaction solution was extracted with ethyl acetate (50 mL) and water (50 mL) and washed with saturated brine (50 mL). The organic phase was dried over anhydrous sodium sulfate and concentrated under reduced pressure to remove the solvent. The remaining was separated by using column chromatography to obtain 4-chloro-6-cyclopropoxy-3-fluoro-2-(1-methyl-1*H*-pyrazol-5-yl)benzonitrile (2.5 g, 50% purity).

¹H NMR (400 MHz, CDCl₃) δ 7.63 (d, *J* = 2.01 Hz, 1H), 7.50 (d, *J* = 5.77 Hz, 1H), 6.52 (d, *J* = 1.76 Hz, 1H), 3.89 (dt, *J=* 8.91, 4.33 Hz, 1H), 3.83 (d, *J* = 1.00 Hz, 3H), 0.96 (m, 4H).

### Step 4: Synthesis of 2-(4-bromo-1-methyl-1H-pyrazol-5-yl)-4-chloro-6-cyclopropoxy-3-fluorobenzonitrile

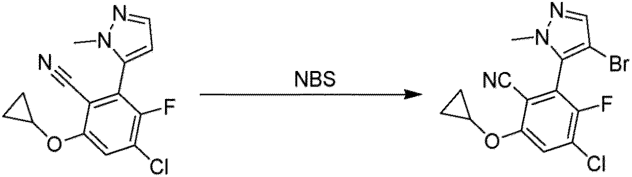

4-chloro-6-cyclopropoxy-3-fluoro-2-(1-methyl-1*H*-pyrazol-5-yl)benzonitrile (4.8 g, 16.46 mmol) and NBS (5.86 g, 32.91 mmol) were dissolved in acetonitrile (50 mL), and it was stirred under nitrogen atmosphere at 40°C for 2 hours. The reaction solution was extracted with ethyl acetate (50 mL) and water (50 mL), and washed with saturated brine (50 mL). The organic phase was dried over anhydrous sodium sulfate and concentrated under reduced pressure to remove the solvent. The remaining was separated by using column chromatography to obtain 2-(4-bromo-1-methyl-1*H*-pyrazol-5-yl)-4-chloro-6-cyclopropoxy-3-fluorobenzonitrile (5 g, 93% purity, yield: 76%).

¹H NMR (400 MHz, CDCl₃) δ 7.54 (s, 1H), 7.48 (d, *J* = 5.88 Hz, 1H), 3.82 (m, 1H), 3.73 (s, 3H), 0.92-0.83 (m, 4H).

### Step 5: Synthesis of 2-(4-bromo-1-methyl-1H pyrazol-5-yl)-6-cyclopropoxy-3-fluoro-4-(3-methoxypyrrolidin-1-yl)benzonitrile

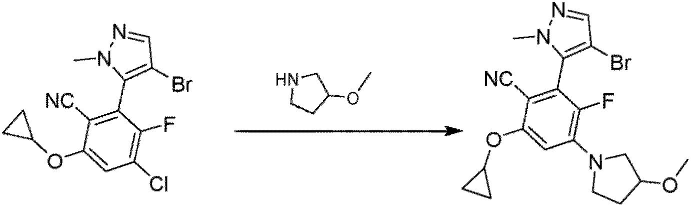

2-(4-bromo-1-methyl-1H-pyrazol-5-yl)-4-chloro-6-cyclopropoxy-3-fluorobenzonitrile (80 mg, 0.22 mmol) was dissolved in N-methylpyrrolidone (2 mL), 3-methoxypyrrolidine (59 mg, 0.43 mmol) and N,N-diisopropylethylamine (134 mg, 1.08 mmol) were added and it was reacted in a sealed tube and stirred overnight at 130°C. After the reaction was completed, it was quenched by the addition of water and extracted with ethyl acetate. The organic phase was combined and washed with water, and then it was dried and concentrated. The crude product was separated by using column chromatography to obtain 2-(4-bromo-1-methyl-1H-pyrazol-5-yl)-6-cyclopropoxy-3-fluoro-4-(3-methoxypyrrolidin-1-yl)benzonitrile (85 mg, yield: 79%). MS m/z (ESI): 435/437 [M+H]⁺.

### Intermediates 25-34 can be prepared by selecting the corresponding raw materials with reference to all or part of the synthesis methods of intermediate 24:

| **Intermediate No.** | **Structural Formula** | **Name** | **[M+H]**⁺ |
|---|---|---|---|
| **25** | 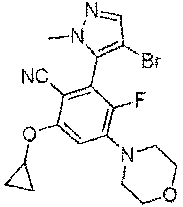 | 2-(4-bromo-1-methyl- 1H-pyrazol-5-yl)-6-cyclopropoxy-3-fluoro-4-morpholinobenzonitrile | 421/423 |
| **26** | 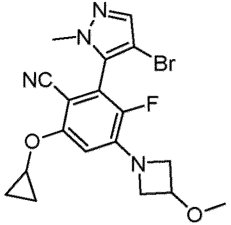 | 2-(4-bromo-1-methyl- 1H-pyrazol-5-yl)-6-cyclopropoxy-3-fluoro-4-(3-methoxyazetidin-1-yl)benzonitrile | 421/423 |
| **27** | 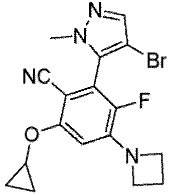 | 4-(azetidin-1-yl)-2-(4-bromo-1-methyl-1H-pyrazol-5-yl)-6-cyclopropoxy-3-fluorobenzonitrile | 391/393 |
| **28** | 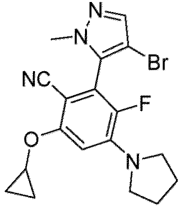 | 2-(4-bromo-1-methyl- 1H-pyrazol-5-yl)-6-cyclopropoxy-3-fluoro-4-(pyrrolidin-1 - yl)benzonitrile | 405/407 |
| **29** | 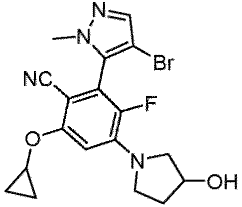 | 2-(4-bromo-1-methyl- 1H-pyrazol-5-yl)-6-cyclopropoxy-3-fluoro-4-(3 -hydroxypyrrolidin-1 - yl)benzonitrile | 421/423 |
| **30** | 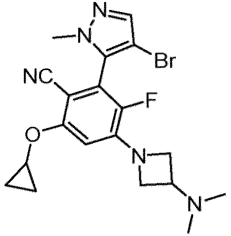 | 2-(4-bromo-1-methyl- 1H-pyrazol-5-yl)-6-cyclopropoxy-4-(3-(dimethylamino)azetidin-1-yl)-3-fluorobenzonitrile | 434/436 |
| **31** | 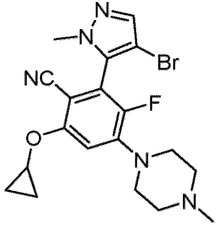 | 2-(4-bromo-1-methyl- 1H-pyrazol-5-yl)-6-cyclopropoxy-3-fluoro-4-(4-methylpiperazine-1-yl)benzonitrile | 434/436 |
| **32** | 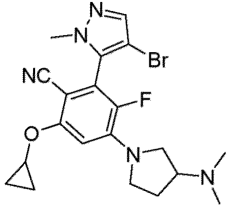 | 2-(4-bromo-1-methyl- 1H-pyrazol-5-yl)-6-cyclopropoxy-4-(3 -(dimethylamino)pyrrolidin-1-yl)-3-fluorobenzonitrile | 448/450 |
| **33** | 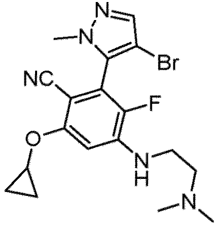 | 2-(4-bromo-1-methyl- 1H-pyrazol-5-yl)-6-cyclopropoxy-4-((2-(dimethylamino)ethyl)amino)-3-fluorobenzonitrile | 422/424 |
| **34** | 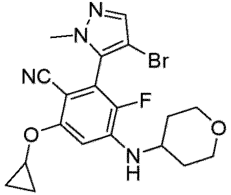 | 2-(4-bromo-1-methyl- 1H-pyrazol-5-yl)-6-cyclopropoxy-3-fluoro-4-((tetrahydro-2H-pyran-4-yl)amino)benzonitrile | 435/437 |

### II. Preparation of Specific Examples

### Example 1: Preparation of 1-(aminomethyl)-7-(5-(1-cyano-3-fluoronaphthalen-2-yl)-1-methyl-1H-pyrazol-4-yl)-4-oxo-3,4-dihydrophthalazine-5-carbonitrile

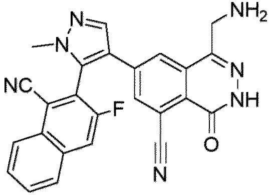

### Step 1: Synthesis of tert-butyl (tert-butoxycarbonyl)((5-cyano-7-(5-(1-cyano-3-fluoronaphthalen-2-yl)-1-methyl-1H-pyrazol-4-yl)-4-oxo-3,4-dihydrophthalazin-1-yl)methyl)carbamate

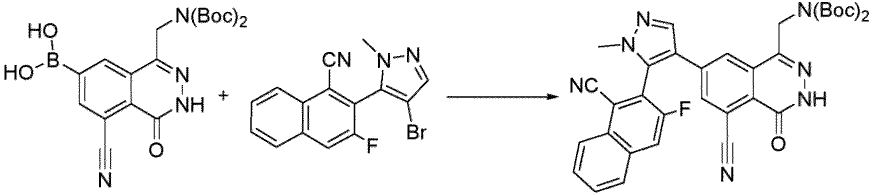

(4-((bis(tert-butoxycarbonyl)amino)methyl)-8-cyano-1 -oxo-1,2-dihydrophthalazin-6-yl)boronic acid (1.0 g, 70% purity, 1.58 mmol), 2-(4-bromo-1-methyl-1H-pyrazol-5-yl)-3-fluoro-1-naphthalenenitrile (594 mg, 1.58 mmol), 1,4-dioxane (20 mL), water (5 mL), sodium carbonate (501 mg, 4.73 mmol) and [1,1'-bis(diphenylphosphine)ferrocene]palladium dichloride dichloromethane complex (230 mg, 0.32 mmol) were added to a single-neck flask of 25 mL and stirred at 80°C for 3 hours. The reaction solution was concentrated under reduced pressure, and the residue was separated by using column chromatography to obtain tert-butyl (tert-butoxycarbonyl)((5-cyano-7-(5-(1-cyano-3-fluoronaphthalen-2-yl)-1-methyl-1*H*-pyrazol-4-yl)-4-oxo-3,4-dihydrophthalazin-1-yl)methyl)carbamate (450 mg, yield: 43%). MS m/z (ESI): 650 [M+H]⁺.

### Step 2: Synthesis of 1-(aminomethyl)-7-(5-(1-cyano-3-fluoronaphthalen-2-yl)-1-methyl-1H-pyrazol-4-yl)-4-oxo-3,4-dihydrophthalazine-5-carbonitrile

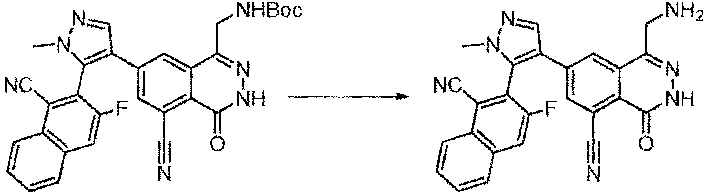

Tert-butyl ((5-cyano-7-(5-(1-cyano-3-fluoronaphthalen-2-yl)-1-methyl-1*H*-pyrazol-4-yl)-4-oxo-3,4-dihydrophthalazin-1-yl)methyl)carbamate (135 mg, 0.015 mmol) was dissolved in dichloromethane (3.0 mL), trifluoroacetic acid (2.0 mL) was added, and the reaction solution was stirred at room temperature for 1 hour. The reaction solution was concentrated under reduced pressure, the residue was diluted with ethyl acetate, the pH was adjusted with saturated sodium bicarbonate to 9-10, the solution was separated, and the aqueous phase was extracted with ethyl acetate. The organic phase was washed with saturated brine, dried over anhydrous sodium sulfate and filtered, and the filtrate was evaporated under reduced pressure to obtain 1-(aminomethyl)-7-(5-(1-cyano-3-fluoronaphthalen-2-yl)-1-methyl-1*H*-pyrazol-4-yl)-4-oxo-3,4-dihydrophthalazine-5-carbonitrile (96 mg, yield: 97%). MS m/z (ESI): 450 [M+H]⁺.

¹H NMR (400 MHz, DMSO-d*₆*) δ 12.75 (brs, 1H), 8.56 (d, *J* = 10.2 Hz, 1H), 8.48 (s, 1H), 8.30-8.24 (m, 2H), 8.20-8.12 (m, 1H), 7.91-7.85 (m, 3H), 3.83 (s, 3H), 3.46 (s, 2H).

Tert-butyl ((5-cyano-7-(5-(1-cyano-3-fluoronaphthalen-2-yl)-1-methyl-1H-pyrazol-4-yl)-4-oxo-3,4-dihydrophthalazin-1-yl)methyl)carbamate (380 mg, 0.585 mmol) was separated by using chiral column to obtain (*M*)-tert-butyl((5-cyano-7-(5-(1-cyano-3-fluoronaphthalen-2-yl)-1-methyl-1*H*-pyrazol-4-yl)-4-oxo-3,4-dihydrophthalazine-1-yl)methyl)carbamate (135 mg, yield: 35%) and (*P*)-tert-butyl((5-cyano-7-(5-(1-cyano-3-fluoronaphthalen-2-yl)-1-methyl-1*H*-pyrazol-4-yl)-4-oxo-3,4-dihydrophthalazine-1-yl)methyl)carbamate (182 mg, yield: 46%). Tert-butoxycarbonyl was subsequently removed with trifluoroacetic acid to obtain the corresponding final products Example 4 and Example 5. The specific data are shown in the table below.

### Examples 2-37 can be prepared by selecting the corresponding raw materials with reference to all or part of the synthesis methods of Example 1:

| **Example No.** | **Structural Formula** | **Name** | **MS m/z (ESI)** |
|---|---|---|---|
| **2** | 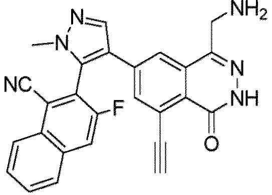 | 2-(4-(4-(aminomethyl)-8-ethynyl-1-oxo-1,2-dihydrophthalazin-6-yl)-1-methyl-1*H*-pyrazol-5-yl)-3-fluoro-1-naphthalenenitrile | 449 |
| **3** | 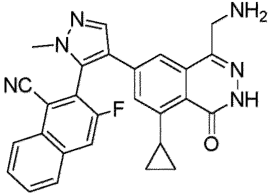 | 2-(4-(4-(aminomethyl)-8-cyclopropyl-1-oxo-1,2-dihydrophthalazin-6-yl)-1-methyl-1*H*-pyrazol-5-yl)-3-fluoro-1-naphthalenenitrile | 465 |
| **4** | 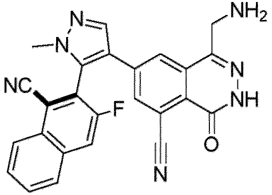 | (*P*)-1-(aminomethyl)-7-(5-(1-cyano-3-fluoronaphthalen-2-yl)-1-methyl-1*H*-pyrazol-4-yl)-4-oxo-3,4-dihydrophthalazine-5-carbonitrile | 450 |
| **5** | 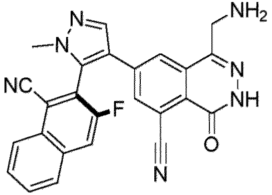 | (*M*)-1-(aminomethyl)-7-(5-(1-cyano-3-fluoronaphthalen-2-yl)-1-methyl-1*H*-pyrazol-4-yl)-4-oxo-3,4-dihydrophthalazine-5-carbonitrile | 450 |
| **6** | 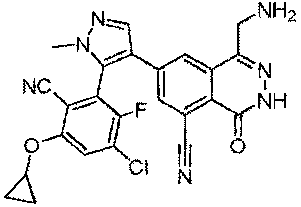 | 1-(aminomethyl)-7-(5-(3-chloro-6-cyano-5-cyclopropoxy-2-fluorophenyl)-1-methyl-1*H*-pyrazol-4-yl)-4-oxo-3,4-dihydrophthalazine-5-carbonitrile | 490 |
| **7** | 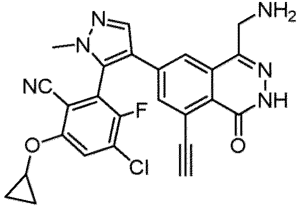 | 2-(4-(4-(aminomethyl)-8-ethynyl-1-oxo-1,2-dihydrophthalazin-6-yl)-1-methyl-1*H*-pyrazol-5-yl)-4-chloro-6-cyclopropoxy-3-fluorobenzonitrile | 489 |
| **8** | 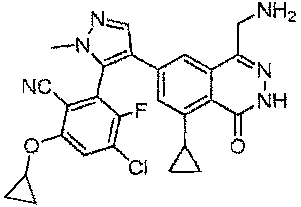 | 2-(4-(4-(aminomethyl)-8-cyclopropyl-1-oxo-1,2-dihydrophthalazin-6-yl)-1-methyl-1*H*-pyrazol-5-yl)-4-chloro-6-cyclopropoxy-3-fluorobenzonitrile | 505 |
| **9** | 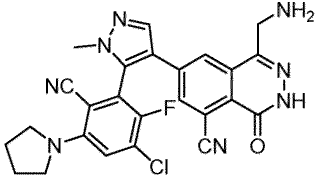 | 1-(aminomethyl)-7-(5-(3-chloro-6-cyano-2-fluoro-5-(pyrrolidin-1-yl)phenyl)-1- methyl-1*H*-pyrazol-4-yl)-4-oxo-3,4-dihydrophthalazine-5-carbonitrile | 503 |
| **10** | 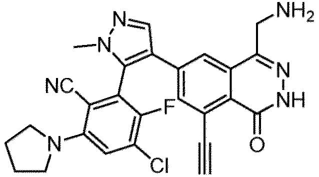 | 2-(4-(4-(aminomethyl)-8-ethynyl-1-oxo-1,2-dihydrophthalazin-6-yl)-1-methyl-1*H*-pyrazol-5-yl)-4-chloro-3 -fluoro-6-(pyrrolidin-1-yl)benzonitrile | 502 |
| **11** | 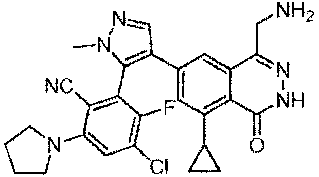 | 2-(4-(4-(aminomethyl)-8-cyclopropyl-1-oxo-1,2-dihydrophthalazin-6-yl)-1- methyl-1*H*-pyrazol-5-yl)-4-chloro-3-fluoro-6-(pyrrolidin-1-yl)benzonitrile | 518 |
| **12** | 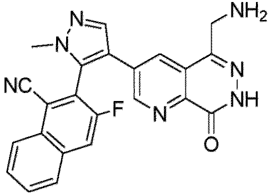 | 2-(4-(5-(aminomethyl)-8-oxo-7,8-dihydropyrido[2,3-d]pyridazin-3-yl)-1-methyl-1*H-*pyrazol-5-yl)-3-fluoro-1- naphthalenenitrile | 426 |
| **13** | 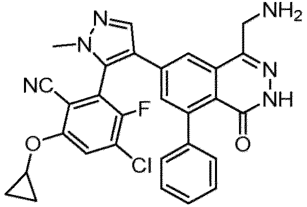 | 2-(4-(4-(aminomethyl)-1-oxo-8-phenyl-1,2-dihydrophthalazin-6-yl)-1-methyl-1*H*-pyrazol-5-yl)-4-chloro-6-cyclopropoxy-3-fluorobenzonitrile | 541 |
| **14** | 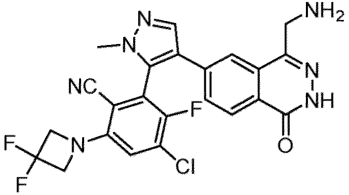 | 2-(4-(4-(aminomethyl)-1-oxo-1,2-dihydrophthalazin-6-yl)-1-methyl-1*H*-pyrazol-5-yl)-4-chloro-6-(3,3-difluoroazetidin-1-yl)-3-fluorobenzonitrile | 500 |
| | | | |
| **15** | 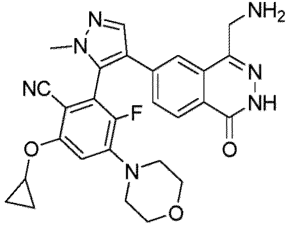 | 2-(4-(4-(aminomethyl)-1-oxo-1,2-dihydrophthalazin-6-yl)-1- methyl-1*H*-pyrazol-5-yl)-6-cyclopropoxy-3-fluoro-4-morpholinobenzonitrile | 516 |
| **16** | 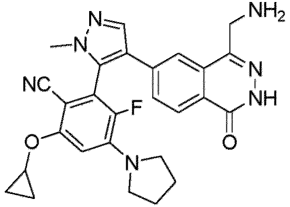 | 2-(4-(4-(aminomethyl)-1-oxo-1,2-dihydrophthalazin-6-yl)-1- methyl-1*H*-pyrazol-5-yl)-6-cyclopropoxy-3-fluoro-4-(pyrrolidin-1-yl)benzonitrile | 500 |
| **17** | 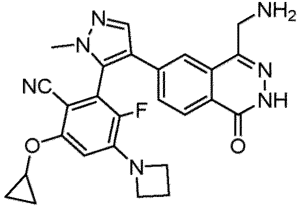 | 2-(4-(4-(aminomethyl)-1-oxo-1,2-dihydrophthalazin-6-yl)-1-methyl-1*H*-pyrazol-5-yl)-4-(azetidin-1-yl)-6-cyclopropoxy-3-fluorobenzonitrile | 486 |
| **18** | 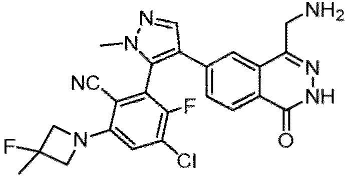 | 2-(4-(4-(aminomethyl)-1-oxo-1,2-dihydrophthalazin-6-yl)-1-methyl-1*H*-pyrazol-5-yl)-4-chloro-3-fluoro-6-(3-fluoro-3- methylazetidin-1-yl)benzonitrile | 496 |
| **19** | 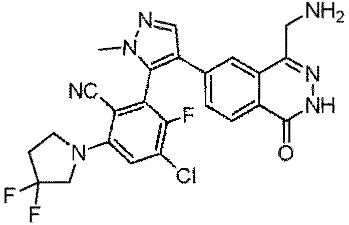 | 2-(4-(4-(aminomethyl)-1-oxo-1,2-dihydrophthalazin-6-yl)-1-methyl-1*H*-pyrazol-5-yl)-4-chloro-6-(3,3-difluoropyrrolidin-1-yl)-3-fluorobenzonitrile | 514 |
| **20** | 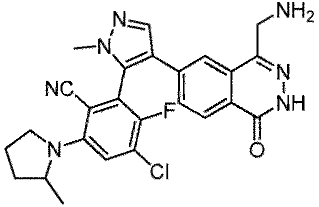 | 2-(4-(4-(aminomethyl)-1-oxo-1,2-dihydrophthalazin-6-yl)-1- methyl-1*H*-pyrazol-5-yl)-4-chloro-3-fluoro-6-(2-methylpyrrolidin-1-yl)benzonitrile | 492 |
| **21** | 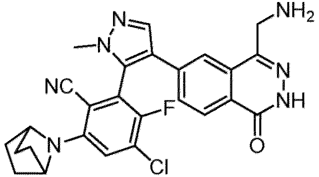 | 2-(4-(4-(aminomethyl)-1-oxo-1,2-dihydrophthalazin-6-yl)-1- methyl-1*H*-pyrazol-5-yl)-6-(7-azabicyclo[2.2.1]heptan-7-yl)-4-chloro-3-fluorobenzonitrile | 504 |
| **22** | 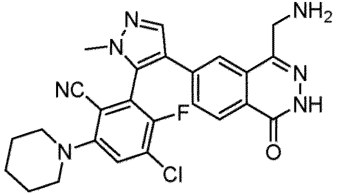 | 2-(4-(4-(aminomethyl)-1-oxo-1,2-dihydrophthalazin-6-yl)-1- methyl-1*H*-pyrazol-5-yl)-4-chloro-3-fluoro-6-(piperidin-1- yl)benzonitrile | 492 |
| **23** | 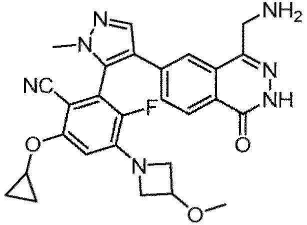 | 2-(4-(4-(aminomethyl)-1-oxo-1,2-dihydrophthalazin-6-yl)-1-methyl-1*H*-pyrazol-5-yl)-6-cyclopropoxy-3-fluoro-4-(3- methoxyazetidin-1-yl)benzonitrile | 516 |
| **24** | 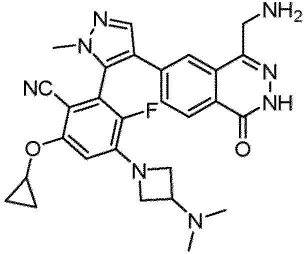 | 2-(4-(4-(aminomethyl)-1-oxo-1,2-dihydrophthalazin-6-yl)-1-methyl-1*H*-pyrazol-5-yl)-6-cyclopropoxy-4-(3-(dimethylamino)azetidin-1-yl)-3-fluorobenzonitrile | 529 |
| **25** | 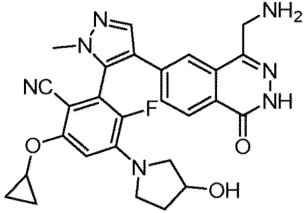 | 2-(4-(4-(aminomethyl)-1-oxo-1,2-dihydrophthalazin-6-yl)-1- methyl-1*H*-pyrazol-5-yl)-6-cyclopropoxy-3-fluoro-4-(3- hydroxypyrrolidin-1-yl)benzonitrile | 516 |
| **26** | 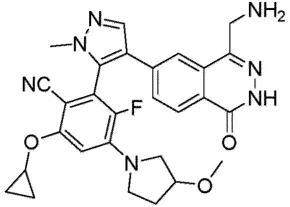 | 2-(4-(4-(aminomethyl)-1-oxo-1,2-dihydrophthalazin-6-yl)-1- methyl-1*H*-pyrazol-5-yl)-6-cyclopropoxy-3-fluoro-4-(3- methoxypyrrolidin-1-yl)benzonitrile | 530 |
| **27** | 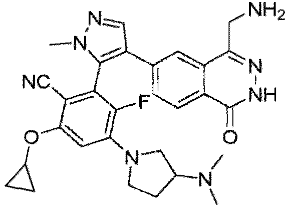 | 2-(4-(4-(aminomethyl)-1-oxo-1,2-dihydrophthalazin-6-yl)-1- methyl-1*H*-pyrazol-5-yl)-6-cyclopropoxy-4-(3-(dimethylamino)pyrrolidin-1-yl)-3-fluorobenzonitrile | 543 |
| **28** | 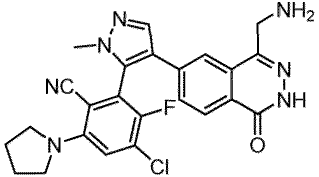 | 2-(4-(4-(aminomethyl)-1-oxo-1,2-dihydrophthalazin-6-yl)-1- methyl-1*H*-pyrazol-5-yl)-4-chloro-3-fluoro-6-(pyrrolidin-1-yl)benzonitrile | 478 |
| **29** | 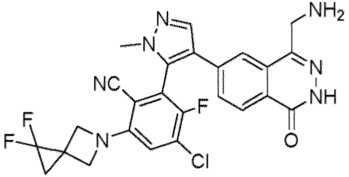 | 2-(4-(4-(aminomethyl)-1-oxo-1,2-dihydrophthalazin-6-yl)-1- methyl-1*H*-pyrazol-5-yl)-4-chloro-6-(1,1-difluoro-5-azaspiro[2.3]hexan-5-yl)-3-fluorobenzonitrile | 526 |
| **30** | 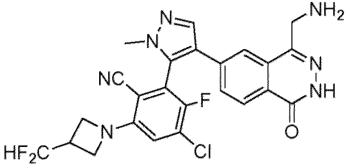 | 2-(4-(4-(aminomethyl)-1-oxo-1,2-dihydrophthalazin-6-yl)-1-methyl-1*H*-pyrazol-5-yl)-4-chloro-6-(3-(difluoromethyl)azetidin-1-yl)-3-fluorobenzonitrile | 514 |
| **31** | 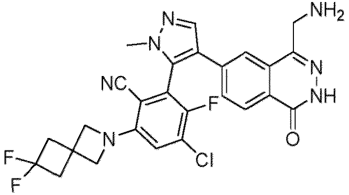 | 2-(4-(4-(aminomethyl)-1-oxo-1,2-dihydrophthalazin-6-yl)-1-methyl-1*H*-pyrazol-5-yl)-4-chloro-6-(6,6-difluoro-2-azaspiro[3.3]heptan-2-yl)-3- fluorobenzonitrile | 540 |
| **32** | 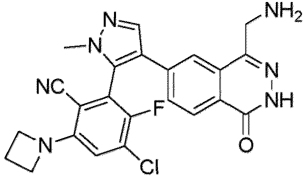 | 2-(4-(4-(aminomethyl)-1-oxo-1,2-dihydrophthalazin-6-yl)-1-methyl-1*H*-pyrazol-5-yl)-6-(azetidin-1-yl)-4-chloro-3- fluorobenzonitrile | 464 |
| **33** | 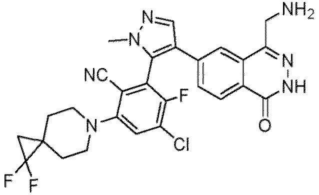 | 2-(4-(4-(aminomethyl)-1-oxo-1,2-dihydrophthalazin-6-yl)-1-methyl-1*H*-pyrazol-5-yl)-4-chloro-6-(1,1-difluoro-6-azaspiro[2.5]octan-6-yl)-3-fluorobenzonitrile | 554 |
| **34** | 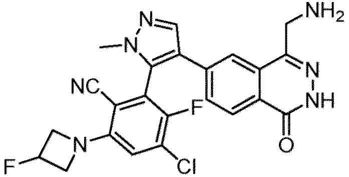 | 2-(4-(4-(aminomethyl)-1-oxo-1,2-dihydrophthalazin-6-yl)-1-methyl-1*H*-pyrazol-5-yl)-4-chloro-3-fluoro-6-(3- fluoroazetidin-1-yl)benzonitrile | 482 |
| **35** | 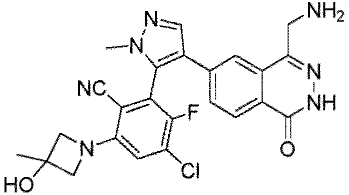 | 2-(4-(4-(aminomethyl)-1-oxo-1,2-dihydrophthalazin-6-yl)-1-methyl-1*H*-pyrazol-5-yl)-4-chloro-3-fluoro-6-(3-hydroxy-3-methylazetidin-1-yl)benzonitrile | 493 |
| **36** | 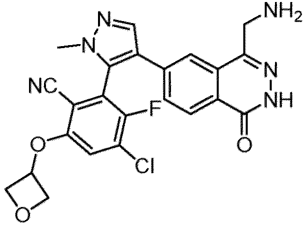 | 2-(4-(4-(aminomethyl)-1-oxo-1,2-dihydrophthalazin-6-yl)-1-methyl-1*H*-pyrazol-5-yl)-4-chloro-3-fluoro-6-(oxetan-3- oxy)benzonitrile | 481 |
| **37** | 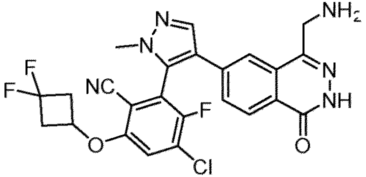 | 2-(4-(4-(aminomethyl)-1-oxo-1,2-dihydrophthalazin-6-yl)-1-methyl-1*H*-pyrazol-5-yl)-4-chloro-6-(3,3-difluorocyclobutoxy)-3-fluorobenzonitrile | 515 |

¹HNMR data for the compounds prepared in the above examples are as follows:

| **Example No.** | **¹HNMR** |
|---|---|
| **2** | (MeOH-*d*₄) δ 8.29 (d, *J* = 6.0 Hz, 1H), 8.28 (s, 1H), 8.28-8.15 (m, 2H), 7.83-7.80 (m, 2H), 7.68 (d, *J* = 1.2 Hz, 1H), 7.60 (d, *J* = 1.2 Hz, 1H), 3.86 (s, 3H), 3.81 (s, 1H), 3.82-3.68 (m, 2H). |
| **3** | (DMSO-*d*₆) δ 12.68 (s, 1H), 8.59 (d, *J* = 10.1 Hz, 1H), 8.44 (d, *J* = 0.9 Hz, 1H), 8.32 (br s, 3H), 8.31-8.26 (m, 1H), 8.21-8.15 (m, 1H), 7.96-7.87 (m, 2H), 7.75 (d, *J* = 1.6 Hz, 1H), 6.52 (d, *J* = 1.6 Hz, 1H), 4.46-4.30 (m, 2H), 3.82 (s, 3H), 3.62-3.54 (m, 1H), 0.72-0.56 (m, 2H), -0.09-0.18 (m, 1H), -0.30 - -0.41 (m, 1H). TFA salt. |
| **4** | (DMSO-*d*₆) δ 12.70 (s, 1H), 8.55 (d, *J* = 10.1 Hz, 1H), 8.48 (s, 1H), 8.31 (d, *J* = 1.7 Hz, 1H), 8.27-8.24 (m, 1H), 8.20-8.11 (m, 1H), 7.94-7.79 (m, 3H), 3.83 (s, 3H), 3.48-3.36 (m, 2H). |
| **5** | (DMSO-*d*₆) δ 12.68 (s, 1H), 8.55 (d, *J* = 10.2 Hz, 1H), 8.48 (s, 1H), 8.32 (d, *J* = 1.7 Hz, 1H), 8.29-8.21 (m, 1H), 8.20-8.11 (m, 1H), 7.93-7.80 (m, 3H), 3.83 (s, 3H), 3.45-3.35 (m, 2H). |
| **6** | (DMSO-*d*₆) δ 12.77 (s, 1H), 8.43 (s, 1H), 8.38 (d, *J =* 1.8 Hz, 1H), 8.03 (d, *J =* 6.0 Hz, 1H), 7.91 (d, *J* = 1.8 Hz, 1H), 4.23-4.18 (m, 1H), 3.80 (s, 3H), 3.77-3.65 (m, 2H), 0.92-0.90 (m, 2H), 0.81-0.79 (m, 2H). |
| **7** | (MeOH-*d*₄) δ 8.16 (s, 1H), 7.93 (d, *J* = 6.0 Hz, 1H), 7.68 (d, *J* = 1.2 Hz, 1H), 7.64 (d, *J* = 1.2 Hz, 1H), 4.10-4.08 (m, 1H), 4.01-3.99 (m, 2H), 3.94 (s, 1H), 3.83 (s, 3H), 0.94-0.90 (m, 2H), 0.85-0.82 (m, 2H). |
| **8** | (DMSO-*d*₆) δ 12.72 (s, 1H), 8.39 (s, 1H), 8.36 (brs, 3H), 8.10 (d, *J* = 6.0 Hz, 1H), 7.69 (d, *J* = 1.6 Hz, 1H), 6.64 (d, *J* = 1.6 Hz, 1H), 4.39 (d, *J* = 4.8 Hz, 2H), 4.27-4.19 (m, 1H), 3.79 (s, 3H), 3.77-3.70 (m, 1H), 1.03-0.91 (m, 4H), 0.88-0.82 (m, 1H), 0.79-0.73 (m, 1H), 0.25-0.09 (m, 2H). TFA salt. |
| **9** | (DMSO-*d*₆) δ 12.75 (s, 1H), 8.44 (s, 1H), 8.38 (d, *J* = 1.7 Hz, 1H), 7.97 (d, *J =* 1.8 Hz, 1H), 7.28 (d, *J* = 6.3 Hz, 1H), 3.79 (s, 3H), 3.71 (s, 2H), 3.63-3.44 (m, 4H), 1.96-1.88 (m, 4H). |
| **10** | (MeOH-*d*₄) δ 8.16 (s, 1H), 7.77 (d, *J* = 1.2 Hz, 1H), 7.70 (d, *J* = 1.2 Hz, 1H), 7.19 (d, *J* = 6.4 Hz, 1H), 4.04-4.00 (m, 2H), 3.95 (s, 1H), 3.83 (s, 3H), 3.62-3.51(m, 4H), 2.02-1.99 (m, 4H). |
| **11** | (DMSO-*d*₆) δ 12.69 (s, 1H), 8.37 (brs, 4H), 7.71 (d, *J* = 1.7 Hz, 1H), 7.31 (d, *J* = 6.3 Hz, 1H), 6.79 (d, *J* = 1.6 Hz, 1H), 4.41 (d, *J* = 5.2 Hz, 2H), 3.78 (s, 3H), 3.75-3.70 (m, 1H), 3.61-3.48 (m, 4H), 2.00-1.90 (m, 4H), 1.04-0.94 (m, 2H), 0.23 (q, *J* = 7.7 Hz, 2H). TFA salt. |
| **12** | (MeOH-*d*₄) δ 8.75 (d, *J* = 2.0 Hz, 1H), 8.34-8.33 (m, 2H), 8.26 (d, *J* = 2.0 Hz, 1H), 8.22-8.17 (m, 2H), 7.86-7.82 (m, 2H), 3.99-3.88 (m, 2H), 3.91 (s, 3H). |
| **13** | (MeOD-d₄) δ 8.20 (s, 1H), 7.89 (d, *J* = 6.0 Hz, 1H), 7.80 (d, *J* = 2.0 Hz, 1H), 7.33-7.31 (m, 3H), 7.17 (d, *J* = 2.0 Hz, 1H), 7.12-7.10 (m, 2H), 4.08-4.04 (m, 3H), 3.83 (s, 3H), 0.94-0.89 (m, 4H). |
| **14** | (DMSO-*d*₆) δ 12.90 (s, 1H), 8.38 (brs, 3H), 8.36 (s, 1H), 8.17 (d, *J* = 8.3 Hz, 1H), 7.91 (d, *J* = 1.7 Hz, 1H), 7.49 (dd, *J* = 8.3, 1.6 Hz, 1H), 7.31 (d, *J* = 6.1 Hz, 1H), 4.75-4.53 (m, 4H), 4.50-4.37 (m, 2H), 3.78 (s, 3H). TFA salt. |
| **15** | (MeOH-*d*₄) δ 8.26 (d, *J* = 8.3 Hz, 1H), 8.14 (s, 1H), 7.74-7.65 (m, 2H), 7.17 (d, *J* = 7.2 Hz, 1H), 4.12-4.02 (m, 1H), 3.95 (d, *J* = 3.3 Hz, 2H), 3.84 (t, *J =* 4.7 Hz, 4H), 3.81 (s, 3H), 3.37-3.31 (m, 4H), 0.97-0.86 (m, 2H), 0.86-0.76 (m, 2H). |
| **16** | (MeOH-*d*₄) δ 8.23 (d, *J* =8.4, 1H), 8.12 (s, 1H), 7.89 (d, *J* = 1.6 Hz, 1H), 7.56 (dd, *J* = 6.4, 1.6 Hz, 1H), 6.76 (d, *J* = 7.2, 1H), 4.53 (d, *J* = 3.2, 2H), 3.99-3.95 (m, 1H), 3.81 (s, 3H), 3.63-3.56 (m, 4H), 2.06-2.00 (m, 4H), 0.89-0.83 (m, 2H), 0.81-0.77 (m, 2H). |
| **17** | (DMSO-*d*₆) δ 12.57 (brs, 1H), 8.31-8.21 (m, 2H), 8.16 (d, *J* = 8.5 Hz, 1H), 7.77 (s, 1H), 7.67 (d, *J* = 8.3 Hz, 1H), 6.53 (d, *J* = 7.5 Hz, 1H), 4.17 (dd, *J* = 8.3, 18.3 Hz, 4H), 4.07 (d, *J* = 2.6 Hz, 1H), 3.95 (s, 2H), 3.73 (s, 3H), 2.40-2.35(m, 2H), 0.88-0.73 (m, 4H) |
| **18** | (CDCl₃) δ 10.38 (brs, 1H), 8.31 (s, 1H), 7.92 (s, 1H), 7.62 (s, 1H), 7.53 (s, 1H), 6.64 (d, *J =* 5.9 Hz, 1H), 4.38-4.26 (m, 1H), 4.25-4.13 (m, 3H), 4.12-3.99 (m, 2H), 3.83 (s, 3H), 1.68 (d, *J* = 21.5 Hz, 3H). |
| **19** | (CDCl₃) δ 10.16 (brs, 1H), 8.29 (s, 1H), 7.93 (s, 1H), 7.62 (s, 1H), 7.52 (s, 1H), 6.90 (s, 1H), 4.05 (s, 2H), 3.93 (q, *J* = 11.8 Hz, 2H), 3.84 (s, 3H), 3.81-3.74 (m, 1H), 3.74-3.64 (m, 1H), 2.54-2.38 (m, 2H). |
| **20** | (DMSO-*d*₆) δ 12.37 (s, 1H), 8.20 (s, 1H), 8.09 (d, *J* = 8.3 Hz, 1H), 7.71 (d, *J* = 1.6 Hz, 1H), 7.67 (dd, *J* = 8.3, 1.7 Hz, 1H), 7.24 (d, *J* = 6.3 Hz, 1H), 4.27-4.10 (m, 1H), 3.78-3.56 (m, 6H), 2.47 (s, 1H), 2.12-1.99 (m, 1H), 1.99-1.82 (m, 1H), 1.78-1.66 (m, 1H), 1.59-1.46 (m, 1H), 0.92 (d, *J* = 6.0 Hz, 3H). |
| **21** | (MeOH-*d*₄) δ 8.26 (d, *J* = 8.3 Hz, 1H), 8.15 (s, 1H), 7.73 (d, *J =* 1.6 Hz, 1H), 7.61 (dd, *J* = 8.3, 1.6 Hz, 1H), 7.46 (d, *J* = 6.3 Hz, 1H), 4.38 (t, *J* = 4.5 Hz, 2H), 4.08-3.93 (m, 2H), 3.84 (s, 3H), 1.92-1.67 (m, 4H), 1.59-1.43 (m, 4H). |
| **22** | (DMSO-*d*₆) δ 12.42-12.61 (m, 1H), 8.30 (s, 1H), 8.16 (d, *J* = 8.38 Hz, 1H), 7.60-7.77 (m, 3H), 3.83 (s, 2H), 3.79 (s, 3H), 3.05-3.18 (m, 4H), 1.62 (brs, 4H), 1.49-1.57 (m, 2H). |
| **23** | (MeOH-*d*₄) δ 8.26 (d, *J* = 8.3 Hz, 1H), 8.12 (s, 1H), 7.81-7.64 (m, 2H), 6.58 (d, *J* = 7.5 Hz, 1H), 4.49-4.28 (m, 3H), 4.13-4.03 (m, 1H), 4.03-3.93 (m, 4H), 3.81 (s, 3H), 3.33 (s, 3H), 0.92-0.85 (m, 2H), 0.85-0.72 (m, 2H). |
| **24** | (DMSO-*d*₆) δ 12.51 (brs, 1H), 8.23-8.32 (m, 1H), 8.16 (d, *J* = 8.38 Hz, 1H), 7.66-7.81 (m, 2H), 6.59 (d, *J* = 7.50 Hz, 1H), 4.14-4.28 (m, 2H), 4.04-4.12 (m, 1H), 3.90-4.01 (m, 2H), 3.86 (s, 2H), 3.74 (s, 3H), 3.17-3.23 (m, 1H), 2.11 (s, 6H), 0.73-0.92 (m, 4H). |
| **25** | (DMSO-*d*₆) δ 12.45 (brs, 1H), 8.25 (s, 1H), 8.16 (d, *J* = 6.9 Hz, 1H), 7.86-7.70 (m, 2H), 6.76 (d, *J* = 6.9 Hz, 1H), 4.39 (brs, 1H), 4.11 (brs, 1H), 3.85-3.48 (m, 8H), 3.43 (brs, 1H), 3.46-3.40 (m, 1H), 2.03-1.86 (m, 2H), 0.93-0.71 (m, 4H). |
| **26** | (MeOH-*d*₄) δ 8.26 (dd, *J* = 8.3, 4.1 Hz, 1H), 8.13 (d, *J* = 2.3 Hz, 1H), 7.81-7.69 (m, 2H), 6.79 (d, *J* = 7.4 Hz, 1H), 4.15-4.07 (m, 1H), 4.05-3.99 (m, 1H), 3.95 (s, 2H), 3.82 (d, *J* = 8.2 Hz, 3H), 3.79-3.51 (m, 4H), 3.35 (d, *J* = 12.5 Hz, 3H), 2.26-2.16 (m, 1H), 2.12-2.03 (m, 1H), 0.92-0.76 (m, 4H). |
| **27** | (DMSO-*d*₆) δ 12.47 (s, 1H), 8.25 (d, *J* = 3.25 Hz, 1H), 8.16 (dd, *J* = 8.25, 3.25 Hz, 1H), 7.70-7.81 (m, 2H), 6.77 (d, *J* = 7.38 Hz, 1H), 4.09-4.16 (m, 1H), 3.72-3.82 (m, 6H), 3.51-3.71 (m, 3H), 2.71-2.82 (m, 1H), 2.19 (d, *J* = 1.38 Hz, 6H), 2.07-2.18 (m, 2H), 1.74-1.88 (m, 1H), 0.87 (d, *J* = 6.00 Hz, 2H), 0.76 (brs, 2H). |
| **28** | (DMSO-*d*₆) δ 12.89 (s, 1H), 8.38 (s, 2H), 8.34 (s, 1H), 8.17 (d, *J* = 8.4 Hz, 1H), 7.90 (d, *J* = 1.7 Hz, 1H), 7.51 (dd, *J* = 8.4, 1.6 Hz, 1H), 7.26 (d, *J* = 6.3 Hz, 1H), 4.42 (s, 2H), 3.78 (s, 3H), 3.66-3.47 (m, 4H), 2.07-1.83 (m, 4H). |
| **29** | (MeOH-*d*₄) δ 8.27 (d, *J* = 8.4 Hz, 1H), 8.15 (s, 1H), 7.74 (d, *J* = 1.7 Hz, 1H), 7.67 (dd, *J* = 8.3, 1.7 Hz, 1H), 7.05 (d, *J* = 6.1 Hz, 1H), 4.40 (d, *J* = 8.2 Hz, 1H), 4.38-4.17 (m, 3H), 4.04 (d, *J* = 2.0 Hz, 2H), 3.84 (s, 3H), 1.62 (t, *J* = 8.8 Hz, 2H). |
| **30** | (MeOH-*d*₄) δ 8.27 (d, *J* = 8.3 Hz, 1H), 8.14 (s, 1H), 7.78-7.64 (m, 2H), 7.00 (d, *J* = 6.1 Hz, 1H), 6.30-6.00 (m, 1H), 4.37-4.25 (m, 2H), 4.23-4.16 (m, 1H), 4.16-4.09 (m, 1H), 4.01 (d, *J* = 3.1 Hz, 2H), 3.83 (s, 3H), 3.25-3.08 (m, 1H). |
| **31** | (MeOH-*d*₄) δ 8.26 (d, *J* = 8.3 Hz, 1H), 8.14 (s, 1H), 7.73 (d, *J* = 1.6 Hz, 1H), 7.67 (dd, *J* = 8.4, 1.6 Hz, 1H), 6.95 (d, *J* = 6.1 Hz, 1H), 4.34-4.20 (m, 4H), 4.06-3.96 (m, 2H), 3.82 (s, 3H), 2.81 (t, *J* = 12.0 Hz, 4H). |
| **32** | (MeOH-*d*₄) δ 8.18 (d, *J* = 8.3 Hz, 1H), 8.05 (s, 1H), 7.68-7.56 (m, 2H), 6.81 (d, *J* = 6.2 Hz, 1H), 4.22-4.01 (m, 4H), 3.93 (d, *J* = 2.1 Hz, 2H), 3.73 (s, 3H), 2.39-2.20 (m, 2H). |
| **33** | (MeOH-*d*₄) δ 8.26 (d, *J* = 8.3 Hz, 1H), 8.16 (s, 1H), 7.71 (d, *J* = 1.6 Hz, 1H), 7.64 (dd, *J* = 8.3, 1.7 Hz, 1H), 7.58 (d, *J* = 6.4 Hz, 1H), 4.05-3.93 (m, 2H), 3.84 (s, 3H), 3.27-3.09 (m, 4H), 1.90-1.67 (m, 4H), 1.20 (t, *J* = 8.5 Hz, 2H). |
| **34** | (DMSO-*d*₆) δ 12.44 (s, 1H), 8.27 (s, 1H), 8.16 (d, *J* = 8.3 Hz, 1H), 7.80 (d, *J =* 1.7 Hz, 1H), 7.72 (dd, *J* = 8.4, 1.7 Hz, 1H), 7.19 (d, *J* = 6.1 Hz, 1H), 5.58-5.35 (m, 1H), 4.61-4.38 (m, 2H), 4.35-4.11 (m, 2H), 3.81 (s, 2H), 3.77 (s, 3H). |
| **35** | (DMSO-*d*₆) δ 12.66 (brs, 1H), 8.31 (s, 1H), 8.17 (d, *J* = 8.28 Hz, 1H), 7.85 (d, *J =* 1.25 Hz, 1H), 7.60 (dd, *J* = 8.41, 1.38 Hz, 1H), 7.11 (d, *J* = 6.27 Hz, 1H), 5.74 (brs, 3H), 4.11 (s, 2H), 4.08-3.93 (m, 4H), 3.78 (s, 3H), 1.43 (s, 3H) |
| **36** | (DMSO-*d*₆) δ 12.90 (s, 1H), 8.37 (brs, 3H), 8.35 (s, 1H), 8.15 (d, *J* = 8.4 Hz, 1H), 7.88 (d, *J* = 1.7 Hz, 1H), 7.54 (d, *J* = 5.8 Hz, 1H), 7.47 (dd, *J* = 8.3, 1.6 Hz, 1H), 5.57-5.49 (m, 1H), 5.04-4.97 (m, 2H), 4.66-4.56 (m, 2H), 4.47-4.35 (m, 2H), 3.80 (s, 3H). |
| **37** | (DMSO-*d*₆) δ 12.45 (s, 1H), 8.29 (s, 1H), 8.16 (d, *J* = 8.3 Hz, 1H), 7.82-7.74 (m, 2H), 7.70 (d, *J* = 8.3 Hz, 1H), 5.03 (d, *J* = 9.5 Hz, 1H), 3.89-3.72 (m, 6H), 2.93-2.73 (m, 3H). |

### Biological Test Evaluation

### I. Human Colon Cancer HCT116 Cell Proliferation Inhibition Test

1. HCT 116 MTAP knockout and MTAP wild-type cells were plated in 96-well flat bottom plates, and cultured overnight with McCoy's 5A medium containing 10% fetal bovine serum + 1% penicillin-streptomycin at 37°C and 5% CO₂.
2. On the next day, the compound was dissolved with DMSO, diluted with DMSO and medium successively and transferred to the cell plate. The final concentration of the compound was 10 µM, the compound was diluted 4 times, with 9 concentration gradients, and DMSO control was added.
3. Cells that have not been treated with the compound were removed. CellTiter-Glo Luminescent Cell Viability Assay (Promega) was used to detect cell viability, the operation was performed with reference to the package insert of the kit, and then the cell plate was placed on EnVision Multilabel Reader to detect the cold light signal.
4. Meanwhile, the cell plate treated with the compound was cultured at 37°C and 5% CO₂ for 6 consecutive days.
5. Then, CellTiter-Glo was also used to detect cell viability.
6. Finally, the four-parameter dose-response curve module of GraphPad Prism v 9.2.0 software was used to plot the dose-response curve and calculate the IC₅₀ for proliferation inhibition (unit: nM).

| | | | | | |
|---|---|---|---|---|---|
| **Example No.** | **HCT116 wild-type cells IC₅₀ (nM)** | **HCT116 MTAP knockout cells IC₅₀ (nM)** | **Example No.** | **HCT116 wild-type cells IC₅₀ (nM)** | **HCT116 MTAP knockout cells IC₅₀ (nM)** |
| **1** | 2360 | 29 | **20** | NT | 61 |
| **2** | NT | 9 | **21** | NT | 161 |
| **3** | 1048 | 29 | **22** | NT | 116 |
| **4** | NT | 188 | **23** | 3655 | 120 |
| **5** | NT | 16 | **24** | NT | 229 |
| **6** | NT | 70 | **25** | NT | 567 |
| **7** | NT | 27 | **26** | 2493 | 34 |
| **8** | NT | 18 | **27** | NT | 262 |
| **9** | NT | 58 | **28** | 761 | 17 |
| **10** | NT | 12 | **29** | 1151 | 20 |
| **11** | NT | 184 | **30** | 2663 | 44 |
| **12** | NT | 1968 | **31** | NT | 93 |
| **13** | NT | 122 | **32** | NT | 57 |
| **14** | 2355 | 14 | **33** | NT | 57 |
| **15** | NT | 100 | **34** | NT | 84 |
| **16** | 2542 | 21 | **35** | NT | 922 |
| **17** | NT | 113 | **36** | NT | NT |
| **18** | 5600 | 123 | **37** | NT | 133 |
| **19** | 3264 | 80 | **MRTX-1979** | 1607 | 53 |
| **Notes** | 1. "NT" is an abbreviation of "Not Tested", which means that it has not been tested for the time being. | | | | |
| | 2. Positive compound MRTX-1979: 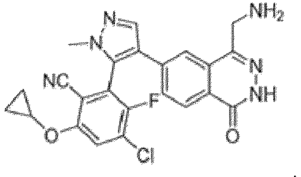 | | | | |

It can be seen from the biological activity data of the compounds of specific examples that the series of compounds of the present invention have a strong inhibitory effect on the proliferation of human colon cancer HCT116 MTAP knockout cells at the cellular level, and a weak inhibitory effect on MTAP wild-type HCT116 cells, which has higher selectivity than the positive compound MRTX-1979.

### Pharmacokinetic Assay in Mice

### 1. Study Purpose

The purpose of this trial is to study the pharmacokinetic behavior of some compounds of the present invention, and the administration method is: Single oral administration (PO) to ICR mice:

### 2. Experimental Protocol

### 2.1 Experimental Drugs

The compounds used in this trial are from the compounds in the specific examples of the present invention.

### 2.2 Experimental Animals

ICR mice male N=3 original source: Shanghai Sippr-Bk Lab Animal Co., Ltd.

### 2.3 Drug Preparation and Administration

The compounds were weighed and dissolved in 20% PG + 10% Solutol HS15 + 70% pH 3 citrate buffer, respectively, shaken well and sonicated to prepare colorless clear solutions or suspensions. Nine mice were administered orally at a dose of 10 mg/kg after an overnight fast.

### 2.4 Sample Collection:

About 90 µL of blood was collected via the submandibular vein per time point, heparin sodium anticoagulant was added, the sample was placed on ice after collection, and plasma was centrifuged within 1 hour (centrifugation conditions: 8,000 rpm, 6 minutes, 2-8°C). The blood collection time points were 0, 0.25, 0.5, 1, 2, 4, 6, 8 and 24 h. The samples were stored in a refrigerator at -20°C.

The plasma sample was 40 µL, 160 µL of ice-cold acetonitrile containing internal standard was added, vortexed for 3 minutes, and centrifuged at 11,000 rpm for 5 minutes. 100 µL of the supernatant was taken and added to 100 µL of water, 5 µL of the sample was taken and injected into LC/MS/MS for analysis (see the table below for the results).

### 2.5 Test Results:

### PK Results for Single Oral Administration (PO) of 10 mg/kg of Compounds to Mice

| **Example No.** | **Cₘₐₓ (ng/mL)** | **AUCₗₐₛₜ (hr*ng/mL)** | **T_{1/2} (h)** |
|---|---|---|---|
| Example 1 | 1753 | 2533 | 1.2 |
| Example 2 | 378 | 1439 | 4.0 |
| Example 6 | 973 | 2896 | 5.1 |
| Example 8 | 576 | 2714 | 3.2 |
| Example 9 | 844 | 2228 | 6.9 |
| Example 28 | 810 | 2979 | 5.0 |
| MRTX-1979 | 266 | 973 | 1.3 |

**Experimental Conclusion:** In the in vivo pharmacokinetic test in mice, the compounds of the examples of the present invention had a significant improvement in Cₘₐₓ and AUC by the route of oral administration compared with the positive compound MRTX-1979, showing better pharmacokinetic properties and better development prospects.

### Liver Microsomal Stability Experiment

### I. Preparation of Reagents

### 1. Preparation of Buffer Solution C:

Buffer solution A: 1.0 L of 0.1 M potassium dihydrogen phosphate buffer solution (containing 1.0 mM EDTA);
Buffer solution B: 1.0 L of 0.1 M dipotassium phosphate buffer solution (containing 1.0 mM EDTA);
Buffer solution C: Buffer solution A was added to 700 mL of buffer solution B. The addition was stopped when pH reached 7.4.

### 2. Preparation of 10 mM Stock Solution:

The selected compounds of the examples and reference standards were dissolved in DMSO to prepare a stock solution of 10 mM.

### 3. Preparation of Dosing Solution:

■ 500 µM solution: 10 µL of 10 mM stock solution was added to 190 µL of ACN;
■ 1.5 µM dosing solution (dissolved in liver microsome solution, human liver microsomes were purchased from Xenotech): 18.75 µL of 20 mg/mL liver microsomes was added to 479.75 µL of buffer solution C, then 1.5 µL of 500 µM solution was added and mixed well with gentle shaking.

### 4. Preparation of 6 mM NADPH Solution:

NADPH was weighted, then an appropriate amount of buffer solution C was added to prepare a 6 mM of NADPH solution.

### II. Specific Experimental Protocol

**1.** 30 µL of 1.5 µM dosing solution was added to the wells set to different time points (0 min, 5 min, 15 min, 30 min and 45 min) on a 96-well plate. 2 replicates were made.
**2.** Preparation of the 0-min sample: 135 µL of ACN (containing internal standard) was first added to the 0-min well, and 15 µL of 6 mM NADPH solution was added.
**3.** A 96-well plate containing 1.5 µM of dosing solution and NADPH solution were pre-warmed in a 37°C water bath for 5 minutes.
**4.** Pre-warmed 15 µL of 6 mM NADPH solution was added to the wells set to 5 min, 15 min, 30 min and 45 min, the reaction was started and the timing was started.
**5.** When the timer showed 5 min, 15 min, 30 min and 45 min, 135 µL of ACN (containing internal standard) was added to stop the reaction. The solution was vortexed for 10 minutes, and the sample was centrifuged on a centrifuge at 5594 ×g for 15 minutes.
**6.** 50 µL of supernatant was taken from the centrifuged sample, transfered to a 96-well sample plate that has been added with 50 µL of water, mixed, and finally sent to LC-MS/MS for analysis. The half-life was calculated based on the test results. See the table below for the experimental results:

| **Example No.** | **Human Liver Microsome Stability Half-Life T_{1/2}, (min)** |
|---|---|
| Example 1 | 202 |
| Example 2 | 43 |
| Example 6 | 123 |
| Example 8 | 52 |
| Example 9 | 38 |
| Example 28 | 27 |
| MRTX-1979 | 20 |

**Experimental Conclusion:** In the in vitro human liver microsome stability experiment, the half-life T_{1/2} of the compounds of the examples of the present invention was significantly improved compared with the positive compound MRTX-1979, showing better in vitro metabolic stability and better development prospects.

All documents mentioned in the present invention are incorporated by reference in this application, just as each document is cited separately as a reference. In addition, it should be understood that various modifications or changes may be made by those skilled in the art after reading the above disclosed content of the present invention, and these equivalent forms also fall within the scope defined by the claims appended hereto.

## Claims

1. A compound of formula (I), a stereoisomer or pharmaceutically acceptable salt thereof: 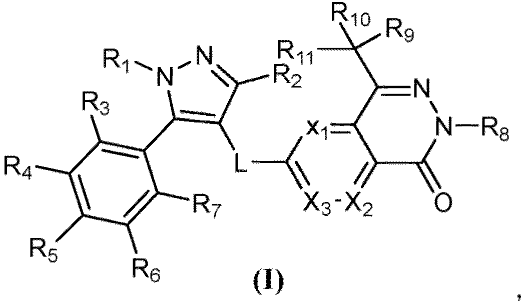
wherein, L is a bond, O, S, C(O), C(O)O, C(O)NH, CH=CH, C=C, S(O), S(O)₂, NR₁₂, S(O)₂NH or CR₁₃R₁₄;
X₁, X₂ and X₃ are each independently N or CR₁₅;
R₁ is selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, and above groups are further optionally and independently substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
R₂ is selected from the group consisting of hydrogen, deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
R₃, R₄, R₅, R₆ and R₇ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, or, two adjacent substituents of R₃, R₄, R₅, R₆ and R₇, together with a moiety to which they are directly attached thereto, form 4-8 membered carbocyclic ring, 4-8 membered heterocycle, 6-8 membered aromatic ring or 5-8 membered heteroaromatic ring, and above groups are further optionally and independently substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkylS(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
Rs is selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, and above groups are further optionally and independently substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
R₉ is selected from the group consisting of hydrogen, deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
R₁₀ and R₁₁ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, or, R₁₀ and R₁₁, together with a carbon atom directly attached thereto, form C(O), C₃₋₁₀ cycloalkyl, 3-10 membered heterocyclyl, C₆₋₁₀ aryl or 5-8 membered heteroaryl, and above groups are further optionally and independently substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkylS(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
R₁₂ is selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, and above groups are further optionally and independently substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
R₁₃ and R₁₄ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, or, R₁₃ and R₁₄, together with a carbon atom directly attached thereto, form C(O), C₃₋₁₀ cycloalkyl, 3-10 membered heterocyclyl, C₆₋₁₀ aryl or 5-8 membered heteroaryl, and above groups are further optionally and independently substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkylS(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
each R₁₅ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SFs, -C₀₋₈ alkylS(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, and above groups are further optionally and independently substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁, provided that when R₁₅ is hydrogen, at least one of R₃, R₄, Rs, R₆ and R₇ is 3-8 membered nitrogen-containing heterocyclyl, and the nitrogen atom is attached to the benzene ring;
each R₁₆ is independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₉, -C₀₋₈ alkyl-C(O)OR₂₀, -C₀₋₈ alkyl-C(O)R₂₁ or -C₀₋₈ alkyl-C(O)NR₂₂R₂₃, and above groups are further optionally substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, -C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
each R₁₇ and each R₁₈ are independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl or 5-10 membered heteroaryl, or, R₁₇ and R₁₈, together with a sulfur atom directly attached thereto, form 3-10 membered heterocyclyl, and above groups are further optionally substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SFs, -C₀₋₈ alkyl-S(O)(=N-R₁₆)R₁₇, -C₀₋₈ alkyl-N=S(O)R₁₇R₁₈, -C₀₋₈ alkyl-N=SR₁₇R₁₈, -C₀₋₈ alkyl-O-S(O)₂R₁₉, -C₀₋₈ alkyl-S(O)ᵣR₁₉, -C₀₋₈ alkyl-P(O)(OR₂₀)R₁₉, -C₀₋₈ alkyl-O-R₂₀, -C₀₋₈ alkyl-C(O)OR₂₀, -C₀₋₈ alkyl-C(O)SR₂₀, -C₀₋₈ alkyl-S-C(O)R₂₁, -C₀₋₈ alkyl-C(O)R₂₁, - C₀₋₈ alkyl-O-C(O)R₂₁, -C₀₋₈ alkyl-NR₂₂R₂₃, -C₀₋₈ alkyl-C(=NR₂₂)R₂₁, -C₀₋₈ alkyl-N(R₂₂)-C(=NR₂₃)R₂₁, -C₀₋₈ alkyl-C(O)NR₂₂R₂₃ and -C₀₋₈ alkyl-N(R₂₂)-C(O)R₂₁;
each R₁₉ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₂₂R₂₃, and above groups are further optionally and independently substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
each R₂₀ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, and above groups are further optionally and independently substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
each R₂₁ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocyclyloxy, C₆₋₁₀ alkyl, C₆₋₁₀ aryloxy, 5-10 membered heteroaryl, 5-10 membered heteroaryloxy and -NR₂₂R₂₃, and above groups are further optionally and independently substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₂₂R₂₃;
each R₂₂ and each R₂₃ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₁₀ alkanoyl, and above groups are further optionally and independently substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkyl amino, di-C₁₋₁₀ alkyl amino and C₁₋₁₀ alkanoyl, or, R₂₂ and R₂₃, together with a nitrogen atom directly attached thereto, form 4-10 membered heterocyclyl or 5-10 membered heteroaryl, and the 4-10 membered heterocyclyl or 5-10 membered heteroaryl is further optionally substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkyl amino, di-C₁₋₁₀ alkyl amino and C₁₋₁₀ alkanoyl; and
each r is independently 0, 1, or 2.

2. The compound of formula (I), the stereoisomer or pharmaceutically acceptable salt thereof according to claim 1, wherein L is a bond, O, S, C(O), S(O), S(O)₂, NR₁₂, or CR₁₃R₁₄;
X₁, X₂ and X₃ are each independently N or CR₁₅;
Ri is selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, and 5-8 membered heteroaryl, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
R₂ is selected from the group consisting of hydrogen, deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
R₃, R₄, Rs, R₆ and R₇ are each independently selected from the group consisting of hydrogen, deuterium, halogen, cyano, nitro, azido, C₁₋₄ alkyl, C₂₋₄ alkenyl, C₂₋₄ alkynyl, C₃₋₈ cycloalkyl, 3-8 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, or, two adjacent substituents of R₃, R₄, Rs, R₆ and R₇, together the moiety to which they are directly attached thereto, form 5-8 membered carbocyclic ring, 5-8 membered heterocycle, 6 membered aromatic ring or 5-6 membered heteroaromatic ring, and above groups are further optionally and independently substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -C₀₋₄ alkyl-SF₅, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
Rs is selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, and 5-8 membered heteroaryl, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
R₉ is selected from the group consisting of hydrogen, deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
R₁₀ and R₁₁ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, or, R₁₀ and R₁₁, together with a carbon atom directly attached thereto, form C(O), C₃₋₆ cycloalkyl, 3-6 membered heterocyclyl, C₆₋₈ aryl or 5-6 membered heteroaryl, and above groups are further optionally and independently substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
R₁₂ is selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, and 5-8 membered heteroaryl, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
R₁₃ and R₁₄ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, or, R₁₃ and R₁₄, together with a carbon atom directly attached thereto, form C(O), C₃₋₆ cycloalkyl, 3-6 membered heterocyclyl, C₆₋₈ aryl or 5-6 membered heteroaryl, and above groups are further optionally and independently substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
each R₁₅ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₆)R₁₇, - C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkyl-S(O)ᵣR₁₉, - C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, and above groups are further optionally and independently substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkylS(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, provided that when R₁₅ is hydrogen, at least one of R₃, R₄, Rs, R₆ and R₇ is 3-8 membered nitrogen-containing heterocyclyl, and the nitrogen atom is attached to the benzene ring;
wherein R₁₆, R₁₇, R₁₈, R₁₉, R₂₀, R₂₁, R₂₂, R₂₃, and r are defined as in claim 1.

3. The compound of formula (I), the stereoisomer or pharmaceutically acceptable salt thereof according to claim 1, wherein each R₁₆ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -S(O)ᵣR₁₉, -C(O)OR₂₀, -C(O)R₂₁ or - C(O)NR₂₂R₂₃, and above groups are further optionally substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₆)R₁₇, - N=S(O)R₁₇R₁₈, -N=SR₁₇R₁₈, -O-S(O)₂R₁₉, -S(O)ᵣR₁₉, -P(O)(OR₂₀)R₁₅, -O-R₂₀, -C(O)OR₂₀, - C(O)SR₂₀, -S-C(O)R₂₁, -C(O)R₂₁, -O-C(O)R₂₁, -NR₂₂R₂₃, -C(=NR₂₂)R₂₁, -N(R₂₂)-C(=NR₂₃)R₂₁, - C(O)NR₂₂R₂₃ and yl-N(R₂₂)-C(O)R₂₁;
each R₁₇ and each Ris are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl or 5-8 membered heteroaryl, or, R₁₇ and R₁₈, together with a sulfur atom directly attached thereto, form 4-6 membered heterocyclyl, and above groups are further optionally substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₆)R₁₇, -N=S(O)R₁₇R₁₈, -N=SR₁₇R₁₈, -O-S(O)₂R₁₉, -S(O)ᵣR₁₉, -P(O)(OR₂₀)R₁₉, -O-R₂₀, -C(O)OR₂₀, -C(O)SR₂₀, -S-C(O)R₂₁, -C(O)R₂₁, -O-C(O)R₂₁, -NR₂₂R₂₃, -C(=NR₂₂)R₂₁, - N(R₂₂)-C(=NR₂₃)R₂₁, -C(O)NR₂₂R₂₃ and -N(R₂₂)-C(O)R₂₁;
each R₁₉ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, 5-8 membered heteroaryl and -NR₂₂R₂₃, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
each R₂₀ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl, and 5-8 membered heteroaryl, and above groups are further optionally and independently substituted by one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
each R₂₁ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃, and above groups are further optionally and independently substituted by one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
each R₂₂ and each R₂₃ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₄ alkanoyl, and above groups are further optionally and independently substituted by one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, mono-C₁₋₄ alkyl amino, di-C₁₋₄ alkyl amino and C₁₋₄ alkanoyl, or, R₂₂ and R₂₃, together with a nitrogen atom directly attached thereto, form 4-6 membered heterocyclyl or 5-6 membered heteroaryl, and the 4-6 membered heterocyclyl or 5-6 membered heteroaryl is further optionally substituted by one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, mono-C₁₋₄ alkyl amino, di-C₁₋₄ alkyl amino and C₁₋₄ alkanoyl; and
each r is independently 0, 1, or 2.

4. The compound of formula (I), the stereoisomer or pharmaceutically acceptable salt thereof according to claim 1, wherein the compound of formula (I) is a compound with the structure shown as formula (II): 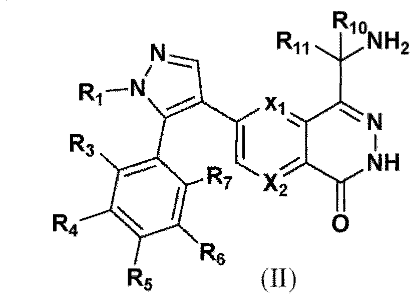
wherein X₁ is N or CH; X₂ is N or CR₁₅;
R₁ is selected from the group consisting of hydrogen, deuterium, C₁₋₄ alkyl, C₃₋₆ cycloalkyl, 3-6 membered heterocyclyl, and above groups are further optionally and independently substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₂₂R₂₃;
R₃, R₄, R₅, R₆ and R₇ are each independently selected from the group consisting of hydrogen, deuterium, halogen, cyano, C₁₋₄ alkyl, C₂₋₄ alkenyl, C₂₋₄ alkynyl, C₃₋₈ cycloalkyl, 3-8 membered heterocyclyl, C₆₋₈ aryl, 5-8 membered heteroaryl, -C₀₋₄ alkyl-SFs, -C₀₋₄ alkyl-S(O)(=N-R₁₆)R₁₇, - C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkyl-S(O)ᵣR₁₉, - C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)Rzi, or, two adjacent substituents of R₃, R₄, Rs, R₆ and R₇, together with a moiety to which they are directly attached thereto, form 5-8 membered carbocyclic ring, 5-8 membered heterocycle, 6 membered aromatic ring or 5-6 membered heteroaromatic ring, and above groups are further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkyl-S(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, - C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁;
R₁₀ and R₁₁ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SFs, -C₀₋₄ alkyl-O-R₂₀, -C₀₋₄ alkyl-C(O)OR₂₀, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(O)NR₂₂R₂₃ and -C₀₋₄ alkyl-N(R₂₂)-C(O)R₂₁, or, R₁₀ and R₁₁, together with a carbon atom directly attached thereto, form C(O), C₃₋₆ cycloalkyl, 3-6 membered heterocyclyl, C₆₋₈ aryl or 5-6 membered heteroaryl, and above groups are further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SFs, -C₀₋₄ alkyl-O-R₂₀, -C₀₋₄ alkyl-C(O)OR₂₀, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(O)NR₂₂R₂₃ and -C₀₋₄ alkyl-N(R₂₂)-C(O)R₂₁;
R₁₅ is selected from the group consisting of hydrogen, deuterium, cyano, C₂₋₄ alkenyl, C₂₋₄ alkynyl, C₃₋₈ cycloalkyl, 3-8 membered heterocyclyl, C₆₋₈ aryl, 5-8 membered heteroaryl, -C₀₋₄ alkyl-SFs, -C₀₋₄ alkyl-S(O)(=N-R₁₆)R₁₇, -C₀₋₄ alkyl-N=S(O)R₁₇R₁₈, -C₀₋₄ alkyl-N=SR₁₇R₁₈, -C₀₋₄ alkyl-O-S(O)₂R₁₉, -C₀₋₄ alkyl-S(O)ᵣR₁₉, -C₀₋₄ alkyl-P(O)(OR₂₀)R₁₉, -C₀₋₄ alkyl-O-R₂₀, -C₀₋₄ alkyl-C(O)OR₂₀, -C₀₋₄ alkyl-C(O)SR₂₀, -C₀₋₄ alkyl-S-C(O)R₂₁, -C₀₋₄ alkyl-C(O)R₂₁, -C₀₋₄ alkyl-O-C(O)R₂₁, -C₀₋₄ alkyl-NR₂₂R₂₃, -C₀₋₄ alkyl-C(=NR₂₂)R₂₁, -C₀₋₄ alkyl-N(R₂₂)-C(=NR₂₃)R₂₁, -C₀₋₄ alkyl-C(O)NR₂₂R₂₃ and -C₀₋₄ alkyl-N(R₂₂)-C(O)R₂₁, and above groups are further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -C₀₋₄ alkyl-SFs, -C₀₋₄ alkyl-O-R₂₀, -C₀₋₄ alkyl-C(O)OR₂₀, -C₀₋₄ alkyl-C(O)R₂₁, -C₀₋₄ alkyl-O-C(O)R₂₁, - C₀₋₄ alkyl-NR₂₂R₂₃, -C₀₋₄ alkyl-C(O)NR₂₂R₂₃ and -C₀₋₄ alkyl-N(R₂₂)-C(O)R₂₁, provided that when R₁₅ is hydrogen, at least one of R₃, R₄, Rs, R₆ and R₇ is 3-8 membered nitrogen-containing heterocyclyl, and the nitrogen atom is attached to the benzene ring;
wherein R₁₆, R₁₇, R₁₈, R₁₉, R₂₀, R₂₁, R₂₂, R₂₃, and r are defined as in claim 1.

5. The compound of formula (I), the stereoisomer or pharmaceutically acceptable salt thereof according to claim 1, wherein the compound of formula (I) is a compound with the structure shown as formula (III): 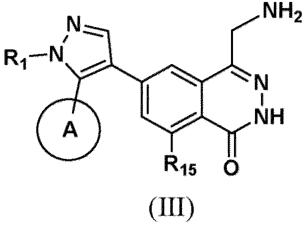
wherein ring A is of the following structure: 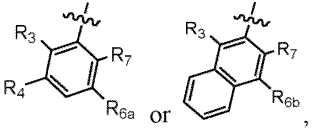
R₁ is selected from the group consisting of hydrogen, deuterium, methyl, ethyl, isopropyl, and cyclopropyl;
each R₃ and each R₇ are independently selected from the group consisting of hydrogen, deuterium, fluorine, chlorine, cyano, C₁₋₄ alkyl, C₃₋₈ cycloalkyl, 3-8 membered heterocyclyl, hydroxy, methoxy, ethoxy, isopropoxy, C₃₋₈ cycloalkoxy, 3-8 membered heterocyclyloxy, methylthio, ethylthio, carboxyl, methoxycarbonyl, ethoxycarbonyl, isopropoxycarbonyl, acetyl, acetoxy, amino, dimethylamino, acetamino, and carbamoyl;
R₁₅ is selected from the group consisting of cyano, ethynyl, cyclopropyl, and hydrogen, and
**when R₁₅ is selected from the group consisting of cyano, ethynyl, and cycloropyl and** ring A is 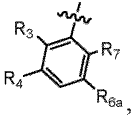 R₄ and R₆ₐ are each independently selected from the group consisting of halogen, C₁₋₄ alkoxy, C₃₋₈ cycloalkoxy, 3-8 membered heterocyclyl, and 3-8 membered heterocyclyloxy, and the C₁₋₄ alkoxy, C₃₋₈ cycloalkoxy, 3-8 membered heterocyclyl, or 3-8 membered heterocyclyloxy is each further optionally and independently substituted by one or more substituents selected from the group consisting of deuterium, halogen, cyano, C₁₋₄ alkyl, C₁₋4 haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀, -C(O)OR₂₀, -C(O)R₂₁, - O-C(O)R₂₁, -NR₂₂R₂₃, -C(O)NR₂₂R₂₃ and -N(R₂₂)-C(O)R₂₁;
**when R₁₅ is selected from the group consisting of cyano, ethynyl and cyclopropyl,** and ring A is 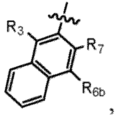 R_{6b} is selected from the group consisting of hydrogen, deuterium, fluorine, chlorine, cyano, C₁₋₄ alkyl, C₃₋₈ cycloalkyl, 3-8 membered heterocyclyl, hydroxy, methoxy, ethoxy, isopropoxy, C₃₋₈ cycloalkoxy, 3-8 membered heterocyclyloxy, carboxyl, amino, and dimethylamino;
**when R₁₅ is hydrogen** and ring A is 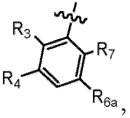 R₄ and R₆ₐ are each independently selected from halogen, C₁₋₄ alkoxy, C₃₋₈ cycloalkoxy, 3-8 membered heterocyclyl or 3-8 membered heterocyclyloxy, and the C₁₋₄ alkoxy, C₃₋₈ cycloalkoxy, 3-8 membered heterocyclyl or 3-8 membered heterocyclyloxy are each further optionally and independently substituted by one or more substituents selected from the group consisting of deuterium, halogen, cyano, C₁₋₄ alkyl, C₁₋4 haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀, -C(O)OR₂₀, -C(O)R₂₁, - O-C(O)R₂₁, -NR₂₂R₂₃, -C(O)NR₂₂R₂₃ and -N(R₂₂)-C(O)R₂₁, provided that at least one of R₄ and R₆ₐ is 3-8 membered nitrogen-containing heterocyclyl, the nitrogen atom is attached to the benzene ring, and the 3-8 membered nitrogen-containing heterocyclyl is further optionally substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀, -C(O)OR₂₀, -C(O)R₂₁, -O-C(O)R₂₁, -NR₂₂R₂₃, -C(O)NR₂₂R₂₃ and -N(R₂₂)-C(O)R₂₁;
wherein R₂₀, R₂₁, R₂₂ and R₂₃ are defined as in claim 1.

6. The compound of formula (I), the stereoisomer or pharmaceutically acceptable salt thereof according to claim 5, wherein the compound of formula (I) is a compound with the structure shown as formula (IVa): 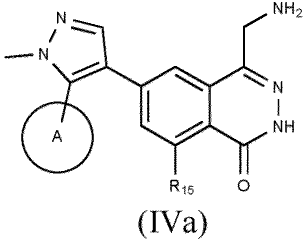
wherein R₁₅ is cyano, ethynyl, or cyclopropyl;
ring A is of the following structure: 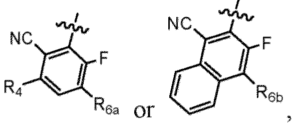
R₄ and R₆ₐ are each independently selected from the group consisting of halogen, C₁₋₄ alkoxy, C₃₋₈ cycloalkoxy, 3-8 membered heterocyclyl, or 3-8 membered heterocyclyloxy, and the C₁₋₄ alkoxy, C₃₋₈ cycloalkoxy, 3-8 membered heterocyclyl, or 3-8 membered heterocyclyloxy are further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀ and -NR₂₂R₂₃;
R_{6b} is selected from the group consisting of hydrogen, deuterium, fluorine, chlorine, cyano, C₁₋₄ alkyl, C₃₋₈ cycloalkyl, 3-8 membered heterocyclyl, hydroxy, methoxy, ethoxy, isopropoxy, C₃₋₈ cycloalkoxy, 3-8 membered heterocyclyloxy, carboxyl, amino, and dimethylamino;
wherein R₂₀, R₂₂, and R₂₃ are defined as in claim 5.

7. The compound of formula (I), the stereoisomer or pharmaceutically acceptable salt thereof according to claim 6, wherein R₄ and R₆ₐ are each independently selected from the group consisting of fluorine, chlorine, cyclopropoxy, cyclobutoxy or 3-8 membered nitrogen-containing heterocyclyl, and the cyclopropoxy, cyclobutoxy or 3-8 membered nitrogen-containing heterocyclyl is further optionally and independently substituted by one or more substituents selected from the group consisting of deuterium, fluorine, chlorine, cyano, methyl, ethyl, n-propyl, isopropyl, trifluoromethyl, difluoromethyl, trideuteriomethyl, dideuteriomethyl, vinyl, ethynyl, cyclopropyl, cyclobutyl, oxa-cyclobutyl, aza-cyclobutyl, phenyl, -SF₅, =O, hydroxy, methoxy, ethoxy, n-propyloxy, isopropyloxy, amino, mono-C₁₋₄ alkylamino and di-C₁₋₄ alkylamino;
R_{6b} is selected from the group consisting of hydrogen, deuterium, fluorine, chloride, cyano, methyl, ethyl, n-propyl, isopropyl, cyclopropyl, cyclobutyl, oxa-cyclobutyl, aza-cyclobutyl, hydroxy, methoxy, ethoxy, isopropoxy, cyclopropoxy, cyclobutoxy, carboxyl, amino, and dimethylamino.

8. The compound of formula (I), the stereoisomer or pharmaceutically acceptable salt thereof according to claim 5, wherein the compound of formula (I) is a compound with the structure shown as formula (IVb): 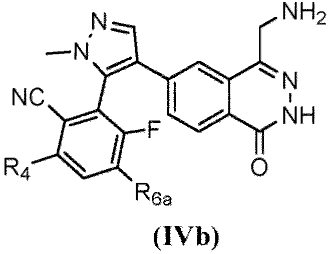
wherein R₄ and R₆ₐ are each independently selected from the group consisting of halogen, C₁₋₄ alkoxy, C₃₋₈ cycloalkoxy, 3-8 membered heterocyclyl or 3-8 membered heterocyclyloxy, and the C₁₋₄ alkoxy, C₃₋₈ cycloalkoxy, 3-8 membered heterocyclyl or 3-8 membered heterocyclyloxy is each further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, -O-R₂₀ and -NR₂₂R₂₃, provided that at least one of R₄ and R₆ₐ is 3-8 membered nitrogen-containing heterocyclyl, the nitrogen atom is attached to the benzene ring, and the 3-8 membered nitrogen-containing heterocyclyl is each further optionally and independently substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₈ cycloalkyl, 3-8 membered heterocyclyl, C₆₋₈ aryl, 5-8 membered heteroaryl, =O, =S, -SF₅, - O-R₂₀ and -NR₂₂R₂₃;
wherein R₂₀, R₂₂, and R₂₃ are defined as in claim 5.

9. The compound of formula (I), the stereoisomer or pharmaceutically acceptable salt thereof according to claim 8, wherein R₄ and R₆ₐ are each independently selected from the group consisting of fluorine, chlorine, cyclopropoxy, cyclobutoxy and 3-8 membered nitrogen-containing heterocyclyl, and the cyclopropoxy, cyclobutoxy or 3-8 membered nitrogen-containing heterocyclyl is further optionally and independently substituted by one or more substituents selected from the group consisting of deuterium, fluorine, chlorine, cyano, methyl, ethyl, n-propyl, isopropyl, trifluoromethyl, difluoromethyl, trideuteriomethyl, dideuteriomethyl, vinyl, ethynyl, cyclopropyl, cyclobutyl, oxa-cyclobutyl, aza-cyclobutyl, phenyl, -SF₅, =O, hydroxy, methoxy, ethoxy, n-propyloxy, isopropyloxy, amino, mono-C₁₋₄ alkyl amino and di-C₁₋₄ alkyl amino, provided that at least one of R₄ and R₆ₐ is 3-8 membered nitrogen-containing heterocyclyl, the nitrogen atom is attached to the benzene ring, and the 3-8 membered nitrogen-containing heterocyclyl is each further optionally and independently substituted by one or more substituents selected from the group consisting of deuterium, fluorine, chlorine, cyano, methyl, ethyl, n-propyl, isopropyl, trifluoromethyl, difluoromethyl, trideuteriomethyl, dideuteriomethyl, vinyl, ethynyl, cyclopropyl, cyclobutyl, oxa-cyclobutyl, aza-cyclobutyl, phenyl, -SF₅, =O, hydroxy, methoxy, ethoxy, n-propyloxy, isopropyloxy, amino, mono-C₁₋₄ alkyl amino and di-C₁₋₄ alkyl amino.

10. The compound of formula (I), the stereoisomer or pharmaceutically acceptable salt thereof according to claim 9, wherein the 3-8 membered nitrogen-containing heterocyclyl is selected from the following structure:

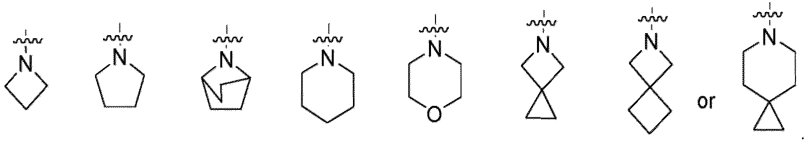

11. The compound of formula (I), the stereoisomer or pharmaceutically acceptable salt thereof according to any one of claims 1-10, wherein it is selected from the following compounds:

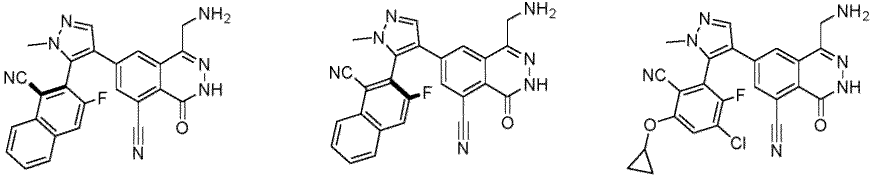

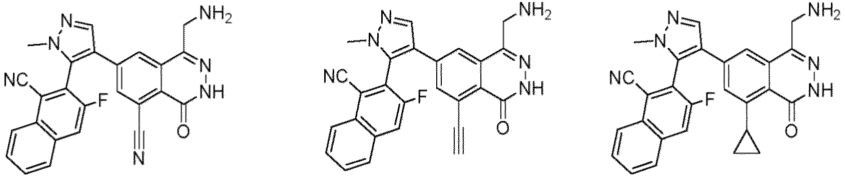

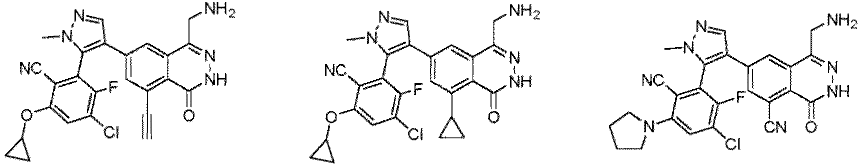

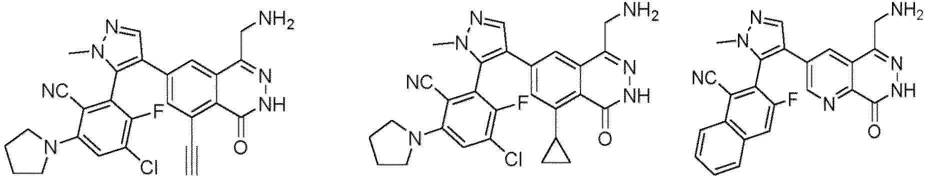

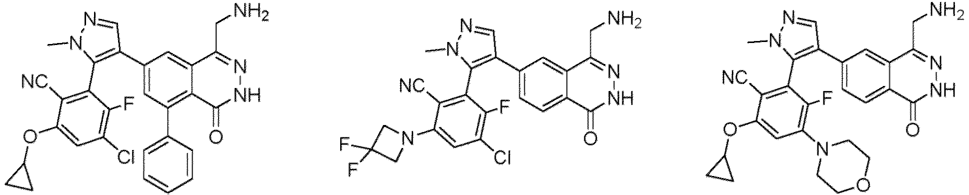

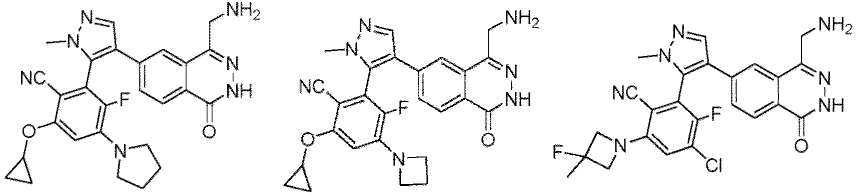

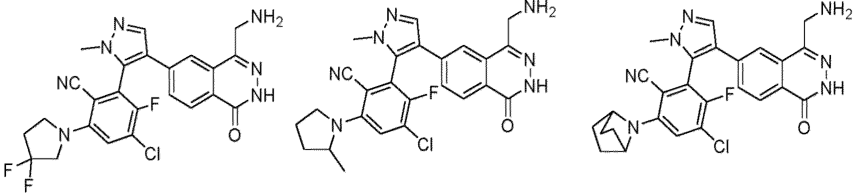

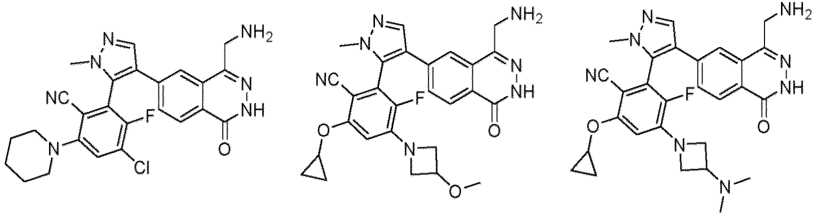

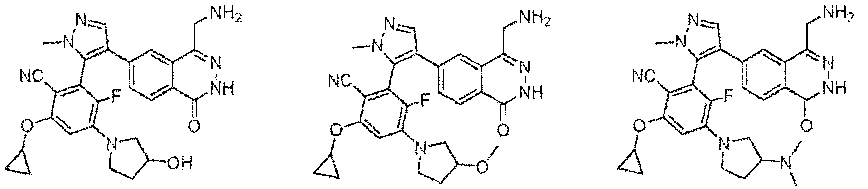

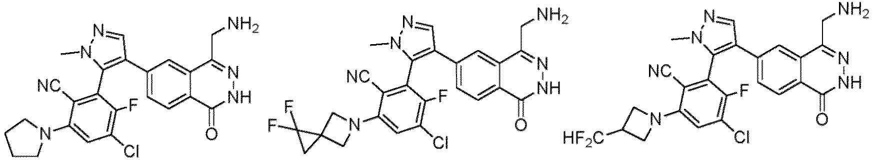

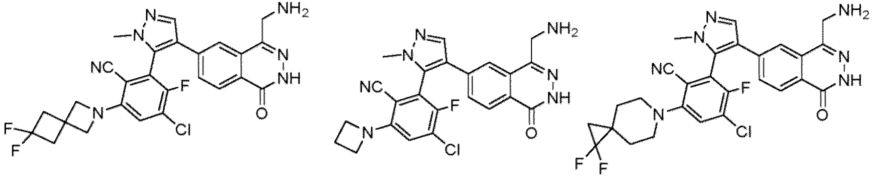

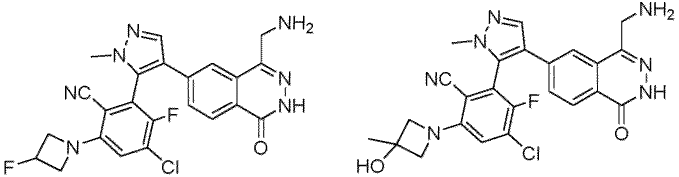

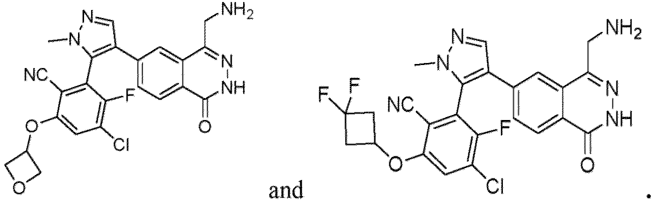

12. A pharmaceutical composition comprising the compound of formula (I), the stereoisomer or pharmaceutically acceptable salt thereof of any one of claims 1-11, and a pharmaceutically acceptable carrier.

13. Use of the compound of formula (I), the stereoisomer or pharmaceutically acceptable salt thereof of any one of claims 1-11 in the preparation of a medicament for treating MATP-associated cancer or tumor.

14. The use according to claim 13, wherein the tumor or cancer is selected from the group consisting of endometrial carcinoma, granulosa-theca cell tumor, Sertoli-Leydig cell tumor, germinomas, malignant teratoma, squamous cell carcinoma, intraepithelial cancer, adenocarcinoma, fibrosarcoma, melanoma, clear cell carcinoma, squamous cell carcinoma, botryoid sarcoma, fallopian tube cancer, adenocarcinoma, nephroblastoma, lymphoma, leukemia, bladder cancer, squamous cell carcinoma, transitional cell carcinoma, adenocarcinoma, prostate cancer, seminoma, teratoma, embryonal carcinoma, teratoma, choriocarcinoma, sarcoma, mesenchymal cell carcinoma, fibroma, fibroadenoma, adenomatoid tumor, lipoma, liver cancer, cholangiocarcinoma, hepatoblastoma, hemangiosarcoma, hepatocellular adenoma, hemangioma, gallbladder cancer, ampullary carcinoma, cholangiocarcinoma, malignant melanoma, basal cell carcinoma, squamous cell carcinoma, Kaposi's sarcoma, moles, dysplastic nevus, lipomyoma, hemangioma, acute and chronic myeloid leukemia, acute lymphoblastic leukemia, chronic lymphoblastic leukemia, myeloproliferative disorder, multiple myeloma, myelodysplastic syndrome, Hodgkin's disease, non-Hodgkin's lymphoma, osteosarcoma, fibrosarcoma, malignant fibrous histiocytoma, chondrosarcoma, Ewing's sarcoma, malignant lymphoma, multiple myeloma, malignant giant cell tumor chordoma, osteochondroma, benign chondroma, chondroblastoma, chondromyxoid fibroma, osteoid osteoma, giant cell tumor, angiosarcoma, fibrosarcoma, rhabdomyosarcoma, liposarcoma, myxoma, rhabdomyoma, fibroma, lipomyoma and teratoma, bronchial carcinoma, alveolar carcinoma, bronchial adenoma, sarcoma, lymphoma, chondromatoid hamartoma, mesothelioma squamous cell carcinoma, adenocarcinoma, leiomyosarcoma, lymphoma, gastric cancer, lymphoma, leiomyosarcoma, ductal adenocarcinoma, insulinoma, glucagonoma, gastrinoma, carcinoid tumor, serpentine tumor, adenocarcinoma, lymphoma, carcinoid tumor, Kaposis sarcoma, leiomyoma, hemangioma, lipomyoma, neurofibroma, fibroma, adenocarcinoma of large intestine, tubular adenoma, villous adenoma, hamartoma, leiomyoma, osteoma of skull, hemangioma, granuloma, xanthoma, osteitis deformans, meningioma, meningeal sarcoma, gliomatosis, astrocytoma, medulloblastoma, glioma, ependymoma, germ cell tumor, glioblastoma multiforme, oligodendroglioma, neurilemmoma, retinoblastoma, congenital tumor, spinal cord neurofibroma, meningioma, glioma and sarcoma.

15. The use according to claim 13, wherein the cancer or tumor is selected from breast cancer, pancreatic cancer, skin cancer, bladder cancer, liver cancer, or head and neck cancer.

16. The compound of formula (I), the stereoisomer or pharmaceutically acceptable salt thereof according to any one of claims 1-11, for use as a PRMT5 inhibitor.
